# EUROPÄISCHE PATENTSCHRIFT

(11) **EP 1 761 524 B1**
(45) Veröffentlichungstag und Bekanntmachung des Hinweises auf die Patenterteilung: **21.11.2007**
(21) Anmeldenummer: 05823871.8
(22) Anmeldetag: 27.12.2005
(51) Int. Cl.: C07D 405/12, C07D 407/12, C07D 409/12, A61K 31/404, A61P 25/00

(54) **SAUERSTOFFHALTIGE ANNELIERTE PHENYLPIPERAZIN- UND PHENYLDIAZEPANCARBOXAMIDE ALS DOPAMIN D3 ANTAGONISTEN**
PHENYL DIAZEPANE CARBOXAMIDES AND ANNELATED PHENYL PIPERAZINE CARBOXAMIDES CONTAINING OXYGEN AND USED AS DOPAMINE D3 ANTAGONISTS
CARBOXAMIDES DE PHENYLE PIPERAZINE ET DE PHENYLE DIAZEPANE ANNELES ET CONTENANT DE L'OXYGENE EN TANT QU'ANTAGONISTES DE LA DOPAMINE D3

(30) Priorität: 30.12.2004 DE 102004063797
(43) Veröffentlichungstag der Anmeldung: 14.03.2007
(73) Patentinhaber: SCHWARZ PHARMA AG, 40789 Monheim/Rhld. (DE)
(72) Erfinder: GMEINER, Peter, 91054 Buckenhof (DE); HÜBNER, Harald, 91336 Heroldsbach (DE); SCHLOTTER, Karin, 86732 Oettingen (DE)
(74) Vertreter: Dressen, Frank
(86) Internationale Anmeldenummer: PCT/EP2005/014047
(87) Internationale Veröffentlichungsnummer: WO 2006/072430

(56) Entgegenhaltungen:
- WO-A-20/04004729
- WO-A-20/04024878

## Beschreibung

Dopamin gilt als wichtiger Neurotransmitter des zentralen Nervensystems. Seine Wirkung vermittelt Dopamin durch Bindung an fünf verschiedene Dopaminrezeptoren. Diese lassen sich aufgrund ihrer Morphologie und ihrer Art der Signalübertragung in die Klassen D1-like (D1 und D5) sowie D2-like (D2-, D3- und D4-Rezeptoren) einteilen (Neve, K.A.The Dopamine Receptors. Humana Press, 1997). Vor allem die Subtypen der D2-Familie spielen bei der Regulationzentralnervöser Vorgänge eine wichtige Rolle. Während die D2-Rezeptoren überwiegend in den Basalganglien exprimiert werden und dort an der Kontrolle und Modulation neuromotorischer Schaltkreise beteiligt sind, befinden sich D3-Rezeptoren vor allem im mesolimbischen System, in dem emotionale und kognitive Vorgänge gesteuert werden. Störungen in der Signaltransduktion dieser Rezeptoren führen zu zahlreichen neuropathologischen Veränderungen, die zum Teil schwerwiegende Erkrankungen hervorrufen. Somit stellt insbesondere der D3-Rezeptor ein vielversprechendes Target für die Entwicklung von Wirkstoffen zur Behandlung von psychiatrischen Erkrankungen wie der Schizophrenie oder der unipolaren Depressionen, von Bewusstseinsstörungen sowie zur Behandlung neurodegenerativer Krankheiten wie dem Parkinsonismus und den im Zuge einer Langzeittherapie auftretendenden Dyskinesien, aber auch zur Behandlung von Drogenabhängigkeit (Pulvirenti, L. et al. Trends Pharmacol. Sci. 2002, 23, 151-153, Joyce, J.N. Pharmacol. Ther. 2001, 90, 231-259) dar. Anzustreben ist dabei ein möglichst D3-Rezeptor-selektives Bindungsprofil für solche Wirksubstanzen. Je nach intrinsischer Aktivität (voller Agonist, partieller Agonist, Antagonist oder inverser Agonist) können solche Liganden stimuliernd, modulierend oder auch hemmend auf das pathologisch veränderte Dopamin-Signaltransduktionssystem Einfluß nehmen und somit zur Therapie dieser Erkrankungen eingesetzt werden.

Verbindungen mit Arylpiperazin-Struktur sind bereits als dopaminrezeptoraktive Liganden beschrieben worden (Robarge, M.J. J. Med. Chem. 2001, 44; 3175-3186). Weiterhin sind Benzamide und Naphthamide mit Arylpiperazin-Partialstruktur als Liganden von Dopaminrezeptoren bekannt (Perrone, R. J. Med. Chem. 1998, 41, 4903-4909; EP 0 779. 284 A1). Vor kurzer Zeit wurden auch Heteroarenamide als D3-Rezeptor-aktive Verbindungen beschrieben (Bettinetti, L. et al. J. Med. Chem. 2002, 45, 4594-4597, Leopoldo, M. et al. J. Med. Chem. 2002, 45, 5727-5735, WO 2004004729 A1). Kürzlich wurde außerdem von einem Phenylpiperazinylnaphthamid als selektiven D3-Partialagonisten berichtet, der im Tiermodell hoffnungsvolle Aktivitäten zeigt, die für die Behandlung der Kokainsucht eingesetzt werden könnten (Pilla, M. et al. Nature 1999, 400, 371-375). Darüber hinaus konnte aufgrund der charakteristischen Eigenschaften dieser Verbindung eine Aufhebung der bei einer Langzeittherapie, des Parkinsonismus mit dem Arzneistoff L-DOPA verursachten schweren Bewegungsanomalien (Dyskinesien) erzielt werden (Bezard, E. et al. Nature Med. 2003, 9, 762-767). Neueste Literatur beschreibt die neuroprotektive Wirkung D3-selektiver Partialagonisten gegen MPTP-induzierten Neuronenverlust bei Mäusen als murines Modell des Parkinsonismus (Böeckler, F. et al. Biochem. Pharmacol. 2003, 6, 1025-1032).

Das gemeinsame Strukturmerkmal vieler hochaffiner Dopaminrezeptor-Liganden besteht aus einer variabel substituierten Phenylpiperazinpartialstruktur, die über einen mehrere Kohlenstoffe langen Abstandshalter an ein Aryl- oder Heteroarylcarboxamid geknüpft ist. Aus der Reihe der Arylpiperazinylheteroarencarboxamide sind vor allem Strukturbeispiele mit sauerstoff-, schwefel- oder stickstoffhaltigen Heteroarencarbonsäurekomponenten beschrieben (ES 2027898; EP 343 961; US 3646047; US 3734915; WO 2004/024878; Leopoldo, M. et al. J. Med. Chem. 2002, 45, 5727-5735, Bettinetti, L. et al. J. Med. Chem. 2002, 45, 4594-4597; Campiani, G. et al. J. Med. Chem. 2003, 46, 3822-3839;Hackling, A. et al. J. Med. Chem. 2003, 46, 3883-3889; WO 2004004729 A1).

Solche Verbindungen bestehen aus einem Indol-, Benzthiophen- oder Benzofuran-Carboxamidteil, der über einen aliphatischen Abstandshalter an ein optional substituiertes Phenylpiperazin gebunden ist.

Als Substituenten des Phenyls sind bisher vor allem alicylische Reste und einfache funktionelle Gruppen beschrieben (Bettinetti, L. et al. J. Med. Chem. 2002, 45, 4594-4597, Chu, W. et al. Bioorg. Med. Chem. 2005, 13, 77-87). In Struktur-Aktivitäts-Untersuchungen mit Liganden für verwandte biogene Aminrezeptoren, die verschiedene Substitutionsmuster an der Phenylgruppe aufweisen, wurde aber gezeigt, dass je nach Art des Substituenten und der Verküpfungsposition am Phenylring eine Modulation der Rezeptoraffinität und -selektivität und auch der intrinsischen Aktivität möglich ist (Heinrich et al. J. Med. Chem. 2004, 47, 4677-4683, Heinrich et al. J. Med. Chem. 2004, 47, 4684-4692, EP0372657).

Ein Ziel der vorliegenden Patentanmeldung ist die Bereitstellung neuer Substanzen mit hoher Affinität zu Dopaminrezeptoren, insbesondere zum humanen D3-Rezeptor.
Unsere intensiven Struktur-Wirkungsuntersuchungen mit verschiedensten Dopaminrezeptorliganden ergaben nun überraschend, dass der Dopamin D3-Rezeptor auch Indol-, Benzthiophen- und Benzofurancarboxamide als hochaffine Liganden erkennt, die über oben beschriebenen aliphatischen Abstandshalter an ein Arylpiperazin gebunden sind, in dem der Arylteil aus einem Phenylring besteht, der mit einem gesättigten, sauerstoffhaltigen 5-, 6- oder 7-gliedrigen Ring anneliert ist und auf diese Weise z.B. ein Dihydrobenzofuran, Chroman oder Tetrahydrobenzoxepin bildet Auch wurde überraschend festgestellt, dass der Piperazinring gegen einen Diazepanring ausgetauscht werden kann, ohne dass die Affinität der Substanzen zum humanen D3-Rezeptor verloren geht.

Diese Verbindungen zeigten bei *in vitro* Untersuchungen hohe Affinität und selektive Bindungseigenschaften am D3-Rezeptor sowie bemerkenswerte Affinität zu adrenergen alpha 1 und serotonergen 5-HT1 a-Rezeptoren. Insbesondere Substanzen mit gleichzeitiger hoher Affinität für den humanen D3 und den humanen 5-HT1 a Rezeptor haben großes Potenzial bei einer Reihe medizinischer Indikationen.

Die erfindungsgemäßen Verbindungen könnten wertvolle Therapeutika zur Behandlung von ZNS-Erkrankungen, wie beispielsweise Schizophrenie-oder verschiedenen Arten der Depression, zur Neuroprotektion bei neurodegenerativen Erkrankungen, bei Suchterkrankungen, Glaukoma, kognitiven Störungen, Restless Leg Syndrom, Hyperaktivitätssyndrom (ADHS), Hyperprolaktinämie, Hyperprolaktinom, Autismus, bei idiopathischen oder Medikamenten-induzierten extrapyramidalmotorischen Bewegungsstörungen, z.B. Akathisie, Rigor, Dystonie und Dyskinesien sowie verschiedenen Erkrankungen des Urinaltraktes und Schmerz darstellen.

Gegenstand dieser Erfindung sind Verbindungen der allgemeinen Formel I, 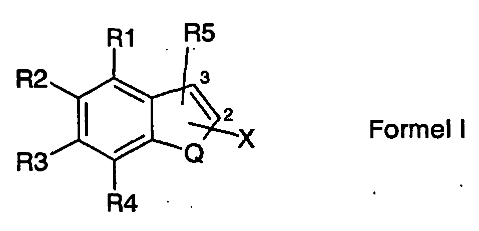 in der bedeuten:
Q ist ausgewählt aus S, O und NR;
R ist ausgewählt aus Wasserstoff, Alkyl, Phenyl, Alkylcarbonyl, Phenylalkylcarbonyl, Phenylcarbonyl, Phenylalkyl und Phenylsulfonyl;
R1, R2, R3 und R4 sind jeweils unabhängig voneinander ausgewählt aus der Gruppe Wasserstoff, Hydroxy, Alkyl, Alkyloxy, Alkylthio, Alkenyl, Alkinyl, Phenyl, Phenylalkyl, Phenoxy, Halogen, Trifluormethyl, Alkylcarbonyl, Phenylcarbonyl, Phenylalkylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Alkylsulfonylamino;
R5 ist eine an Position 2 oder 3 des bizyklischen Heteroaryls gebundene Gruppe, die ausgewählt ist aus Wasserstoff, Alkyl, Halogen, Alkoxy und Amino und die bevorzugt Wasserstoff oder Halogen repräsentiert.

X ist eine an Position 2 oder 3 des bizyklischen Heteroaryls gebundene Gruppe der allgemeinen Formel X1 in der gilt: 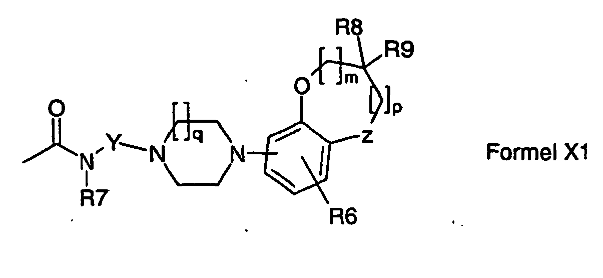
R6 ist ausgewählt aus der Gruppe Wasserstoff, Hydroxy, Alkyl, Alkyloxy, Alkylthio, Alkenyl, Alkinyl, Phenyl, Phenylalkyl, Phenoxy, Halogen, Trifluormethyl, Alkylcarbonyl, Phenylcarbonyl, Phenylalkylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Alkylsulfonylamino;
R7 ist Wasserstoff, Alkyl oder Phenylalkyl;
Y ist eine unverzweigte, gesättigte oder ungesättigte Kohlenwasserstoffkette mit 2-5 Kohlenstoffatomen;
m und p sind jeweils unabhängig voneinander 0, 1, oder 2, wobei die Summe aus m und p höchstens 2 ist; bevorzugt ist die Summe aus m und p 1 oder 2, ganz besonders bevorzugt 2;
q ist 1 oder 2;
Z ist CH₂, NH oder O und bevorzugt ist Z CH₂ oder O;
R8 und R9 sind jeweils unabhängig voneinander ausgewählt aus Wasserstoff, Alkyl und Phenyl oder bilden gemeinsam eine Oxo-Gruppe;
in Form der freien Base, deren physiologisch akzeptable Salze sowie möglicher Enantiomere und Diastereomere.

In einer bevorzugten Ausführungsform der Erfindung sind die Substituenten R1, R2, R3, R4 und R6 in den erfindungsgemäßen Verbindungen der allgemeinen Formeln I bis VII (Formel II-VII wie weiter unten aufgeführt) ausgewählt aus der Gruppe Wasserstoff, Hydroxy; Fluor; Chlor; Brom; Trifluormethyl; Cyano; Amino; Carboxy; Sulfo; Sulfamoyl; unsubstituiertes oder mit Hydroxy substituiertes C1-C6 Alkyl; unsubstituiertes oder mit Hydroxy substituiertes C1-C6 Alkyloxy; unsubstituiertes oder mit Hydroxy substituiertes C1-C6 Alkylthio; unsubstituiertes C2-C6 Alkinyl; unsubstituiertes oder mit Fluor, Chlor oder Brom und/oder mit einer oder mehreren Methoxygruppen substituiertes Phenyl; Phenyl(C1-C6)Alkyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; unsubstituiertes oder mit Fluor, Chlor oder Brom und/oder mit einem oder mehreren Methoxygruppen substituiertes Phenoxy; - C(O)-(C1-C6)Alkyl, wobei das Alkyl unsubstituiert oder mit Hydroxy substituiert ist; -C(O)-Phenyl, wobei das Phenyl unsubstituiert oder mit Fluor, Chlor oder Brom und/oder mit einer oder mehreren Methoxygruppen substituiert ist; -C(O)- (C1-C6)Alkyl-Phenyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; C1-C6 Alkyloxycarbonyl, wobei das Alkyl unsubstituiert oder mit Hydroxy substituiert ist; Phenyl(C1-C6)Alkyloxycarbonyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; C1-C6 Alkylaminosulfonyl, insbesondere Methylaminosulfonyl und C1-C6 Alkylsulfonylamino; insbesondere Methansulfonylamino.

X stellt bevorzugt eine Gruppe der allgemeinen Formel X2 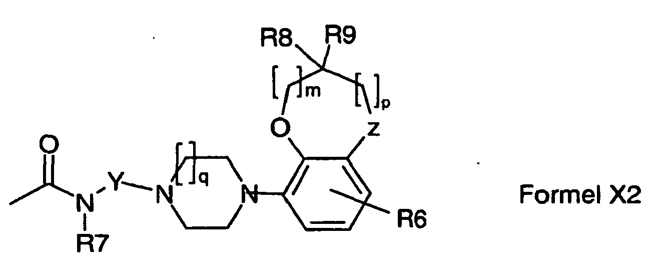 dar, in der R6, R7, R8, R9, m, p, q, Y und Z die Bedeutung haben, wie weiter oben definiert.

In einer bevorzugten Ausführungsform der Erfindung repräsentiert R7 in der Formel X1 oder X2 ein Wasserstoffatom.

in einer anderen Ausführungsform der Erfindung repräsentiert R6 in der Formel X1 oder X2 ein Wasserstoffatom.

In einer anderen Ausführungsform der Erfindung repräsentieren R6 und R7 in der Formel X1 oder X2 beide jeweils ein Wasserstoffatom.

In einer Ausführungsform der Erfindung repräsentieren R8 und R9 in der Formel X1 oder X2 beide jeweils ein Wasserstoffatom.

In einer anderen Ausführungsform der Erfindung repräsentieren R8 und R9 gemeinsam eine Oxo-Gruppe, insbesondere wenn.Z für NH steht. In diesem Fall hat p bevorzugt den Wert 0.

In einer bevorzugten Ausführungsform der Erfindung repräsentieren R6, R7, R8 und R9 in der Formel X1 oder X2 jeweils Wasserstoff.

In einer anderen, bevorzugten Ausführungsform der Erfindung repräsentieren R6, R7, R8 und R9 in der Formel X1 oder X2 jeweils ein Wasserstoffatom und Y eine gesättigte, unverzweigte Kohlenstoffkette mit 2-5 und bevorzugt mit 4 oder 5 Kohlenstoffen.
In einer anderen, bevorzugten Ausführungsform der Erfindung repräsentieren R1, R4, R5, R6 und R7 jeweils ein Wasserstoffatom und Y eine gesättigte, unverzweigte Kohlenstoffkette mit 2-5 und bevorzugt mit 4 oder 5 Kohlenstoffen.

In einer bevorzugten Ausführungsform der Erfindung ist Y in den erfindungsgemäßen Verbindungen eine Kette der Formel -(CH₂)ₙ- mit n = 2, 3, 4 oder 5, ganz besonders bevorzugt mit n = 3, 4 oder 5, insbesondere mit n= 4 oder 5.

In einer Ausführungsform der Erfindung repräsentiert Z in der Formel X1 oder X2 die Gruppe CH₂. In einer Ausführungsform der Erfindung repräsentiert Z in der Formel X1 oder X2 ein O oder eine CH₂-Gruppe. In einer anderen Ausführungsform der Erfindung ist Z eine NH-Gruppe.

In einer bevorzugten Ausführungsform der Erfindung repräsentiert q in der Formel X1 oder X2 den Wert 1. In einer anderen bevorzugten Ausführungsform der Erfindung repräsentiert q in der Formel X1 oder X2 den Wert 2.

In einer anderen, bevorzugten Ausführungsform der Erfindung repräsentieren R1, R4, R5, R6 und R7 in der Formel X1 oder X2 jeweils ein Wasserstoffatom, Y ist eine gesättigte, unverzweigte Kohlenstoffkette mit 3-5 Kohlenstoffen, m und p sind jeweils 0 und Z ist CH₂ oder Sauerstoff.

In einer anderen bevorzugten Ausführungsform der Erfindung gilt:
- Q ist ausgewählt aus S, O oder NH;
- R1 und R4 sind H;
- R5 ist H oder Halogen;
- R2 und R3 sind ausgewählt aus Wasserstoff, Hydroxy; Fluor; Chlor; Brom; Trifluormethyl; Cyano; Amino; Carboxy; Sulfo; Sulfamoyl; unsubstituiertes oder mit Hydroxy substituiertes C1-C6 Alkyl; unsubstituiertes oder mit Hydroxy substituiertes C1-C6 Alkyloxy; unsubstituiertes oder mit Hydroxy substituiertes C1-C6 Alkylthio; unsubstituiertes C2-C6 Alkinyl; unsubstituiertes oder mit Fluor, Chlor oder Brom und/oder mit einer oder mehreren Methoxygruppen, substituiertes Phenyl; Phenyl(C1-C6)Alkyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; unsubstituiertes oder mit Fluor, Chlor oder Brom und/oder mit einem oder mehreren Methoxygruppen substituiertes Phenoxy; -C(O)-(C1-C6)Alkyl, wobei das Alkyl unsubstituiert oder mit Hydroxy substituiert ist; -C(O)-Phenyl, wobei das Phenyl unsubstituiert oder mit Fluor, Chlor oder Brom und/oder mit einer oder mehreren Methoxygruppen substituiert ist; -C(O)-(C1-C6)Alkyl-Phenyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; C1-C6 Alkyloxycarbonyl, wobei das Alkyl unsubstituiert oder mit Hydroxy substituiert ist; Phenyl(C1-C6)Alkyloxycarbonyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; C1-C6 Alkylaminosulfonyl, insbesondere Methylaminosulfonyl und C1-C6 Alkylsulfonylamino; insbesondere Methansulfonylamino
- X ist eine Gruppe der Formel X1 oder X2, für die gilt:
   o R6 repräsentiert Wasserstoff, C1-C6 Alkyl, C1-C6 Alkoxy oder Halogen;
   o R7 repräsentiert Wasserstoff oder C1-C6 Alkyl;
   o R8 und R9 sind Wasserstoff;
   O Z ist CH2 oder O;
   O Summe aus m und p = 0, 1 oder 2 und besonders bevorzugt 1 oder 2;
   o q ist 1 oder 2;
   o Y ist eine unverzweigte gesättigte Kohlenwasserstoffkette mit 3, 4 oder 5 C-Atomen.

In einer bevorzugten Ausführungsform der Erfindung ist die Gruppe X mit der allgemeinen Formel X1 oder X2 in 2-Position an das bizyklische Heteroaryl der allgemeinen Formel I gebunden und weist die allgemeine Formel II auf: 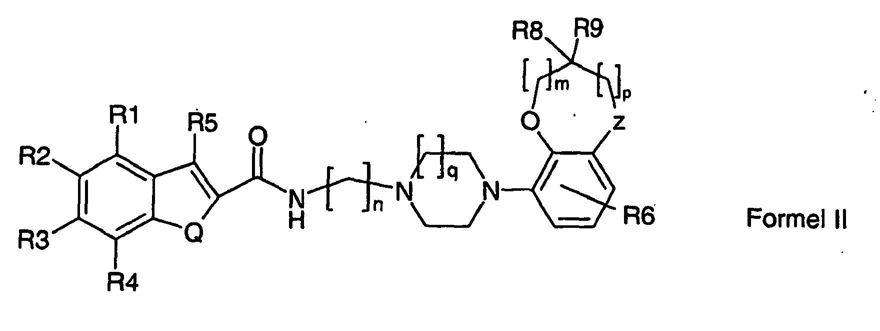 worin R, R1, R2, R3, R4, R5, R6, R8, R9, Q, Z, m, p und q jeweils die Bedeutung haben, wie weiter oben definiert und worin n den Wert 2, 3, 4 oder 5 hat.

In einer bevorzugten Ausführungsform der Erfindung repräsentieren R1, R4, R5 und R6 in den Verbindungen der allgemeinen Formel 11 jeweils ein Wasserstoffatom.

In einer bevorzugten Ausführungsform*der Erfindung repräsentiert Z in den Verbindungen der allgemeinen Formel 11 die Gruppe CH₂ und m hat den Wert 0.

In einer bevorzugten Ausführungsform der Erfindung repräsentiert Z in den Verbindungen der allgemeinen Formel II eine CH₂ -Gruppe oder ein O.

In einer Ausführungsform der Erfindung repräsentieren R8 und R9 in den Verbindungen der allgemeinen Formel II jeweils ein Wasserstoffatom.

In einer anderen Ausführungsform der Erfindung repräsentieren R8 und R9 in der Verbindung der allgemeinen Formel II eine Oxogruppe, insbesondere wenn Z für NH steht. In diesem Fall hat p bevorzugt den Wert 0.

In einer bevorzugten Ausführungsform der Erfindung hat q in den Verbindungen der allgemeinen Formel II den Wert 1.

in einer anderen, bevorzugten Ausführungsform der Erfindung repräsentieren in Formel II R1, R4, R5 und R6 jeweils ein Wasserstoffatom, n ist 2, 3, 4 oder 5, m ist 0, q ist 1 und Z ist CH₂ oder Sauerstoff, wobei n bevorzugt 3, 4 oder 5 und besonders bevorzugt 4 oder 5 ist.

In einer anderen, bevorzugten Ausführungsform der Erfindung repräsentieren in Formel II
- R1, R4, R6, R8 und R9 jeweils ein Wasserstoffatom;
- R2 und R3 Wasserstoff, Hydroxy; Fluor; Chlor; Brom; Trifluormethyl; Cyano; Amino; Carboxy; Sulfo; Sulfamoyl; unsubstituiertes oder mit Hydroxy substituiertes C1-C6 Alkyl; unsubstituiertes oder mit Hydroxy substituiertes C1-C6 Alkyloxy; unsubstituiertes oder mit Hydroxy substituiertes C1-C6 Alkylthio; unsubstituiertes C2-C6 Alkinyl; unsubstituiertes oder mit Fluor, Chlor oder Brom und/oder mit einer oder mehreren Methoxygruppen substituiertes Phenyl; Phenyl(C1-C6)Alkyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; unsubstituiertes oder mit Fluor, Chlor oder Brom und/oder mit einem oder mehreren Methoxygruppen substituiertes Phenoxy; -C(O)-(C1-C6)Alkyl, wobei das Alkyl unsubstituiert oder mit Hydroxy substituiert ist; -C(O)-Phenyl, wobei das Phenyl unsubstituiert oder mit Fluor, Chlor oder Brom und/oder mit einer oder mehreren Methoxygruppen substituiert ist; -C(O)-(C1-C6)Alkyl-Phenyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; C1-C6 Alkyloxycarbonyl, wobei das Alkyl unsubstituiert oder mit Hydroxy substituiert ist; Phenyl(C1-C6)Alkyloxycarbonyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; C1-C6 Alkylaminosulfonyl, insbesondere Methylaminosulfonyl und C1-C6 Alkylsulfonylamino; insbesondere Methansulfonylamino;
- R5 ist H oder Halogen;
- n ist 3, 4 oder 5;
- q ist 1 oder 2;
- Z ist CH₂ oder Sauerstoff;
- die Summe aus m und p ist 0,1 oder 2 und besonders bevorzugt 1 oder 2;
wobei R2 und R3 besonders bevorzugt H, Halogen, Cyano oder C2-C6 Alkinyl sind.

Beispielhafte Verbindungen entsprechend der vorliegenden Erfindung entsprechend Formel II sind ausgewählt aus
1: N-(4-(4-(2,3-Dihydrobenzofuran-7-yl)piperazin-1-yl)butyl)benzo[b]thiophen-2-ylcarbamid
138: N-(4-(4-(2,3-Dihydrobenzofuran-7-yl)piperazin-1-yl)butyl)-3-chlorbenzo[b]thiophen-2-ylcarbamid
29: N-(4-(4-(2,3-Dihydrobenzofuran-7-yl)piperazin-1-yl)butyl)-5-cyanbenzo[b]thiophen-2-ylcarbamid
139: N-(4-(4-(2,3-Dihydrobenzofuran-7-yl)piperazin-1-yl)butyl)-6-ethinylbenzo[b]thiophen-2-ylcarbamid
30: N-(4-(4-(2,3-Dihydrobenzofuran-7-yl)piperazin-1-yl)butyl)benzofuran-2-ylcarbamid
31: N-(4-(4-(2,3-Dihydrobenzofuran-7-yl)piperazin-1-yl)butyl)-5-brombenzofuran-2-ylcarbamid
2: N-(4-(4-(2,3-Dihydrobenzofuran-7-yl)piperazin-1-yl)butyl)indol-2-ylcarbamid
32: N-(4-(4-(2,3-Dihydrobenzofuran-7-yl)piperazin-1-yl)butyl)-6-cyanindol-2-ylcarbamid
8: N-(4-(4-(2,3-Dihydrobenzofuran-7-yl)-1,4-diazepan-1-yl)butyl)benzo[b]thiophen-2-ylcarbamid
142: N-(4-(4-(2,3-Dihydrobenzofuran-7-yl)-1,4-diazepan-1-yl)butyl)-3-chlorbenzo[b]thiophen-2-ylcarbamid
42: N-(4-(4-(2,3-Dihydrobenzofuran-7-yl)-1,4-diazepan-1-yl)butyl)-5-cyanbenzo[b]thiophen-2-ylcarbamid
143: N-(4-(4-(2,3-Dihydrobenzofuran-7-yl)-1,4-diazepan-1-yl)butyl)-6-ethinylbenzo[b]thiophen-2-ylcarbamid
44: N-(4-(4-(2,3-Dihydrobenzofuran-7-yl)-1,4-diazepan-1-yl)butyl)benzofuran-2-ylcarbamid
45: N-(4-(4-(2,3-Dihydrobenzofuran-7-yl)-1,4-diazepan-1-yl)butyl)-5-brombenzofuran-2-ylcarbamid
47: N-(4-(4-(2,3-Dihydrobenzofuran-7-yl)-1,4-diazepan-1-yl)butyl)indol-2-ylcarbamid
48: N-(4-(4-(2,3-Dihydrobenzofuran-7-yl)-1,4-diazepan-1-yl)butyl)-6-cyanindol-2-ylcarbamid
3: N-(4-(4-(Chroman-8-yl)piperazin-1-yl)butyl)benzo[b]thiophen-2-ylcarbamid
140: N-(4-(4-(Chroman-8-yl)piperazin-1-yl)butyl)-3-chlorbenzo[b]thiophen-2-ylcarbamid
33: N-(4-(4-(Chroman-8-yl)piperazin-1-yl)butyl)-5-cyanbenzo[b]thiophen-2-ylcarbamid
141: N-(4-(4-(Chroman-8-yl)piperazin-1-yl)butyl)-6-ethinylbenzo[b]thiophen-2-ylcarbamid 34: N-(4-(4-(Chroman-8-yl)piperazin-1-yl)butyl)benzofuran-2-ylcarbamid
35: N-(4-(4-(Chroman-8-yl)piperazin-1-yl)butyl)-5-brombenzofuran-2-ylcarbamid
4: N-(4-(4-(Chroman-8-yl)piperazin-1-yl)butyl)indol-2-ylcarbamid
37: N-(4-(4-(Chrornan-8-yl)piperazin-1-yl)butyl)-6-cyanindol-2-ylcarbamid
10: N-(4-(4-(Chroman-8-yl)-1,4-diazepan-1-yl)butyl)benzo[b]thiophen-2-ylcarbamid
144: N-(4-(4-(Chroman-8-yl)-1,4-diazepan -1-yl)butyl)-3-chlorbenzo[b]thiophen-2-ylcarbamid
50: N-(4-(4-(Chroman-8-yl)-1,4-diazepan-1-yl)butyl)-5-cyanbenzo[b]thiophen-2-ylcarbamid
145: N-(4-(4-(Chroman-8-yl)-1,4-diazepan-1-yl)butyl)-6-ethinylbenzo[b]thiophen-2-ylcarbamid
11: N-(4-(4-(Chroman-8-yl)-1,4-diazepan-1-yl)butyl)benzofuran-2-ylcarbamid
52: N-(4-(4-(Chroman-8-yl)-1,4-diazepan-1-yl)butyl)-5-brombenzofuran-2-ylcarbamid
54- N-(4-(4-(Chroman-8-yl)-1,4-diazepan-1-yl)butyl)indol-2-ylcarbamid
55: N-(4-(4-(Chroman-8-yl)-1,4-diazepan-1-yl)butyl)-6-cyanindol-2-ylcarbamid
12: N-(4-(4-(2,3,4,5-Tetrahydrobenzo[b]oxepin-9-yl)piperazin-1-yl)butyl)benzo[b]thiophen-2-ylcarbamid
136: N-(4-(4-(2,3,4,5-Tetrahydrobenzo[b]oxepin-9-yl)piperazin-1-yl)butyl)-3-chlorbenzo[b]thiophen-2-ylcarbamid
13: N-(4-(4-(2,3,4,5-Tetrahydrobenzo[b]oxepin-9-yl)piperazin-1-yl)butyl)-5-cyanbenzo[b]thiophen-2-ylcarbamid
137: N-(4-(4-(2,3,4,5-Tetrahydrobenzo[b]oxepin-9-yl)piperazin-1-yl)butyl)-6-ethinyibenzo[b]thiophen-2-ylcarbamid
14: N-(4-(4-(2,3,4,5-Tetrahydrobenzo[b]oxepin-9-yl)piperazin-1-yl)butyl)benzofuran-2-ylcarbamid
15: N-(4-(4-(2,3,4,5-Tetrahydrobenzo[b]oxepin-9-yl)piperazin-1-yl)butyl)-5-brombenzofuran-2-ylcarbamid
16: N-(4-(4-(2,3,4,5-Tetrahydrobenzo[b]oxepin-9-yl)piperazin-1-yl)butyl)indol-2-ylcarbamid
17: N-(4-(4-(2,3,4,5-Tetrahydrobenzo[b]oxepin-9-yl)piperazin-1-yl)butyl)-6-cyanindol-2-ylcarbamid
18: N-(4-(4-(2,3,4,5-Tetrahydrobenzo[b]oxepin-9-yl)-1,4-diazepan-1-yl)butyl)benzo[b]thiophen-2-ylcarbamid
146: N-(4-(4-(2,3,4,5-Tetrahydrobenzo[b]oxepin-9-yl)-1,4-diazepan -1-yl)butyl)-3-chlorbenzo[b]thiophen-2-ylcarbamid
57: N-(4-(4-(2,3,4,5-Tetrahydrobenzo[b]oxepin-9-yl)-1,4-diazepan-1-yl)butyl)-5-cyanbenzo[b]thiophen-2-ylcarbamid
147: N-(4-(4-(2,3,4,5-Tetrahydrobenzo[b]oxepin-9-yl)-1,4-diazepan-1-yl)butyl)-6-ethinylbenzo[b]thiophen-2-ylcarbamid
19: N-(4-(4-(2,3,4,5-Tetrahydrobenzo[b]oxepin-9-yl)-1,4-diazepan-1-yl)butyl)benzofuran-2-ylcarbamid
59: N-(4-(4-(2,3,4,5-Tetrahydrobenzo[b]oxepin-9-yl)-1,4-diazepan-1-yl)butyl)-5-brombenzofuran-2-ylcarbamid
61: N-(4-(4-(2,3,4,5-Tetrahydrobenzo[b]oxepin-9-yl)-1,4-diazepan-1-yl)butyl)indol-2-ylcarbamid
62: N-(4-(4-(2,3,4,5-Tetrahydrobenzo[b]oxepin-9-yl)-1,4-diazepan-1-yl)butyl)-6-cyanindol-2-ylcarbamid
24: N-(4-(4-(Benzo[1,3]dioxol-4-yl)piperazin-1-yl)butyl)benzo[b]thiophen-2-ylcarbamid
148: N-(4-(4-(Benzo[1,3]dioxol-4-yl)piperazin-1-yl)butyl)-3-chlorbenzo[b]thiophen-2-ylcarbamid
64: N-(4-(4-(Benzo[1,3]dioxol-4-yl)piperazin-1-yl)butyl)-5-cyanbenzo[b]thiophen-2-ylcarbamid
149: N-(4-(4-(Benzo[1,3]dioxol-4-yl)piperazin-1-yl)butyl)-6-ethinylbenzo[b]thiophen-2-ylcarbamid
66: N-(4-(4-(Benzo[1,3]dioxol-4-yl)piperazin-1-yl)butyl)benzofuran-2-ylcarbamid
67: N-(4-(4-(Benzo[1,3]dioxol-4-yl)piperazin-1-yl)butyl)-5-brombenzofuran-2-ylcarbamid
69: N-(4-(4-(Benzo[1,3]dioxol-4-yl)piperazin-1-yl)butyl)indol-2-ylcarbamid
70: N-(4-(4-(Benzo[1,3]dioxol-4-yl)piperazin-1-yl)butyl)-6-cyanindol-2-ylcarbamid
158: N-(4-(4-(6-Chlor-3-oxo-3,4-dihydro-2H-benzo[1,4]oxazin-8-yl)piperazin-1-yl)butyl)benzo[b]thiophen-2-ylcarbamid

In einer bevorzugten Ausführungsform der Erfindung ist die Gruppe X mit der allgemeinen Formel X1 oder X2 in 3-Position an das bizyklische Heteroaryl der allgemeinen Formel I gebunden und weist die allgemeine Formel III auf: 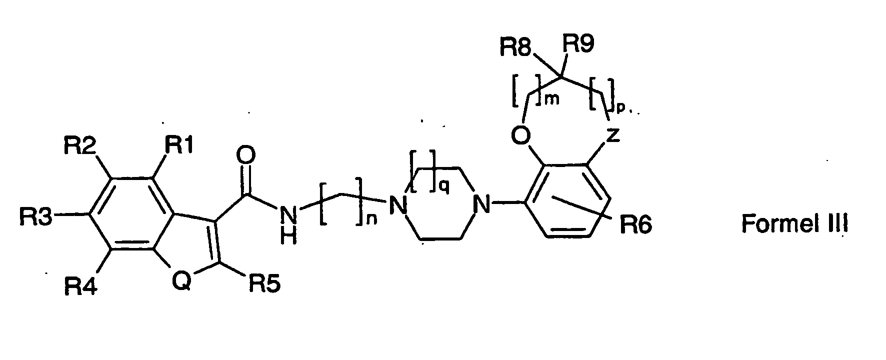 worin R, R1, R2, R3, R4, R5, R6, R8, R9, Q, Z, m, p und q jeweils die Bedeutung haben, wie weiter oben definiert und worin n den Wert 2, 3, 4 oder 5 hat.

In bevorzugten Ausführungsformen haben R, R1, R2, R3, R4, R5, R6, R8, R9, Q, Z, n, m, p und q die Bedeutung, wie vorstehend bei den bevorzugten Verbindungen der Formeln II beschrieben, wobei in bevorzugten exemplarisch aufgeführten Ausführungsformen der Formel III gilt: Q= O, S, NH; R1, R4, R5, R6, R8 und R9 = H; R2 und R3 = Wasserstoff, Halogen, Cyano oder C2-C6 Alkinyl; n = 4; q = 1, 2; Z = O, CH₂.

Beispielhafte Verbindungen entsprechend Formel III der vorliegenden Erfindung sind ausgewählt:
5: N-(4-(4-(2,3-Dihydrobenzofuran-7-yl)piperazin-1-yl)butyl)benzo[b]thiophen-3-ylcarbamid
6: N-(4-(4-(2,3-Dihydrobenzofuran-7-yl)piperazin-1-yl)butyl)benzofuran-3-ylcarbamid
7: N-(4-(4-(2,3-Dihydrobenzofuran-7-yl)piperazin-1-yl)butyl)indol-3-ylcarbamid
43: N-(4-(4-(2,3-Dihydrobenzofuran-7-yl)-1,4-diazepan-1-yl)butyl)benzo[b]thiophen-3-ylcarbamid
46: N-(4-(4-(2,3-Dihydrobenzofuran-7-yl)-1,4-diazepan-1-yl)butyl)benzofuran-3-ylcarbamid
49: N-(4-(4-(2,3-Dihydrobenzofuran-7-yl)-1,4-diazepan-1-yl)butyl)indol-3-ylcarbamid
51: N-(4-(4-(Chroman-8-yl)piperazin-1-yl)butyl)benzo[b]thiophen-3-ylcarbamid
36: N-(4-(4-(Chroman-8-yl)piperazin-1-yl)butyl)benzofuran-3-ylcarbamid
38: N-(4-(4-(Chroman-8-yl)piperazin-1-yl)butyl)indol-3-ylcarbamid
9: N-(4-(4-(Chroman-8-yl)-1,4-diazepan-1-yl)butyl)benzo[b]Miophen-3-ylcarbamid
53: N-(4-(4-(Chroman-8-yl)-1,4-diazepan-1 -yl)butyl)benzofuran-3-ylcarbamid
56: N-(4-(4-(Chroman-8-yl)-1,4-diazepan-1-yl)butyl)indol-3-ylcarbamid
39: N-(4-(4-(2,3,4,5-Tetrahydrobenzo[b]oxepin-9-yl)piperazin-1-yl)butyl)benzo[b]thiophen-3-ylcarbamid
40: N-(4-(4-(2,3,4,5- Tetrahydrobenzo[b]oxepin-9-yl)piperazin-1-yl)butyl)benzofuran-3-ylcarbamid
41: N-(4-(4-(2,3,4,5-Tetrahydrobenzo[b]oxepin-9-yl)piperazin-1-yl)butyl)indol-3-ylcarbamid
58: N-(4-(4-(2,3,4,5- Tetrahydrobenzo[b]oxepin-9-yl)-1 ,4-diazepan-1-yl)butyl)benzo[b]thiophen-3-ylcarbamid
60: N-(4-(4-(2,3,4,5-Tetrahydrobenzo[b]oxepin-9-yl)-1,4-diazepan-1-yl)butyl)benzofuran-3-ylcarbamid
63: N-(4-(4-(2,3,4,5-Tetrahydrobenzo[b]oxepin-9-yl)-1,4-diazepan-l-yl)butyl)indol-3-ylcarbamid
65: N-(4-(4-(Benzo[1,3]dioxol-4-yl)piperazin-1-yl)butyl)benzo[b]thiophen-3-ylcarbamid
68: N-(4-(4-(Benzo[1,3]dioxol-4-yl)piperazin-1-yl)butyl)benzofuran-3-ylcarbamid
71: N-(4-(4-(Benzo[1,3]dioxol-4-yl)piperazin-1-yl)butyl)indol-3-ylcarbamid

Eine andere bevorzugte Ausführungsform der Erfindung betrifft Verbindungen der Formel IV, in der die Gruppe X mit der allgemeinen Formel X1 oder X2 in 2-Position an das bizyklische Heteroaryl der allgemeinen Formel I gebunden ist: 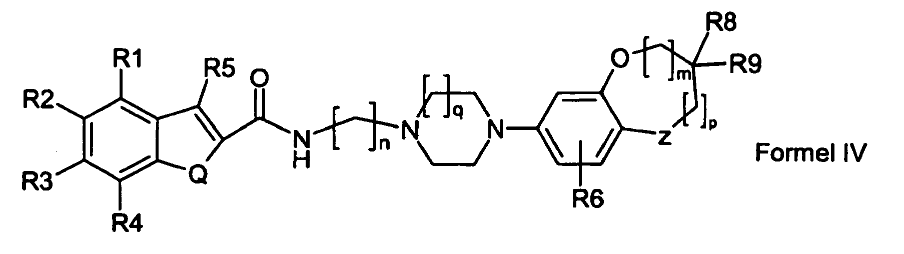 worin R, R1, R2, R3, R4, R5, R6, R8, R9, Q, Z, m, p und q die Bedeutung haben, wie weiter oben definiert und worin n den Wert 2, 3, 4 oder 5 hat.

In bevorzugten Ausführungsformen haben R, R1. R2, R3, R4, R5, R6, R8, R9, Q, Z, n, m, p und q die Bedeutung, wie vorstehend bei den bevorzugten Verbindungen der Formeln II und III beschrieben, wobei in bevorzugten exemplarisch aufgeführten Ausführungsformen der Formel IV gilt: Q = S; R1, R2, R3, R4, R5, R6, R8 und R9 = H; n = 4; q = 1, 2; Z = O, CH₂.

Beispielhafte Verbindungen entsprechend Formel IV der vorliegenden Erfindung sind:
20: N-(4-(4-(Chroman-7-yl)piperazin-1-yl)butyl)benzo[b]thiophen-2-ylcarbamid
150: N-(4-(4-(Chroman-7-yl)piperazin-1-yl)butyl)-3-chlorbenzo[b]thiophen-2-ylcarbamid
72: N N-(4-(4-(Chroman-7-yl)piperazin-1-yl)butyl)-5-cyanbenzo[b]thiophen-2-ylcarbamid
151: N-(4-(4-(Chroman-7-yl)piperazin-1-yl)butyl)-6-ethinylbenzo[b]thiophen-2-ylcarbamid
74: N-(4-(4-(Chroman-7-yl)piperazin-1-yl)butyl)benzofuran-2-ylcarbamid
75: N-(4-(4-(Chroman-7-yl)piperazin-1-yl)butyl)-5-brombenzofuran-2-ylcarbamid
77: N-(4-(4-(Chroman-7-yl)piperazin-1-yl)butyl)indol-2-ylcarbamid
78: N-(4-(4-(Chroman-7-yl)piperazin-1-yl)butyl)-6-cyanindol-2-ylcarbamid
25: N-(4-(4-(Benzo[1,3]dioxol-5-yl)piperazin-1-yl)butyl)benzo[b]thiophen-2-ylcarbamid
152: N-(4-(4-(Benzo[1,3]dioxol-5-yl)piperazin-1-yl)butyl)-3-chlorbenzo[b]thiophen-2-ylcarbamid
80: N-(4-(4-(Benzo[1,3]dioxol-5-yl)piperazin-1-yl)butyl)-5-cyanbenzo[b]thiophen-2-ylcarbamid
153: N-(4-(4-(Benzo[1,3]dioxol-5-yl)piperazin-1-yl)butyl)-6-ethinytbenzo[b]thiophen-2-ylcarbamid
82: N-(4-(4-(Benzo[1,3]dioxol-5-yl)piperazin-1-yl)butyl)benzofuran-2-ylcarbamid
83: N-(4-(4-(Benzo[1,3]dioxol-5-yl)piperazin-1-yl)butyl)-5-brombenzofuran-2-ylcarbamid
85: N-(4-(4-(Benzo[1,3]dioxol-5-yl)piperazin-1-yl)butyl)indol-2-ylcarbamid
86: N-(4-(4-(Benzo[1,3]dioxol-5-yl)piperazin-1-yl)butyl)-6-cyanindol-2-ylcarbamid
26: N-(4-(4-(2,3-Dihydrobenzo[1,4]dioxin-6-yl)piperazin-1-yl)butyl)benzo[b]thiophen-2-ylcarbamid
154: N-(4-(4-(2,3-Dihydrobenzo[1,4]dioxin-6-yl)piperazin-1-yl)butyl)-3-chlorbenzo[b]thiophen-2-ylcarbamid
88: N-(4-(4-(2,3-Dihydrobenzo[1,4]dioxin-6-yl)piperazin-1-yl)butyl)-5-cyanbenzo[b]thiophen-2-ylcarbamid
155: N-(4-(4-(2,3-Dihydrobenzo[1,4]dioxin-6-yl)piperazin-1-yl)butyl)-6-ethinylbenzo[b]thiophen-2-ylcarbamid
90: N-(4-(4-(2,3-Dihydrobenzo[1,4]dioxin-6-yl)piperazin-1-yl)butyl)benzofuran-2-ylcarbamid
91: N-(4-(4-(2,3-Dihydrobenzo[1,4]dioxin-6-yl)piperazin-1-yl)butyl)-5-brombenzofuran-2-ylcarbamid
93: N-(4-(4-(2,3-Dihydrobenzo[1,4]dioxin-6-yl)piperazin-1-yl)butyl)indol-2-ylcarbamid
94: N-(4-(4-(2,3-Dihydrobenzo[1,4]dioxin-6-yl)piperazin-1-yl)butyl)-6-cyanindol-2-ylcarbamid
27: N-(4-(4-(2,3-Dihydrobenzo[1,4]dioxin-6-yl)-1,4-diazepan-1-yl)butyl)benzo[b]thiophen-2-ylcarbamid
104: N-(4-(4-(2,3-Dihydrobenzo[1,4]dioxin-6-yl)-1,4-diazepan-1-yl)butyl)-5-cyanbenzo[b]thiophen-2-ylcarbamid
106: N-(4-(4-(2,3-Dihydrobenzo[1,4]dioxin-6-yl)-1,4-diazepan-1-yl)butyl)benzofuran-2-ylcarbamid
107: N-(4-(4-(2,3-Dihydrobenzo[1,4]dioxin-6-yl)-1,4-diazepan-1-yl)butyl)-5-brombenzofuran-2-ylcarbamid
109: N-(4-(4-(2,3-Dihydrobenzo[1,4]dioxin-6-yl)-1,4-diazepan-1-yl)butyl)indol-2-ylcarbamid
110: N-(4-(4-(2,3-Dihydrobenzo[1,4]dioxin-6-yl)-1,4-diazepin-1-yl)butyl)-6-cyanindol-2-ylcarbamid
28: N-(4-(4-(3,4-Dihydro-2H-benzo[b][1,4]dioxepin-7-yl)piperazin-1-yl)butyl)benzo[b]thiophen-2-ylcarbamid
156: N-(4-(4-(3,4-Dihydro-2H-benzo[b][1,4]dioxepin-7-yl)piperazin-1-yl)butyl)-3-chlorbenzo[b]thiophen-2-ylcarbamid
96: N-(4-(4-(3,4-Dihydro-2H-benzo[b][1,4]dioxepin-7 -yl)piperazin-1-yl)butyl)-5-cyanbenzo[b]thiophen-2-ylcarbamid
157: N-(4-(4-(3,4-Dihydro-2H-benzo[b][1,4]dioxepin-7-yl)piperazin-1-yl)butyl)-6-ethinylbenzo[b]thiophen-2-ylcarbamid
98: N-(4-(4-(3,4-Dihydro-2H-benzo[b][1,4]dioxepin-7-yl)piperazin-1-yl)butyl)benzofuran-2-ylcarbamid
99: N-(4-(4-(3,4-Dihydro-2H-benzo[b][1,4]dioxepin-7-yl)piperazin-1-yl)butyl)-5-brombenzofuran-2-ylcarbamid
101: N-(4-(4-(3,4-Dihydro-2H-benzo[b][1,4]dioxepin-7-yl)piperazin-1-yl)butyl)indol-2-ylcarbamid .
102: N-(4-(4-(3,4-Dihydro-2H-benzo[b][1,4]dioxepin-7-yl)piperazin-1-yl)buty1)-6-cyanindol-2-ylcarbamid

Eine andere bevorzugte Ausführungsform der Erfindung betrifft Verbindungen der Formel V, in der die Gruppe X mit der allgemeinen Formel X1 oder X2 in 3-Position an das bizyklische Heteroaryl der allgemeinen Formel I gebunden ist: 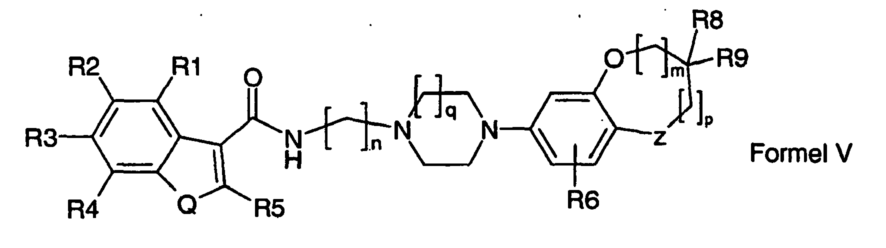 worin R, R1, R2, R3, R4, R5, R6, R8, R9, Q, Z, m, p und q die Bedeutung haben, wie weiter oben definiert und worin n den Wert 2, 3, 4 oder 5 hat.

In bevorzugten Ausführungsformen haben R, R1, R2, R3, R4, R5, R6, R8, R9, Q, Z, n, m, p und q die Bedeutung, wie vorstehend bei den bevorzugten Verbindungen der Formeln II, III und IV beschrieben.

Beispielhafte Verbindungen entsprechend Formel V der vorliegenden Erfindung sind:
73: N-(4-(4-(Chroman-7-yl)piperazin-1-yl)butyl)benzo[b]thiophen-3-ylcarbamid
76: N-(4-(4-(Chroman-7-yl)piperazin-1-yl)butyl)benzofuran-3-ylcarbamid
79: N-(4-(4-(Chroman-7-yl)piperazin-1-yl)butyl)indol-3-ylcarbämid
81: N-(4-(4-(Benzo[1,3]dioxol-5-yl)piperazin-1-yl)butyl)benzo[b]thiophen-3-ylcarbamid
84: N-(4-(4-(Benzo[1,3]dioxol-5-yl)piperazin-1-yl)butyl)benzofuran-3-ylcarbamid
87: N-(4-(4-(Benzo[1,3]dioxol-5-yl)piperazin-1-yl)butyl)indol-3-ylcarbamid
89: N-(4-(4-(2,3-Dihydrobenzo[1,4]dioxin-6-yl)piperazin-1-yl)butyl)benzo[b]thiophen-3-ylcarbamid
92: N-(4-(4-(2,3-Dihydrobenzo[1,4]dioxin-6-yl)piperazin-1-yl)butyl)benzofuran-3-ylcarbamid
95: N-(4-(4-(2,3-Dihydrobenzo[1,4]dioxin-6-yl)piperazin-1-yl)butyl)indol-3-ylcarbamid
105: N-(4-(4-(2,3-Dihydrobenzo[1,4]dioxin-6-yl)-1,4-diazepan-1-yl)butyl)benzo[b]thiophen-3-ylcarbamid
108: N-(4-(4-(2,3-Dihydrobenzo[1,4]dioxin-6-yl)-1,4-diazepan-1-yl)butyl)benzofuran-3-ylcarbamid
111: N-(4-(4-(2,3-Dihydrobenzo[1,4]dioxin-6-yl)-1,4-diazepan-1 -yl)butyl)iridol-3-ylcarbamid
97: N-(4-(4-(3,4-Dihydro-2H-benzo[b][1,4]dioxepin-7-yl)piperazin-1-yl)butyl)benzo[b]thiophen-3-ylcarbamid
100: N-(4-(4-(3,4-Dihydro-2H-benzo[b][1,4]dioxepin-7-yl)piperazin-1-yl)butyl)benzofuran-3-ylcarbamid
103: N-(4-(4-(3,4-Dihydro-2H-benzo[b][1,4]dioxepin-7-yl)piperazin-1-yl)butyl)indol-3-ylcarbamid

Eine andere Ausführungsform der Erfindung betrifft Verbindungen der Formel VI, in der die Gruppe X mit der allgemeinen Formel X1 oder X2 in 2-Position an das bizyklische Heteroaryl der allgemeinen Formel 1 gebunden ist: 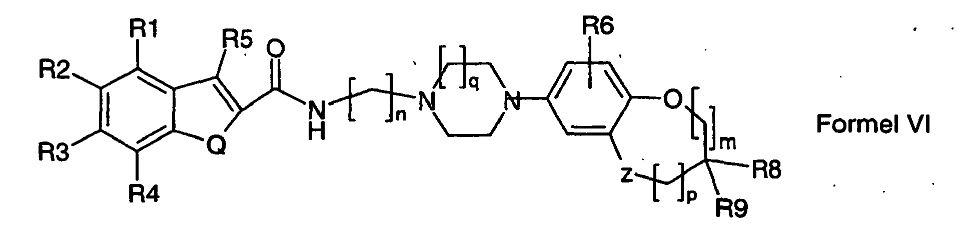 worin R, R1, R2, R3, R4, R5, R6, R8, R9, Q, Z, m, p und q die Bedeutung haben, wie weiter oben definiert und worin n den Wert 2, 3, 4 oder 5 hat.

In bevorzugten Ausführungsformen haben R, R1, R2, R3, R4, R5, R6, R8, R9, Q, Z, n, m, p und q die Bedeutung, wie vorstehend bei den bevorzugten Verbindungen der Formeln II, III und IV beschrieben.

Beispielhafte Verbindungen der Formel VI entsprechend der vorliegenden Erfindung sind: 21: N-(4-(4-(2,3-Dihydrobenzofuran-5-yl)piperazin-1-yl)butyl)benzo[b]thiophen-2-ylcarbamid
112: N-(4-(4-(2,3-Dihydrobenzofuran-5-yl)piperazin-1-yl)butyl)-5-cyanbenzo[b]thiophen-2-ylcarbamid
114: N-(4-(4-(2,3-Dihydrobenzofuran-5-yl)piperazin-1-yl)butyl)benzofuran-2-ytcarbamid
115: N-(4-(4-(2,3-Dihydrobenzofuran-5-yl)piperazin-1-yl)butyl)-5-brombenzofuran-2-ylcarbamid
117: N-(4-(4-(2,3-Dihydrobenzofuran-5-yl)piperazin-1-yl)butyl)indol-2-ylcarbamid
118: N-(4-(4-(2,3-Dihydrobenzofuran-5-yl)piperazin-1-yl)butyl)-6-cyanindol-2-ylcarbamid
22: N-(4-(4-(Chroman-6-yl)piperazin-1-yl)butyl)benzo[b]thiophen-2-ylcarbamid
120: N-(4-(4-(Chroman-6-yl)piperazin-1-yl)butyl)-5-cyanbenzo[b]thiophen-2-ylcarbamid
122: N-(4-(4-(Chroman-6-yl)piperazin-1-yl)butyl)benzofuran-2-ylcarbamid
123: N-(4-(4-(Chroman-6-yl)piperazin-1-yl)butyl)-5-brombenzofuran-2-ylcarbamid
125: N-(4-(4-(Chroman-6-yl)piperazin-1-yl)butylindol-2-ylcarbamid
126: N-(4-(4-(Chroman-6-yl)piperazin-1-yl)butyl)-6-cyanindol-2-ylcarbamid
23: N-(4-(4-(2,3,4,5-Tetrahydrobenzo[b]oxepin-7-yl)piperazin-1-yl)butyl)benzo[b]thiophen-2-ylcarbamid
128: N-(4-(4-(2,3,4,5-Tetrahydrobenzo[b]oxepin-7-yl)piperazin-1-y1)buiyl)-5-cyanbenzo[b]thiophen-2-ylcarbamid
130: N-(4-(4-(2,3,4,5-Tetrahydrobenzo[b]oxepin-7-yl)piperazin-1-yl)buiyl)benzofuran-2-ylcarbamid
131: N-(4-(4-(2,3,4,5-Tetrahydrobenzo[b]oxepin-7-yl)piperazin-1-yl)butyl)-5-brombenzofuran-2-ylcarbamid
133: N-(4-(4-(2,3,4,5-Tetrahydrobenzo[b]oxepin-7-yl)piperazin-1-yl)butyl)indol-2-ylcarbamid
134: N-(4-(4-(2,3,4,5-Tetrahydrobenzo[b]oxepin-7-yl)piperazin-1-yl)butyl)-6-cyanindol-2-ylcarbamid

Eine andere Ausführungsform der Erfindung betrifft Verbindungen der Formel VII, in der die Gruppe X mit der allgemeinen Formel X1 oder X2 in 3-Position an das bizyklische Heteroaryl der allgemeinen Formel I gebunden ist: 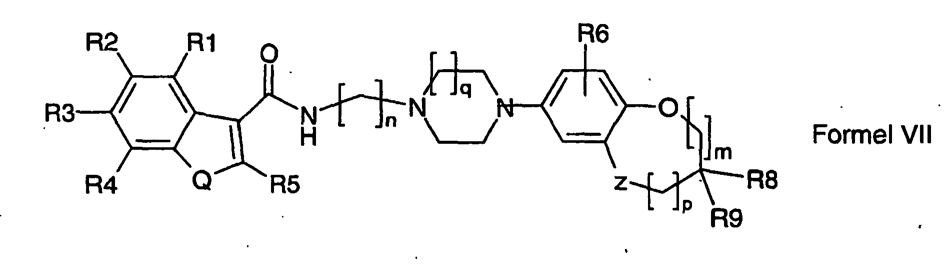 worin R, R1, R2, R3, R4, R5, R6, R8, R9, Q, Z, m, p und q die Bedeutung haben, wie weiter oben definiert und worin n den Wert 2, 3, 4 oder 5 hat.

In bevorzugten Ausführungsformen der Formel VII haben R, R1, R2, R3, R4, R5, R6, R8, R9, Q, Z, n, m, p und q die Bedeutung, wie vorstehend bei den bevorzugten Verbindungen der Formeln II, III und IV beschrieben.

Beispielhafte Verbindungen entsprechend Formel VII der vorliegenden Erfindung sind ausgewählt aus:
113: N-(4-(4-(2,3-Dihydrobenzofuran-5-yl)piperazin-1-yl)butyl)benzo[b]thiophen-3-ylcarbamid
116: N-(4-(4-(2,3-Dihydrobenzofuran-5-yl)piperazin-1-yl)butyl)benzofuran-3-ylcarbamid
119: N-(4-(4-(2,3-Dihydrobenzofuran-5-yl)piperazin-1-yl)butyl)indol-3-ylcarbamid
121: N-(4-(4-(Chroman-6-yl)piperazin-1-yl)butyl)benzo[b]thiophen-3-ylcarbamid
124: N-(4-(4-(Chroman-6-yl)piperazin-1-yl)butyl)benzofuran-3-ylcarbamid
127: N-(4-(4-(Chroman-6-yl)piperazin-1-yl)butyl)indol-3-ylcarbamid
129: N-(4-(4-(2,3,4,5-Tetrahydrobenzo[b]oxepin-7-yl)piperazin-1-yl)butyl)benzo[b]thiophen-3-ylcarbamid
132: N-(4-(4-(2,3,4,5-Tetrahydrobenzo[b]oxepin-7-yl)piperazin-1-yl)butyl)benzofuran-3-ylcarbamid
135: N-(4-(4-(2,3,4,5-Tetrahydrobenzo[b]oxepin-7-yl)piperazin-1-yl)butyl)indol-3-ylcarbamid

Die Erfindung betrifft auch physiologisch akzeptable Salze der erfindungsgemäßen Verbindungen. Beispiele für solche Salze sind in den nachstehenden Definitionen beschrieben.

Dem Fachmann ist ferner klar, dass je nach Wahl der Substituenten geometrische isomere und/oder optisch aktive Verbindungen entstehen können. In diesem Fall sind sowohl die Isomere, Razemate als auch die jeweiligen reinen enantiomeren bzw. gegebenenfalls diastereomeren Formen Gegenstand der vorliegenden Erfindung.

Die in der Beschreibung und in den anliegenden Patentansprüchen genannten Substituenten umfassen insbesondere die nachfolgend erläuterten Gruppen.

"Alkyl" kann eine verzweigte oder unverzweigte Alkylgruppe sein, die vorzugsweise 1 bis 10 C-Atome, besonders bevorzugt 1 bis 6 C-Atome ("C1-C6 Alkyl") und ganz besonders bevorzugt 1, 2 oder 3 C-Atome aufweist. "C1-C6 Alkyl" umfasst z.B. Methyl, Ethyl, n-Propyl, iso-Propyl, n-Butyl, iso-Butyl, s-Butyl, t-Butyl, n-Pentyl, iso-Pentyl, Neopentyl, t-Pentyl, 1-Methylbutyl, 2-Methylbutyl, 1-Ethylpropyl, 1,2-Dimethylpropyl und n-Hexyl. "Alkyl" kann auch zyklisch sein oder einen zyklischen Teil enthalten, wobei Zyklen mit 3-7 C-Atomen bevorzugt werden, z.B. Cyclopropyl, Cyclobutyl, Cyclopentyl, Cyclohexyl oder Cycloheptyl. Bevorzugt ist "Alkyl" nicht zyklisch und enthält keinen zyklischen Teil. Alkylgruppen können zusätzlich mit einem oder mehreren Substituenten substituiert sein, insbesondere mit Hydroxy oder Amin. Bevorzugt ist "Alkyl" unsubstituiert oder mit Hydroxy oder Alkyloxy substituiert.

"Alkenyl" und "Alkinyl" weisen mindestens eine Doppel- bzw. Dreifachbindung auf. Sie können verzweigt oder unverzweigt sein und weisen vorzugsweise 2 bis 6 C-Atome auf. Alkenyle oder Alkinyle sind vorzugsweise so an den Heteroaren- oder Phenylring des Grundgerüsts der Verbindung gebunden, dass die Doppel- bzw. Dreifachbindung mit dem aromatischen Ring konjugiert ist. Alkenyl und Alkinyl können zusätzlich mit einem oder mehreren Substituenten substituiert sein, vorzugsweise mit Phenyl, wobei sich die Phenylgruppe dann besonders bevorzugt am C-Atom 2 befindet (wenn Alkenyl oder Alkinyl über das C-Atom 1 an den Heteroaren- oder Phenylring des Grundgerüsts gebunden ist). Bevorzugt sind die Alkenyle oder Alkinyle unsubstituiert.

"Alkyloxy" ist die Gruppe -O-Alkyl, worin Alkyl vorzugsweise aus den oben für "Alkyl" angegebenen Gruppen ausgewählt ist. Bevorzugt ist "Alkyloxy" eine C1-C6-Alkyloxygruppe, besonders bevorzugt Methoxy.

"Alkylthio" ist die Gruppe -S-Alkyl, worin Alkyl vorzugsweise aus den oben für "Alkyl" angegebenen Gruppen ausgewählt ist. Bevorzugt ist "Alkylthio" eine C1-C6-Alkyl-S-Gruppe.

"Alkylaminosulfonyl" umfasst die Gruppen -SO₂-NH-Alkyl und -SO₂-N-Dialkyl, worin Alkyl vorzugsweise aus den oben für "Alkyl" angegebenen Gruppen ausgewählt ist. Bevorzugt ist "Alkyl" in "Alkylaminosulfonyl" eine C1-C6-Alkylgruppe. Beispiele für "Alkylaminosulfonyl" sind z.B. Methylaminosulfonyl, N,N-Dimethylaminosulfonyl oder Butylaminosulfonyl.

"Alkylsulfonylamino" ist die Gruppe -NH-SO₂-Alkyl, worin Alkyl vorzugsweise aus den oben für "Alkyl" angegebenen Gruppen ausgewählt ist. Bevorzugt ist "Alkylsulfonylamino" eine C1-C6-Alkylsulfonylaminogruppe, z.B. Methansulfonylamino.

"Amino" umfasst primäre, sekundäre oder tertiäre Amine. Sekundäre oder tertiäre Amine können Substituenten aus der Gruppe Alkyl oder Phenylalkyl tragen. Alkyl kann zusätzlich Hydroxy oder Alkyloxy tragen. Amino ist insbesondere ein primäres, d.h. ausschließlich mit Wasserstoffen substituiertes Amin.

"Phenyl" ist bevorzugt unsubstituiert, kann aber gegebenenfalls ein oder mehrfach unabhängig substituiert sein, z.B. mit Alkoxy, Alkyl, Trifluormethyl oder Halogen.

"Phenylalkyl" ist die Gruppe -Alkyl-Phenyl, wobei Phenyl und Alkyl die Bedeutung haben, wie vorstehend definiert. Phenylalkyl umfasst beispielsweise Phenylethyl und Benzyl und ist bevorzugt Benzyl.

"Phenoxy" ist die Gruppe -O-Phenyl, worin Phenyl die Bedeutung hat, wie weiter vorstehend definiert.

"Alkylcarbonyl" umfasst die Gruppe -C(O)-Alkyl, worin Alkyl vorzugsweise aus den oben für "Alkyl" angegebenen Gruppen ausgewählt ist, und besonders bevorzugt -C(O)-C1-C6- , Alkyl ist. "Alkylcarbonyl" ist vorzugsweise Acetyl, Propionyl oder Butyryl.

"Phenylcarbonyl" ist -C(O)-Phenyl, worin Phenyl die Bedeutung hat, wie weiter oben definiert

"Phenylalkylcarbonyl" ist -C(O)-Alkyl-Phenyl, worin Alkyl und Phenyl die Bedeutung haben wie weiter oben definiert.

"Alkyloxycarbonyl" ist die Gruppe -C(O)-O-Alkyl, worin Alkyl vorzugsweise aus den oben für "Alkyl" angegebenen Gruppen ausgewählt ist. Bevorzugt ist "Alkoxycarbonyl" eine (C1-C6-Alkyl)oxycarbonylgruppe.

"Phenylalkyloxycarbonyl" ist die Gruppe -C(O)-O-Alkyl-Phenyl, worin Alkyl und Phenyl die Bedeutung haben wie weiter oben definiert.

"Halogen" umfasst Fluor, Chlor, Brom und lod, und ist bevorzugt Fluor, Chlor oder Brom.

"Sulfamoyl" umfasst die Gruppe -SO₂-NH₂.

"Sulfonylamino" umfasst die Gruppe -NH-SO₂H.

"Physiologisch akzeptable Salze" schließen nicht-toxische Additionssalze einer Base, insbesondere einer Verbindung der Formeln (1) bis (VII) in Form der freien Base, mit organischen oder anorganischen Säuren ein. Beispiele für anorganische Säuren schließen HCl, HBr, Schwefelsäure und Phosphorsäure ein. Organische Säuren schließen Essigsäure, Propionsäure, Brenztraubensäure, Buttersäure, α-, β- oder γ-Hydroxybuttersäure, Valeriansäure, Hydroxyvaleriansäure, Capronsäure, Hydroxycapronsäure, Caprylsäure, Caprinsäure, Laurinsäure, Myristinsäure, Palmitinsäure, Stearinsäure, Glykolsäure, Milchsäure, D-Glucuronsäure, L-Glucuronsäure, D-Galacturonsäure, Glycin, Benzoesäure, Hydroxybenzoesäüre, Gallussäure, Salicylsäure, Vanillinsäure, Cumarsäure, Kaffeesäure, Hippursäure, Orotsäure, L-Weinsäure, D-Weinsäure, D,L-Weinsäure, meso-Weinsäure, Fumarsäure, L-Äpfelsäure, D-Äpfelsäure, D,L-Äpfelsäure, Oxalsäure, Malonsäure, Bernsteinsäure, Maleinsäure, Oxalessigsäure, Glutarsäure, Hydroxyglutarsäure, Ketoglutarsäure, Adipinsäure, Ketoadipinsäure, Pimelinsäure, Glutaminsäure, Asparaginsäure, Phthalsäure, Propantricarbonsäure, Zitronensäure, Isozitronensäure, Methansulfonsäüre, Toluolsulfonsäure, Benzolsulfonsäure, Camphersulfonsäure, Embonsäure und Trifluormethansulfonsäure ein.

Verbindungen der Formeln (I) bis (VII) wie definiert, sind als Arzneimittel geeignet. Die erfindungsgemäßen Verbindungen umfassen affine oder sogar hochaffine Liganden für D3-Rezeptoren.

Der Begriff "affiner D3-Ligand" umfasst Verbindungen, die in einem Radioligandexperiment (vgl. Hübner, H. et al. J. Med. Chem. 2000, 43, 756-762 sowie der Abschnitt "Biologische Aktivität") Bindung an humane Dopamin D3-Rezeptoren mit einem Ki-Wert von nicht mehr als 500 nM zeigen Für "affine" Liganden anderer Rezeptoren gilt die Definition entsprechend.

Der Begriff "hochaffine D3-Liganden" umfasst Verbindungen, die in einem Radioligandexperiment (vgl. Hübner, H. et al. J. Med. Chem. 2000, 43, 756-762 sowie der Abschnitt "Biologische Aktivität") Bindung an humane Dopamin D3-Rezeptoren mit einem Ki-Wert von vorzugsweise nicht mehr als etwa 30 nM, besonders bevorzugt nicht mehr als 3 nM zeigen. Für "hochaffine" Liganden anderer Rezeptoren gilt die Definition entsprechend.

Ein Aspekt der vorliegenden Erfindung betrifft selektive D3-Liganden. Der Begriff "selektive D3-Liganden" umfasst Verbindungen, die im Radioligandexperiment für den D3-Rezeptor, wie im nachfolgenden Abschnitt "Biologische Aktivität" beschrieben, einen Ki-Wert aufweisen, der um einen Faktor von zumindest 10 niedriger als für mindestens fünf der sieben folgenden Rezeptoren ist: Dopamin-Rezeptoren D1, D2long, D2short und D4.4, Serotonin-Rezeptoren 5-HT1a und 5-HT2 und alpha1 Adrenozeptor.

Ein anderer Aspekt der Erfindung betrifft hochselektive Dopamin D3-Liganden. Der Begriff "hochselektive D3-Liganden" umfasst Verbindungen, die im Radioligandexperiment für den D3-Rezeptor, wie im nachfolgenden Abschnitt "Biologische Aktivität" beschrieben, einen Ki-Wert aufweisen, der um einen Faktor von zumindest 100 niedriger als für mindestens fünf der sieben folgenden Rezeptoren ist: Dopamin-Rezeptoren D1, D2long, D2short und D4.4, Serotonin-Rezeptoren 5-HT1a und 5-HT2 und alpha1 Adrenozeptor.

Für "affine" oder "hochaffine" Liganden des 5-HT1 a- oder alpha 1-Adrenozeptors gelten entsprechende Definitionen.

D3-Liganden können am D3-Rezeptor agonistische, antagonistische oder partialagonistische Wirkung haben. Die entsprechenden intrinsischen Aktivitäten der erfindungsgemäßen Verbindungen lassen sich in Mitogeneseassays messen, wie in der Literatur beschrieben (Hübner, H. et al. J. Med. Chem. 2000, 43, 4563-4569 und Löber, S. Bioorg. Med . Chem. Lett. 2002, 12.17, 2377-2380). In Abhängigkeit von der Pathophysiologie der zugrunde liegenden Erkrankung kann therapeutisch eine stärker agonistische, stärker antagonistische oder eine partialagonistische Aktivität gewünscht sein.

Beispielsweise werden zur Behandlung der idiopathischen Parkinson-Erkrankung häufig Dopamin-Modulatoren mit stark agonistischem Anteil gewünscht, während bei der Behandlung der Schizophrenie in der Regel reine Antagonisten zum Einsatz kommen.

Partielle 03-Agonisten haben dagegen beispielsweise Potential bei der Behandlung von L-DOPA induzierten Dyskinesien.

Schließlich weisen einige der erfindungsgemäßen Substanzen auch signifikante Affinität für weitere pharmakologisch interessante Rezeptoren, wie z.B. den Serotonin-Rezeptor, insbesondere den 5-HT1a-Rezeptor, oder den adrenergen alpha1-Rezeptor auf.

Anstelle einer hochselektiven Dopamin D3-Rezeptorbindung kann je nach Art der zu behandelnen Erkrankung auch eine Bindung an einen weiteren Rezeptor gewünscht sein.

Beispielsweise kann zur Behandlung der Schizophrenie eine Verbindung attraktiv sein, die ein hochaffiner D3-Ligand und gleichzeitig ein affiner oder sogar hochaffiner 5-HT1a-Rezeptortigand ist. In einer anderen Ausführungsform der Erfindung kann zur Behandlung von Dyskinesien eine Verbindung gewünscht sein, die neben D3-modulatorischen Eigenschaften auch D2-agonistische, alpha 1- und/oder 5-HT1a-modulatorische Eigenschaften aufweist.

Die vorliegende Erfindung erlaubt daher eine Feineinstellung bzw. eine gezielte Auswahl der gewünschten Affinität, Aktivität und Selektivität bezüglich verschiedener pharmakologisch bedeutsamer Rezeptoren insbesondere der Dopamin D3- Rezeptoren, aber auch beispielsweise bezüglich des 5-HT1 a-Rezeptors oder des D2-Rezeptors, .

Ein weiterer Gegenstand der Erfindung ist daher ein Arzneimittel, das eine oder mehrere der Verbindungen der allgemeinen Formeln (I) bis (VII) oder eine der konkret aufgeführten Verbindungen wie oben definiert, gegebenenfalls in Form eines pharmazeutisch akzeptablen Salzes sowie eines pharmazeutisch akzeptablen Hilfsmittels enthält.

Die Erfindung betrifft auch die Verwendung einer oder mehrerer der Verbindungen der allgemeinen Formeln (I) bis (VII) oder einer der konkret aufgeführten Verbindungen, gegebenenfalls in Form eines pharmazeutisch akzeptablen Salzes, zur Herstellung eines Arzneimittels für die hier genannten Indikationen.

Der Begriff "Behandlung" einer Erkrankung umfasst in dies,er Patentanmeldung (a) die Therapie einer bereits bestehenden Erkrankung sowie (b) die Prophylaxe einer hoch nicht oder noch nicht vollständig ausgeprägten Erkrankung, wenn für das Auftreten einer solchen Erkrankung ein Risiko besteht.

Bevorzugt werden zur Herstellung von Arzneimitteln solche erfindungsgemäßen Verbindungen ausgewählt, die hochaffine D3-Liganden sind. Besonders bevorzugt werden selektive oder sogar hochselektive D3-Liganden verwendet.

In einer anderen Ausführungsform der Erfindung werden Verbindungen ausgewählt, die affin oder sogar hochaffin auch oder insbesondere für den adrenergen alpha 1- oder den 5-HT1 a-Rezeptor sind.

Die erfindungsgemäßen Verbindungen haben Potential in der Therapie oder Prophylaxe einer Reihe von Erkrankungen, die insbesondere mit einer Störung des Dopaminstoffwechsels oder der dopaminergen Signalkaskade, bzw. gegebenenfalls der serotonergen Signalübertragung einhergehen.

Ein Gegenstand der Erfindung ist daher die Verwendung einer erfindungsgemäßen Verbindung, wie in dieser Patentanmeldung inklusive der Ansprüche und den Beispielen beschrieben, zur Herstellung eines Arzneimittels zur Behandlung von Erkrankungen, die mit einer Störung des Dopaminstoffwechsels und/oder der dopaminergen Signalkaskade einhergehen.

Ein weiterer Gegenstand der Erfindung ist die Verwendung einer erfindungsgemäßen Verbindung, wie in dieser Patentanmeldung inklusive der Ansprüche und den Beispielen beschrieben, zur Herstellung eines Arzneimittels zur Behandlung von Erkrankungen, die mit einer Störung der adrenergen Signalkaskade einhergehen, bzw. die daraus resultieren und/oder die durch Gabe alpha-adrenerger Mimetika oder Inhibitoren therapierbar sind. Beispiele für solche Erkrankungen sind die Hypertonie oder die benigne Prostatahyperplasie.

Ein anderer Gegenstand der Erfindung ist die Verwendung einer erfindungsgemäßen Verbindung, wie in dieser Patentanmeldung inklusive der Ansprüche und den Beispielen' beschrieben, zur Herstellung eines Arzneimittels zur Behandlung von Erkrankungen, die mit einer Störung des Serotoninstoffwechsels und/oder der serotonergen Signalübertragung einhergehen.

Erkrankungen, in deren Pathogenese dopaminerge und/oder serotonerge Prozesse involviert sind, sind insbesondere Erkrankungen des zentralen Nervensystems (ZNS). Ein Gegenstand der Erfindung ist daher die Verwendung einer erfindungsgemäßen Verbindung, wie in dieser Patentanmeldung inklusive der Ansprüche und den Beispielen beschrieben, zur Herstellung eines Arzneimittels zur Behandlung von ZNS-Erkrankungen.

Der Begriff "ZNS-Erkrankungen" umfasst in dieser Patentanmeldung sowohl Störungen, die ihren Ursprung im ZNS haben und deren Symptome sich überwiegend oder ausschließlich im ZNS bemerkbar machen, wie z.B. Psychosen, Depressionen oder kognitive Störungen, als auch Erkrankungen, die ihren Ursprung im ZNS haben, deren Symptome sich aber zumindestens zum Teil in anderen Zielorganen bemerkbar machen, wie z.B. extrapyramidal-motorische Bewegungsstörungen oder Hyperprolaktinämie.

Beispiele für ZNS-Erkrankungen, die mit den erfindungsgemäßen Verbindungen behandelt werden können, sind
(1) Psychosen und Angststörungen, inklusive Manien, idiopathischen Psychosen, - Schizophrenie, Zwangsstörungen, Panikattacken, Phobien, Essstörungen, aggressive und autoagressive Störungen, Stereotypien und andere Persönlichkeitsstörungen
(2) Drogenabhängigkeit, z.B. Kokain-, Alkohol-, Opiat- und Nikotinsucht;
(3) Stimmungsstörungen, z.B. depressive Störungen insbesondere "major depression", manisch-depressive Störungen, organisch-bedingte Depressionen, z.B. im Zusammenhang mit neurodegenerativen Erkrankungen wie Morbus Parkinson oder Alzheimer
(4) Bewegungsstörungen, inklusive Tremor, Rigor, Dyskinesien, Dystonien, wie bei Morbus Parkinson, Parkinsonismus (idiopathisch, z.B. bei Parkinson-Plus-Syndrom oder Medikamenten-induziert, z.B. nach L-Dopa oder Neuroleptika-Behandlung), Segawa-Syndrom, Tourette-Syndrom, Restless-Leg Syndrom
(5) Schlafstörungen, inklusive durch Dopaminagonisten ausgelöste Narkolepsie oder Morbus Parkinson-assoziierte Schlafstörungen
(6) Übelkeit: hier können Dopaminantagonisten entweder alleine oder in Kombination mit 5-HT3 Antagonisten eingesetzt werden
(7) Kognitive Störungen und Demenzerkrankungen
(8) Hyperprolaktinämie; Hyperprolaktinom sowie bei Medikamenten-unterstütztem Abstillen nach Schwangerschaften
(9) Glaukoma
(10) Hyperaktivitätssyndrom (ADHS);
(11) Autismus, bzw. mit Autismus verbundene Störungen, insbesondere bei Verbindungen mit ausgeprägter serotonerger Wirkkomponente
(12) Schlaganfall, insbesondere bei Verbindungen mit ausgeprägter serotonerger Wirkkomponente

Als weitere therapeutische Anwendung kann die Behandlung und Vorbeugung von neurodegenerativen Erkrankungen genannt werden, da die Substanzen auf Grund ihrer neuroprotektiven Wirkung die Zerstörung oder den Verlust von Neuronen als Ursache oder Folge eines pathophysiologischen Ereignisses verzögern oder zum Stillstand bringen können. Solche Erkrankungen sind beispielsweise die amyotrope Lateralsklerose, die Alzheimersche Erkrankung, Chorea Huntington, Epilepsie, Morbus Parkinson oder Synucleopathien, z.B. vom Typ des Parkinson-Plus-Syndroms.

Neben der Behandlung von Erkrankung, die eindeutig unter Beteiligung des ZNS entstehen und/oder verlaufen, können die erfindungsgemäßen Substanzen auch zur Behandlung weiterer Erkrankungen verwendet werden, die nicht, nicht eindeutig oder nicht ausschließlich ZNS-assoziiert sind. Solche Erkrankungen sind insbesondere Schmerzformen oder Störungen des Urinaltrakts, wie z.B. sexuelle Dysfunktion, insbesondere männliche erektile Dysfunktion und urinale Inkontinenz. Zur Behandlung urinaler Inkontinenz sind insbesondere Verbindungen mit ausgeprägter serotonerger Wirkkomponente geeignet.

Ein Gegenstand der Erfindung ist daher die Verwendung einer erfindungsgemäßen Verbindung zur Herstellung eines Arzneimittels zur Behandlung von Schmerzen öder von Erkrankungen des Urinaltrakts, insbesondere von männlicher erektiler Dysfunktion und urinaler Inkontinenz.

Erkrankungen, für die die erfindungsgemäßen Verbindungen besonders geeignet sind, sind Schizophrenie, depressive Störungen, L-Dopa- oder Neuroleptika-induzierte Bewegungsstörungen, Morbus Parkinson, Segawa-Syndrom, Restless-Leg-Syndrom, Hyperprolaktinämie, Hyperprolaktinom, Hyperaktivitätssyndrom (ADHS) und urinale Inkontinenz.

Bewegungsstörungen, die der Therapie mit den erfindungsgemäßen Substanzen besonders zugänglich sind, sind insbesondere
- Morbus Parkinson-assoziierte Bewegungsstörungen, z.B. Rigor, Tremor, Dystonie und Dyskinesie,
- Segawa-Syndrom
- Neuroleptika-induzierte (tardive) extrapyramidalmotorische Bewegungsstörungen, insbesondere Dyskinesie, Dystonie und Akathisie,
- L-Dopa-induzierte extrapyramidalmotorische Bewegungsstörungen, insbesondere Dyskinesien und Dystonien
- Restless Leg Syndrom

Schließlich können die erfindungsgemäßen Arzneimittel in Abhängigkeit von der zu behandelnden Erkrankung auch als Kombinationspräparat-zur gleichzeitigen oder sequentiellen Gabe ausgebildet sein.

Beispielsweise kann eine Verkaufseinheit, die eine zur Behandlung der Parkinson-Erkrankung enthaltende L-Dopa Medikation enthält, auch eine pharmazeutische Zusammensetzung umfassen, die eine oder mehrere der erfindungsgemäßen Verbindungen mit z.B. hochselektivem, partialagonistischem dopaminergem und/oder serotonergem Wirkprofil enthält. Dabei können L-Dopa und die erfindungsgemäße Verbindung in der gleichen pharmazeutischen Formulierung, z.B. einer Kombinationstablette, oder auch in unterschiedlichen Applikationseinheiten vorliegen, z.B. in Form zweier separater Tabletten. Je nach Bedarf können beide Wirkstoffe gleichzeitig oder zeitlich getrennt verabreicht werden.

In einem Kombinationspräparat kann eine sequentielle Gabe beispielsweise erreicht werden, indem eine Darreichungsform, z.B. eine orale Tablette, zwei unterschiedliche Schichten mit differierendem Freisetzungsprofil für die verschiedenen pharmazeutisch aktiven Bestandteile aufweist. Dem Fachmann ist klar, dass im Kontext der vorliegenden Erfindung verschiedene Darreichungsformen und Applikatiorisschemata denkbar sind, die alle Gegenstand der Erfindung sind.

Eine Ausführungsform der Erfindung betrifft daher ein Arzneimittel, das L-Dopa oder ein Neuroleptikum sowie eine erfindungsgemäße Verbindung zur gleichzeitigen oder zeitlich aufeinanderfolgenden Verabreichung an den Patienten enthält.

In einer anderen Ausführungsform der Erfindung kann die Verkaufseinheit ein Kombinationspräparat sein oder zwei Applikationseinheiten enthalten, die zwei der erfindungsgemäßen Verbindungen mit unterschiedlichem Rezeptorprofil, z.B. einen hochaffinen, hochselektiven D3-Modulator und einen hochaffinen 5-HT1 a-Modulator enthalten.

Ein weiterer Gegenstand der Erfindung ist eine Methode zur Behandlung einer Erkrankung ausgewählt aus den weiter oben aufgeführten Erkrankungen, durch Verabreichung einer oder mehrerer der erfindungsgemäßen Verbindungen, jeweils alleine oder in Kombination mit anderen Arzneimitteln an einen Säuger, der einer solchen Behandlung bedarf, wobei der Begriff "Säuger" auch und insbesondere Menschen umfasst.

Üblicherweise bestehen die erfindungsgemäßen Arzneimittel aus einer pharmazeutischen Zusammensetzung, die neben den erfindungsgemäßen Verbindungen, wie oben beschrieben, mindestens einen pharmazeutisch annehmbaren Träger oder Hilfsstoff enthält.

Dem Fachmann ist klar, dass die pharmazeutische Formulierung in Abhängigkeit vom beabsichtigten Applikationsweg unterschiedlich ausgestaltet sein kann. So kann die pharmazeutische Formulierung beispielsweise zur intravenösen, intramuskulären, intrakutanen, subkutanen, oralen, bukkalen, sublingualen, nasalen, transdermalen, inhalativen, rektalen oder intraperitonealen Verabreichung angepasst sein.

Entsprechende Formulierungen und hierfür geeignete pharmazeutische Träger bzw. Hilfsstoffe, wie Füllstoffe, Sprengmittel, Bindemittel, Gleitmittel, Stabilisatoren, Aromastoffe, Antioxidantien, Konservierungsmittel, Dispersions- oder Lösungsmittel; Puffer oder Elektrolyte, sind dem Fachmann auf dem Gebiet der Pharmazeutik bekannt und sind beispielsweise in Standardwerken wie Sucker, Fuchs und Speiser ("Pharmazeutische Technologie", Deutscher Apotheker Verlag, 1991) und Remington ("The Science and Practice of Pharmacy", Lippincott, Williams & Wilkins, 2000) beschrieben.

In einer bevorzugten Ausführungsform-der Erfindung werden die pharmazeutischen Zusammensetzungen, die die erfindungsgemäßen Verbindungen enthalten, oral verabreicht und können beispielsweise als Kapsel, Tablette, Pulver, Granulat, Dragee oder in flüssiger Form vorliegen.

Dabei kann die Formulierung als schnell freisetzende Darreichungsform ausgestaltet sein, wenn ein rascher Wirkeintritt gewünscht ist. Entsprechende orale Formulierungen sind beispielsweise beschrieben in EP 0 548 356 oder EP 1 126 821.

Ist dagegen eine protrahierte Freisetzung erwünscht, bietet sich eine Formulierung mit retardierter Wirkstofffreisetzung an. Entsprechende orale Formulierungen sind ebenfalls aus dem Stand der Technik bekannt.

Alternative pharmazeutische Zubereitungen können beispielsweise Infusions- oder Injektionslösungen, Öle, Suppositorien, Aerosole, Sprays, Pflaster, Mikrokapseln oder Mikropartikel sein.

Die Verbindungen der Formeln I bis VII werden nach Methoden hergestellt, die teilweise bereits in der Literatur beschrieben sind (Bettinetti, L. et al. J. Med. Chem. 2002, 45, 4594-4597). Dazu werden die Säurederivate vom Typ (A), die entweder käuflich erworben, nach Literaturvorschrift synthetisiert oder deren Herstellungsmethoden in unseren Labors ausgearbeitet worden sind, in Form ihrer Carbonsäurechloride oder alternativ bei Einsatz der Carbonsäuren durch Verwendung spezieller Aktivierungsreagenzien wie zum Beispiel Hydroxybenzotriazol, Hydroxyazabenzotriazol, HATU (Kienhöfer, A. Synlett 2001, 1811-1812) oder TBTU (Knorr, R. Tetrahedron Lett. 1989, 30, 1927-1930) aktiviert und mit der freien Base vom Typ (C) zu den Derivaten der Formel I bis VII umgesetzt.

Die Herstellung der erfindungsgemäßen Verbindungen erfolgt durch Umsetzung eines Säurederivats A 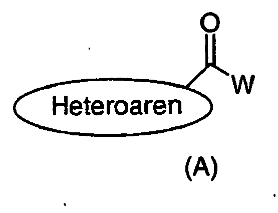 mit einer freien Base der allgemeinen Formel C 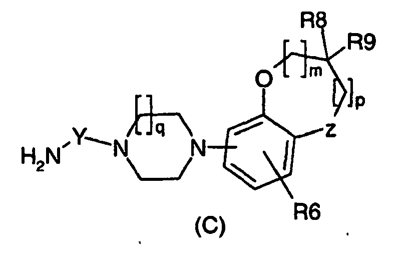 wobei gilt:
W ist ausgewählt aus OH, Cl, Br oder einer Gruppe

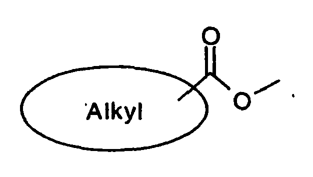

Heteroaren steht für eine Gruppe der allgemeinen Formel la 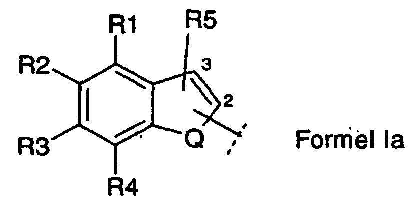
wobei R, R1, R2, R3, R4, R5, R6, R8, R9, Q, Z, m, p und q die Bedeutung haben, wie weiter oben definiert und wobei die durchkreuzte Bindung im Heteroaren für die Bindung der Gruppe -C(O)-W an die 2- oder 3-Position des besagten Heteroarens mit der Formel la steht;
und wobei für den Fall, dass der Substituent W eine Hydroxygruppe ist, die entsprechende Säuregruppe vor der Umsetzung mit der freien Base der allgemeinen Formel C durch Zugabe von Aktivierungsreagenzien, wie z.B. Hydroxybenzotriazol, Hydroxyazabenzotriazol, HATU oder TBTU aktiviert wird.
W ist bevorzugt Chlor, Brom oder OH und besonders bevorzugt Chlor oder OH.

### SYNTHESE DER AUSFÜHRUNGSBEISPIELE:

### Zugang zu den käuflich erwerbbaren Heteroarencarbonsäuren (hier als "Typ A1". bezeichnet):

### Benzo[b]thiophen-2-earbonsäure; 5-Brombenzo[b]thiophen 2-carbonsäure; Benzo[b]thiophen-3-carbonsäure; Benzofuran-2-carbonsäure; Indol-2-carbonsäure; Indol-3-carbonsäure; 3-Chlorbenzo[b]thiophen-2-carbonsäureehlorid

Heteroarencarbonsäuren vom Typ A1 sind käuflich erwerbbar, wie z.B. Benzo[b]thiophen-2-carbonsäure (z.B. von *Aldrich, Taufkirchen;* Nr: 46,746-4); 5-Brombenzo[b]thiophen-2-carbonsäure (z.B. von *Maybridge, Tintangel, UK;* Nr: CC 31201); Benzo[b]thiophen-3-carbonsäure (z.B. von *Maybridge, Tintangel, UK;* Nr: CC 12301); Benzofuran-2-carbonsäure (z.B. von *Aldrich, Taufkirchen;* Nr: 30,727-0); Indol-2-carbonsäure (z.B. von *Aldrich, Taufkirchen;* Nr: 1-510-9) oder Indol-3-carbonsäure (z.B. von *Aldrich, Taufkirchen;* Nr: 28,473-4). Derivate der Carbonsäuren, wie das 3-Chlorbenzo[b]thiophen-2-carbonsäurechlorid (z.B. von *Maybridge, Tintangel, UK;* Nr. BTB 00300), können ebenfalls käuflich erworben werden.

### De novo Synthese von Heteroarencarbonsäuren (hier als Typ "A2" bezeichnet):

### Benzofuran-3-carbonsäure; 6-Cyanindol-2-carbonsäure; 5-Cyanbenzolblthiophen-2-carbonsäure; 6-Ethinylbenzo[b]thiophen-2-carbonsäure

Die Heteroarencarbonsäuren des Typs A2 wurden in unseren Labors wie nachfolgend beschrieben hergestellt.

### Benzofuran-3-carbonsäure

Benzofuran-2,3-dicarbonsäure (2,06 g; 10,0 mmol) *(Aldrich, Taufkirchen;* Nr: 64,274-6) wird mit Kupferpulver (1,15 g;18,1 mmol) und Chinolin (2,0 g; 15,48 mmol) zusammen für 2 Stunden bei 195°C im Ölbad gerührt. Nach dem Abkühlen nimmt man in Dichlormethan auf, saugt durch eine Glasfritte, und wäscht den Rüchstand mit Dichlormethan nach. Das Filtrat wird am Rotationsverdampfer abgedampft, und der Rüchstand durch Flashchromatographie gereinigt (CH₂Cl₂-MeOH: 95-5 mit 5% HCOOH) anschließend (CH₂Cl₂MeOH: 98-2 mit 5% HCOOH)
Ausbeute: 845 mg (52%)
MS m/z 162 (M⁺). ¹HNMR (CDCl₃, 360MHz) ∂ (ppm): 7,38-7,45 (m, 2H, H-5, H-6), 7,55-7,61 (m, 1 H, H-7), 8,10-8,16 (m, 1 H, H-4), 8,39 (s, 1 H, H-2).

### 6-Cyanindol-2-carbonsäure

Der nach Literatur (Dann, O.; Wolff, H. P.; Schlee, R.; Ruff, J. Liebigs Annalen der Chemie, 1986, 2164-2178) hergestellte 6-Cyanindol-2-carbonsäuremethylester (0,05 g (0,24 mmol)) wird in 5 ml Methanol gelöst. Anschließend wird mit 2,5 ml 2n NaOH versetzt und 16 Stunden bei Raumtemperatur gerührt. Die Reaktionslösung wird am Rotationsverdampfer eingeengt und mit Wasser verdünnt, anschließend mit Hexan gewaschen, mit HCl auf pH 3-4 eingestellt und in Diethylether aufgenommen. Nach Trocknung mit MgSO₄ wird das Lösungsmittel abgedampft.
Ausbeute: 0,04 g (87%).
MS: m/z 187 ((M+H)⁺).

### 5-Cyanbenzo[b]thiophen-2-carbonsäure

Der nach Literatur (Bridges, A. J.; Lee, A.; Maduakor, E. C.; Schwartz, C. E. Tetrahedron Letters, 1992, 33, 7499-7502) hergestellte 5-Cyanbenzo[b]thiophen-2-carbonsäuremethylester (0,08 g, 0,36 mmol) wird in 8 ml THF gelöst und auf 0 °C gekühlt. Anschließend wird Methanol (8 ml) und 2N NaOH (4 ml) zugetropft und 4 Stunden bei Raumtemperatur gerührt. Danach wird mit Wasser und THF verdünnt, die organischen Lösungsmittel abrotiert und anschließend die wässrige Phase mit Ethylacetat gewaschen. Die wässrige Phase wird mit HCl auf pH 2 eingestellt und mehrmals mit Diethylether extrahiert. Die vereinigten Etherphasen werden mit Magnesiumsulfat getrocknet und am Rotationsverdampfer abgedampft.
Ausbeute: 0,06 g (80%) weißer Feststoff
m/z 203 (M⁺). IR (NaCl) v (cm⁻1): 3375; 2359, 2226 (CN), 1676 (COOH), 1598, 1385, 1086, 720.¹H-NMR (CDCl₃, 360 MHz) δ (ppm): 3.68 (br.s, 1H, COOH), 7.86 (dd, J= 1.8 Hz, J=8.5 Hz, 1 H, H-6), 8.19 (s, 1 H, H-3), 8.30 (d, J=8.2 Hz, 1 H, H-7), 8.55 (d, J=1.4 Hz, H-4). ¹³C-NMR (CD₃OD, 90 MHz) δ (ppm): 109.9 (C-5), 119.8 (CN), 125.3 (C-7). 129.4 (C-4), 130.9 (C-6), 131.4 (C-3), 134.1 (C-3a), 140.0 (C-2), 147.4 (C-7a).

### 6-Ethinylbenzo[b]thiophen-2-carbonsäure

Der nach Literatur (Bridges, A. J.; Lee, A.; Maduakor, E. C.; Schwartz, C. E. Tetrahedron Letters, 1992, 33, 7499-7502) hergestellte 6-lodbenzo[b]thiophen-2-carbonsäuremethylester (0,15 g, 0,47 mmol) wird in trockenem THF (5 ml) unter N₂-Atmosphäre gelöst, dann fein zerteiltes Cul (3,6 mg, 0,019 mmol, 4 mol%) und PdCl₂(PPh₃)₂ (6,6 mg, 0,01 mmol, 2 mol%) zugegeben und unter Rühren NEt₃ (0,10 ml 0,70 mmol) und anschließend in 2 ml THF gelöstes Trimethylsilylacetylen (0,10 ml, 0,70 mmol) zugetropft. Nach Rühren bei Raumtemperatur für 18 h wird das Lösungsmittel am Rotationsverdampfer abgezogen und der Rückstand und flashchromatographisch (Hexan-Ethylacetat: 99-1) gereinigt.
Ausbeute: 0,11 g (81 %) weißer Feststoff
Smp.: 110 °C. MS m/z 288 (M⁺). IR (NaCl) v (cm⁻¹): 3406 (CCH), 2955, 2898, 2154 (CC), 1716 (C=O), 1250, 1250', 756. ¹H-NMR (CDCl₃, 360 MHz) δ (ppm): 0.27 (s, 9H, Si(CH₃)₃), 3.94 (s, 3H, OCH₃), 7.46 (dd, J=1.4 Hz, J=8.3 Hz, 1H, H-5), 7.78 (dd, J=0.5, J= 8.3 Hz, 1H, H-4), 7.97-7.98 (m, 1 H, H-3), 8.01 (d, J=0.9 Hz, 1 H, H-7). ¹³C-NMR (CDCl₃, 90 MHz) δ (ppm): 0.0, 0.1, 0.2, 52.6, 96.2, 104.6, 121.9, 125.2, 126.6, 128.5, 130.3, 134.7, 138.4, 141.9, 162.9. Der so hergestellte 6-Trimethylsilylethinylbenzo[*b*]thiophen-2-carbonsäuremethylester wird (23,0 mg, 0,08 mmol) wird in THF (2ml) gelöst, auf -15 °C abgekühlt und unter Rühren 1 M NH₄Bu₄F-Lösung (in THF) (0,09 ml, 0,09 mmol) zugetropft. Nach 30 Minuten wird das Reaktionsgemisch bei Raumtemperatur am Rotationsverdampfer eingeengt und auf Kieselgel aufgezogen. Reinigung mit Flashchromatographie (Hexan-Ethylacetat: 99-1) führt zu 6-Ethinylbenzo[b]thiophen-2-carbonsäurämethylester.
Ausbeute: 0,11 g (79%) weißer Feststoff
Smp.: 147 °C. MS m/z 288 (M⁺). IR (NaCI) v (cm⁻¹): 3245 (CCH), 2921 (CH₃), 2154 (CH₃), 1699 (COOCH₃), 1069, 756. ¹H-NMR (CDCl₃, 360 MHz) δ (ppm): 3.19 (s, 1 H, CCH), 3.95 (s, 3H, OCH₃), 7.49 (dd, J=1.4 Hz, J=8.4 Hz, 1H, H-6), 7.81 (dd, J=0.5 Hz, J=8.4 Hz, H-4), 8.00 (s, 1 H, H-3), 8.02 (d, J=0.7 Hz, 1 H, H-7). ¹³C-NMR (CDCl₃, 90 MHz) ö (ppm): 52.6 (OCH₃), 78.7, 83.3, 120.8, 125.3, 126.2, 128.5, 130.2, 134.9, 138.7, 141.9, 162.9.
6-Ethinylbenzo[b]thiophen-2-carbonsäüremethylester (0,08 g, 0,36 mmol) wird in 8 ml THF gelöst und auf 0 °C abgekühlt. Anschließend wird Methanol (8 ml) und 2N NaOH (4 ml) zugetropft und 4 Stunden bei Raumtemperatur gerührt. Danach wird Wasser und THF zugegeben, die organischen Lösungsmittel abgedampft und die verbleibende, wässrige Phase mit Ethylacetat gewaschen. Die wässrige Phase wird mit HCl auf pH 2 eingestellt und mehrmals mit Diethylether extrahiert. Die vereinigten Etherphasen werden mit Magnesiumsulfat getrocknet und am Rotationsverdampfer abgedampft.
Ausbeute: 0,08 g (94%) weißer Feststoff
Smp.: 214 °C. MS m/z 202 (M⁺). IR (NaCl) v (cm⁻¹ ): 3288, 2952, 2816, 2103 , 1672, 1516, 1421, 1182, 1045, 813, 758. ¹H-NMR (CD₃OD, 360 MHz) δ (ppm): 3.65 (s, 1H, CCH), 7.51 (dd, J=1.2 Hz, 8.2 Hz, 1H, H-5), 7.92 (d, J=8.2 Hz, 1H, H-4), 8.06 (s, 1H, H-3), 8.07 (s,1H, H-7). ¹³C-NMR (CDCl₃, 90 MHz) δ (ppm): 80.1, 84.2, 122.3, 126.5), 127.4, 129.5, 131.1, 137.8, 140.3, 143.4), 165.6.

### Herstellung der Amine:

Die Aminkomponenten der erfindungsgemäßen Verbindungen wurden wie nachfolgend beschrieben hergestellt, wobei die Gruppierung der Amine in die Typen "C1" bis "C9" nach chemisch-strukturellen Eigenschaften erfolgte.

### Herstellung der Amine vom Typ C1:

### 4-(4-(2,3-Dihydrobenzofuran-7 yl)piperazin-1 yl)butylamin; 4-(4-(Chroman-8 yl)piperazin-1-yl)butylamin

Die Synthese des Piperazin-substituierten Dihydrobenzofurans erfolgt analog Literatur (Kerrigan, F. Tetrahedron Lett. 1998, 2219-2222) bis zur Gewinnung von (2,3-Dihydrobenzofuran-7-yl)piperazin mit einer Ausbeute von 54% über 4 Reaktionsschritte.

Anschließend wird die freie Base mit einem Cyanoalkylhalogenid entsprechender Kettenlänge alkyliert, wie es exemplarisch im folgenden Reaktionsschema verdeutlicht ist:

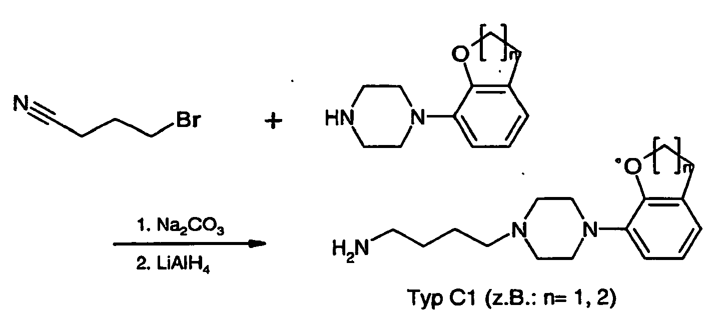

Dazu werden 3,7 mmol entsprechend substituiertes Piperazin und 0,8 g (7,5 mmol) Na₂CO₃ in 20 ml Acetonitril gelöst, 3,1 mmol ω-Bromalkylnitril dazugegeben und für 15 Stunden unter Rückfluss erhitzt, anschließend auf Raumtemperatur abgekühlt und die Lösung im Vakuum abgedampft. Der Rückstand wird mit Wasser aufgenommen und die wässrige Phase mit Methylenchlorid extrahiert, dieses getrocknet (mit MgSO₄) und das Lösungsmittel abgedampft. Reinigung durch Flashchromatographie (z.B. mit CHCl₃-EtOAc:1-1) liefert das entsprechende ω-(4-Phenylpiperazin-1 yl)alkylnitril.
Anschließend werden 0,5 mmol ω-(4-Phenylpiperazin-1yl)alkylnitril in 5 ml trockenem Diethylether gelöst und auf 0°C gekühlt. Dann werden langsam 1,0 ml LiAlH₄-Lösung (1 M in Diethylether) zugetropft und 1 Stunde bei Raumtemperatur gerührt. Nach erneutem Abkühlen auf 0°C wird mit gesättigter NaHCO₃-Lösung versetzt, durch eine Glasfritte mit Celite/MgSO₄/Celite filtriert und mit Methylenchlorid gewaschen. Evapurieren des Filtrats ergibt das gewünschte ω-(4-Phenylpiperazin-1 yl)alkylamin.

### 4-(4-(2,3-Dihydrobenzofuran-7-yl)piperazin-1-yl)butylamin

Die Synthese von 4-(4-(2,3-Dihydrobenzofuran-7-yl)piperazin-1 yl)butylamin erfolgt auf oben beschriebene Weise.
Ausbeute: 0,27 g (86% über 2 Reaktionsschritte).
MS: m/z 275 (M⁺). IR: (NaCl): 3359, 2939, 2820, 1609, 1487, 1456, 1254, 1190, 1132, 1012, 942, 870, 755, 661.'H NMR (CDCl₃, 360 MHz) δ (ppm): 1.43-1.63 (m, 4H,CH₂-CH₂); 2.34-2.40 (m, 2H, H₂N-CH₂); 2.62 (m, 4H, pip); 2.72-2.74 (m, 2H, O-CH₂-CH₂); 3.15-3.21 (m, 6H, pip, CH₂N); 4.56-4.61 (m, 2H, O-CH₂-CH₂); 6.69-6.71 (m, 1H, Phenyl); 6.77-6.86 (m, 2H, Phenyl).

### 4-(4-(Chroman-8-yl)piperazin-1-yl)butylamin

Die Herstellung von 4-(4-(Chroman-8-yl)piperazin-1-yl)butylamin erfolgt analog der für 4-(4-(2,3-Dihydrobenzofuran-7-yl)piperazin-1yl)butylamin beschriebenen Bedingungen. Ausbeute: 0,058 g (57% über 2 Reaktionsschritte).
MS: m/z 289 (M⁺). IR: (NaCl): 3354, 2933, 2870, 2814, 1664, 1479, 1461, 1247, 1196, 1024, 870, 737. ¹H NMR (CDCl₃, 360 MHz) δ (ppm): 1.46-1.59 (m, 4H,CH₂-CH₂); 1.96-2.03 (m, 2H, O-CH₂-CH₂-CH₂); 2.39-2.44 (m, 2H, CH₂-N); 2.65 (m, 4H, pip); 2.70-2.74 (m, 2H, O-CH₂-CH₂-CH₂); 2.77-2.80 (m, 2H, CH₂-NH₂); 3.08 (m, 4H, pip); 4.24-4.27 (m, 2H, O-CH₂-CH₂-CH₂); 6.71-6.79 (m, 3H, Phenyl).

### Herstellung der Amine vom Typ C2:

### 4-(4-(2,3,4,5-Tetrahydrobenzo[b]oxepin-9-yl)piperazin-1-yl)butylamin

In Ergänzung der Synthesevorschrift nach Typ C1 können 5- 6- und 7-Ring-annelierte bicyclische Systeme nach folgendem allgemeinen Reaktionsschema hergestellt werden:

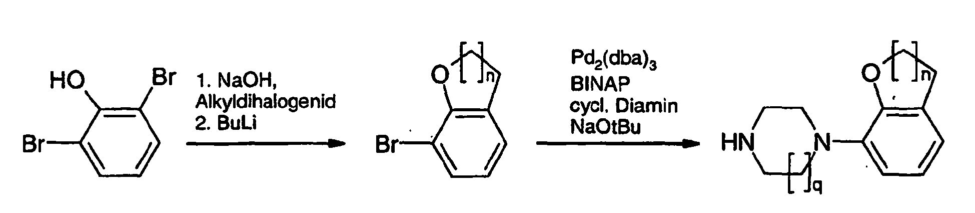

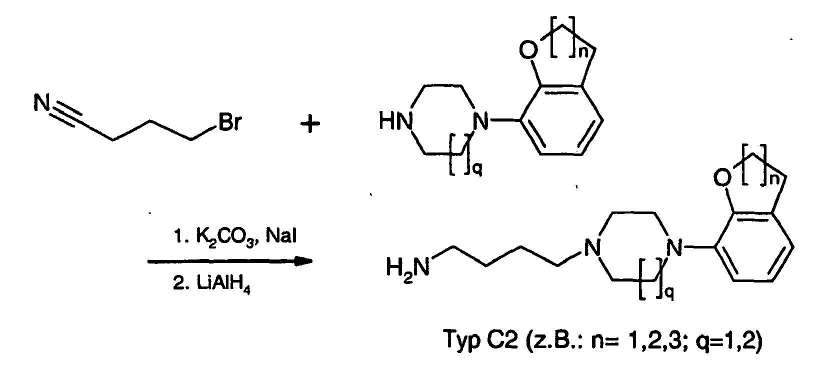

### Exemplarisch für oben aufgeführtes allgemeines Schema wird die Synthese von 4-(4-(2,3,4,5-Tetrahydrobenzo[b]oxepin-9-yt)piperazin-1 -yt)butylamin beschrieben:

Dazu wird zunächst 2,6-Dibromphenol (28.8 mmol) unter basischen Bedingungen (wässrige NaOH) mit 1,4-Dibrombutan (28.8 mmol) unter Rückfluß für 17 h erhitzt. Das dabei entstandene 2,6-Dibromphenoxybutylbromid (16.75 mmol) wird in THF/Hexan (4/1) gelöst, auf -80 °C gekühlt und langsam eine 2,5 M Lösung von Butyllithium in Hexan (17.1 mmol) zugetropft. Das so erhaltene annelierte 9-Brom-2,3,4,5-tetrahydrobenzo[b]oxepin (4 mmol) wird mit NaOtBu (20 mmol), Pd₂(dba)₃ (2 mol%), BINAP (2 mol%) und Piperazin (8 mmol) in 5 ml trockenem Toluol suspendiert und für 6 Stunden bei 117 °C erhitzt. Nach Aufarbeitung werden das erhaltene 1-(2,3,4,5-Tetrahydrobenzo[b]oxepin-9-yl)piperazin (1,5 mmol) und 0,63 g (4,5 mmol) K₂CO₃ in 20 ml Acetonitril gelöst, 0,15 ml (1,5 mmol) 4-Brombutyronitril zugegeben und für 15 Stunden unter Rückfluss erhitzt, anschließend auf Raumtemperatur abgekühlt und die Lösung im Vakuum abgedampft. Der Rückstand wird mit Wasser aufgenommen und die wässrige Phase mit Methylenchlorid extrahiert, dieses getrocknet (mit MgSO₄) und das Lösungsmittel abgedampft. Reinigung durch Flashchromatographie (CHCl₃-EtOAc:1-1) liefert das entsprechende 4-(4-(2,3,4,5-Tetrahydrobenzo[b]oxepin-9-yl)piperazin-1 yl)butyronitril. Davon werden anschließend 0,5 mmol in 5 ml trockenem Diethylether gelöst und auf 0°C gekühlt. Dann werden langsam 1,0 ml LiA1H₄-Lösung (1 M in Diethylether) zugetropft und 1 Stunde bei Raumtemperatur gerührt. Nach erneutem Abkühlen auf 0°C wird mit gesättigter NaHCO₃-Lösung versetzt, durch eine Glasfritte mit Celite/MgSO₄/Celite filtriert und mit Methylenchlorid gewaschen.

Evapurieren des Filtrats ergibt 4-(4-(2,3,4,5- Tetrahydro-benzo[b]oxepin-9-yl)piperazin-1-yl)butylamin.
Ausbeute: 0,52 g (86%).
(APCI) MS: m/z 304 ((M+H)⁺). IR: (NaCl): 2933, 2870, 2814, 1666, 1579,1475, 1450; 1246, 1192, 1038, 785, 733. ¹H NMR (CDCl₃, 360 MHz) δ (ppm): 1.47-1.63 (m, 4H, CH₂-CH₂); 1.68-1.75 (m, 2H, O-CH₂-CH₂-CH₂-CH₂); 1.93-2.00 (m, 4H, H₂O, O-CH₂-CH₂-CH₂-CH₂); 2.41-2.45 (m, 2H, CH₂-N); 2.61-2.65 (m, 4H, pip); 2.73-2.81 (m, 4H, O-CH₂-CH₂-CH₂-CH₂, CH₂-NH₂); 3.10-3.12 (m, 4H, pip); 3.98-4.00 (m, 2H, O-CH₂-CH₂-CH₂-CH₂); 6.77-6.81 (m, 2H, Phenyl); 6.88-6.93 (m, 1H, Phenyl). ¹³C NMR (CDCl₃, 90 MHz) δ (ppm): 153.5; 144.8; 136.9; 123.9; 123.4; 116.8; 73.3; 58.6; 53.7; 51.0; 42.0; 34.5; 32.5; 31.6; 26.1; 24.3.

### Herstellung der Amine vom Typ C3:

### 4-(4-(2,3-Dihydrobenzofuran-7-yl)-1,4-diazepan-1-yl)butylamin; 4-(4-(Chroman-8-yl)-1,4-diazepan-1-yl)butylamin; 4-(4-(2,3,4,5-Tetrahydrobenzo[b]oxepin-9-yl)-1,4-diazepan-1-yl)butylamin

Anstelle von Piperazin können bei der unter Typ C2 beschriebenen Pd-katalysierten Sübstitution auch andere cyclische Diamine, wie z.B. 1,4-Diazepan, für die Herstellung der Aminbausteine eingesetzt werden.

So erfolgt die Synthese von 4-(4-(2,3-Dihydrobenzofuran-7-yl)-1,4-diazepan-1-yl)-butylamin analog zur Herstellung der Amine vom Typ C2.
Ausbeute: 0,28 g (96%)
(APCI) MS: m/z 290 ((M+H)⁺). ¹H NMR (CDCl₃, 360 MHz) δ (ppm): 1.44-1.49 (m, 2H, CH₂-CH₂); 1.52-1.57 (m, 2H, CH₂-CH₂); 1.79-1.85 (m, 2H, N(CH₂-CH₂)N(CH₂-CH₂-CH₂)N; 1.95-1.99 (m, 2H, N(CH₂-CH₂)N(CH₂-CH₂-CH₂)N); 2.50-2.53 (m, 2H, CH₂-N); 2.70-2.73 (m, 4H, N(CH₂-CH₂)N(CH₂-CH₂-CH₂)N); 2.80-2.81 (m, 2H, CH₂-NH₂); 3.18 (t, J=8.9 Hz, 2H, O-CH₂-CH₂); 3.43-3.55 (m, 2H, N(CH₂-CH₂)N(CH₂-CH₂-CH₂)N); 3.51-3.53 (m, 2H, N(CH₂-CH₂)N(CH₂-CH₂-CH₂)N); 4.53 (t, J=8.9Hz, 2H, O-CH₂-CH₂); 6.61-6.62 (m, 1H, H-Phenyl); 6.70-6.71 (m, 1H, H-Phenyl); 6.75-6.78 (m, 1H, H-Phenyl).

Die Synthese von 4-(4-(Chroman-8-yl)-1,4-diazepan-1-yl)-butylamin erfolgt in Analogie zu der für Typ C2 beschriebenen Weise.
Ausbeute: 0,27 g (88%)
MS: m/z 303 (M⁺). ¹H NMR (CDCl₃, 360 MHz) δ (ppm): 1.42-1.49 (m, 4H, CH₂-CH₂); 1.62-1.70 (m, 2H, N(CH₂-CH₂)N(CH₂-CH₂-CH₂)N); 1.92-2.03 (m, 2H, O-CH₂-CH₂-CH₂); 2.50-2.54 (m, 2H, CH₂-N); 2.70-2.84 (m, 8H, N(CH₂-CH₂)N(CH₂-CH₂-CH₂)N); 3.28-3.32 (m, 2H, CH₂-NH₂); 4.20-4.23 (m, 2H, O-CH₂-CH₂); 6.61-6.63 (m, 1H, H-Phenyl); 6.73-6.75 (m, 2H, H-Phenyl).

4-(4-(2,3,4,5-Tetrahydrobenzo[b]oxepin-9-yl)-1,4-diazepan-1-yl)buty)amin wird analog synthetisiert.
Ausbeute: 0,3 g (94%)
(APCI) MS: m/z 318 ((M+H⁺)).

### Herstellung der Amine vom Typ C4:

### 4-(4-(Chroman-7-yl)piperazin- 1-yl)butylamin

Die Herstellung der Amine des Typs C4 erfolgt in Analogie zur Synthese der Amine vom Typ C2, wobei für die Synthese von 4-(4-(Chroman-7-yl)piperazin-1-yl)butylamin 2,5-Dibromphenol *(Interchim Building Blocks, Montlucon, Frankreich;* Nr: BC708) mit 1,3-Dibrompropan umgesetzt wird.
Ausbeute: 0,14g (92%)
(APCI) MS: m/z 304 (M⁺).

### Herstellung der Amine vom Typ C5:

### 4-(4-(2,3-Dihydrobenzofuran-5-yl)piperazin- 1 -yl)butylamin; 4-(4-(Chroman-6-yl)piperazin-1-yl)butylamin; 4-(4-(2,3,4,5- Tetrahydrobenzo[b]oxepin- 7-yl)piperazin- 1 -yl)butylamin

Die Herstellung der Amine des Typs C4 erfolgt in Analogie zur Synthese der Amine vom Typ C2, wobei für die Synthese des bicyclischen annelierten Bromheteroarensystems das von *Aldrich, Taufkirchen* käuflich erwerbbare 2,4-Dibromphehol (Nr: 25,816-4) mit 1 ,ω-Dibromalkanen umgesetzt wird.

### 4-(4-(2,3-Dihydrobenzofuran-5-yl)piperazin- 1-yl)butylamin

Für die Herstellung von 4-(4-(2,3-Dihydrobenzofuran-5-yl)piperazin-1-yl)butylamin wird 2,4-Dibromphenol mit 1,2-Dibromethan umgesetzt.
Ausbeute: 0,28 g (92%)
(APCl) MS: m/z 276 (M⁺). ¹H NMR (CDCl₃, 360 MHz) δ (ppm): 1.46-1.61 (m, 4H, CH₂-CH₂); 2.40 (t, J=7.5 Hz, 2H, H₂N-CH₂); 2.59-2.62 (m, 4H, pip); 2.72 (t, J=7.0 Hz, 2H, CH₂N); 3.06-3.09 (m, 4H, pip); 3.16 (t, J=8.6 Hz, 2H, O-CH₂-CH₂); 4.51 (t, J=8.6 Hz, 2H, O-CH₂-CH₂); 6.69 (d, J=8.4 Hz, 1H, Phenyl); 6.72 (dd, J=8.4 Hz, J=2.2 Hz, 1H, H-Phenyl); 6.86 (d, J=2.2 Hz, 1H, H-Phenyl).

### 4-(4-(Chroman-6-yl)piperazin-yl)butylamin

Für die Synthese von 4-(4-(Chroman-6-yl)piperazin-1-yl)butylamin wird 1,3-Dibrompropan eingesetzt.
Ausbeute: 0,2 g (97%).
(APCI) MS: m/z 290 ((M+H)⁺).

### 4-((4-(2,3,4,5-Tetrahydrobenzo[b]oxepin-7-yl)piperazin-1-yl)butylamin

Die Synthese von 4-(4-(2,3,4,5-Tetrahydrobenzo[b]oxepin-7-yl)piperazin-1-yl)butylamin erfolgt durch Umsetzung des 2,4-Dibromphenols mit 1,4-Dibrombutan.
Ausbeute: 0,549 g (90%).
(APCI) MS: m/z 304 ((M+H)⁺).

### Herstellung der Amine vom Typ C6:

### 4-((4-Benzo[1,3]dioxol-4-yl)piperazin-1-yl)butylamin

Amine vom Typ C6 werden analog zur Herstetiungsvorschrift für Typ C2 synthetisiert, wobei für die Synthese von 4-((4-Benzo[1,3]dioxol-4-yl)piperazin-1-yl)butylamin das bei *Maybridge, Tintangel, UK* gekaufte 4-Brom-1,3-benzodioxol (Nr: CC01710) für die Pd-gekuppelte Aminsubstitution eingesetzt wurde. Die anschließende Alkylierung und Reduktion liefert 4-((4-Benzo[1,3]dioxo)-4-yl)piperazin-1 yl)butylamin.
Ausbeute: 0,53 g (96%)
(APCI) MS: m/z 278 ((M+H)⁺).

### Herstellung der Amine vom Typ C7:

### 4-((4-Benzo[1,3]dioxol-5-yl)piperazin-7yl)butylamin; 4-(4-(2,3-Dihydrobenzo[1,4]dioxin-6-yl)piperazin-1-yl)butylamin; 4-(4-(3,4-Dihydro-2H-benzo[b][l,4jdioxepin-7-yl)piperazin-7-yl)butylamin

Amine des Typs C7 werden hergestellt in Analogie zur Synthese der Amine des Typs C2 durch Einsatz entsprechend substituierter bicyclisch annelierter Bromarene.

### 4-((4-Benzo[1,3]dioxol-5- yl)piperazin-1-yl)butylamin

Umsetzung von 5-Brom-1,3-benzodioxol *(Aldrich, Tautkirchen;* Nr: 28,831-4) führte zur Synthese von 4-(4-Benzo[1,3]dioxol-5-yl)piperazin-1 yl-butylamin.
Ausbeute: 0,24 g (96%)
(APCI) MS: m/z 278 ((M+H)⁺). ¹H NMR (CDCl₃, 360 MHz) ö (ppm): 1.44-1.61 (m, 4H, CH₂-, CH₂); 1.64-1.69 (brs, 2H NH₂); 2.40 (t, J=7.0 Hz, 2H, H₂N-CH₂); 2.58-2.61 (m, 4H, pip); 2.73 (t, J=7.0 Hz, 2H, CH₂N); 3.06-3.09 (m, 4H, pip); 5.88 (s, 2H, O-CH₂-O); 6.36 (d, J=8.4, Hz, J=2.4 Hz, 1H, H-Phenyl); 6.56 (d, J=2.4 Hz, 1H, H-Phenyl); 6.71 (d, J=8.4 Hz, 1H, H-Phenyl).

### 4-(4-(2,3-Dihydrobenzo[1,4]dioxin-6 yl)piperazin-1-yl)butylamin

Der Einsatz von 6-Brom-2,3-Dihydrobenzo[1,4]dioxin (*Lancester, Frankfurt;* Nr: 6207) führte zur Herstellung von 4-(4-(2,3-Dihydrobenzo[1,4]dioxin-6-yl)piperazin-1-yl)butylamin. Ausbeute: 0,12 g (59%).
(APCI) MS: m/z 292 ((M+H)⁺). IR: (NaCl): 2937, 2875, 2817, 1587, 1508, 1454, 1284, 1219, 1070, 752. ' H NMR (CDCl₃, 360 MHz) ö (ppm): 1.46-1.67 (m, 4H, CH₂-CH₂); 1.88-, 1.98 (brs, 2H, NH₂); 2.40 (t, J=7.0 Hz, 2H, CH₂-N); 2.57-2.61 (m, 4H, pip); 2.74 (t, J=7.0 Hz, 2H, CH₂-NH₂); 3.06-3.11 (m, 4H, pip); 4.18-4.25 (m, 4H, O-CH₂-CH₂-O); 6.44-6.48 (m, 2H, H-Phenyl); 6.75-6.78 (m, 1H, H-Phenyl).

### 4-(4-(3,4-Dihydro-2H-benzo[b][1,4]dioxepin-7-yl)piperazin-1-yl)butylamin

Die Herstellung von 4-(4-(3,4-Dihydro-2H-benzo[b][1,4]dioxepin-7-yl)piperazin-1-yl)butylamin erfolgt ausgehend von dem bei *Maybridge, Tintangel, UK gekauften* 7-Brom-. 3,4-dihydro-2H-1,5-benzodioxepin (Nr: CC 13210) analog der bei der Synthese der Herstellung der Amine vom Typ C2 beschriebenen Bedingungen.
Ausbeute: 0,58 g (95%)
(APCI) MS: m/z 306 (M+H)⁺).

### Herstellung der Amine vom Typ C8:

### 4-(4-(2,3-Dihydrobenzo[1,4]dioxin-6-yl)-1,4-diazepan-1-yl)butylamin

Die Synthese der Amine des Typs C8 erfolgt analog der Vorschriften für die Herstellung der Amine des Typs C3.
Für die Herstellung von 4-(4-(2,3-Dihydrobenzo[1,4]dioxin-6-yl)-1,4-diazepan-1-yl)butylamin wurde 6-Brom-2,3-Dihydrobenzo[1,4]dioxin *(Lancester, Frankfurt;* Nr: 6207) eingesetzt.
Ausbeute: 0,6 g (98%)
(APCI) MS: m/z 306 ((M+H)⁺). ¹H NMR (CDCl₃, 360 MHz) δ (ppm): 1.53-1.59 (m, 4H, CH₂-CH₂); 1.92-1.99 (m, 2H, O-CH₂-CH -CH₂-O); 2.48-2.52 (m, 2H, CH₂-N); 2.62-2.65 (m, 2H, N(CH₂-CH₂)N(CH₂-CH₂-CH₂)N); 2.73-2.79 (m, 4H, N(CH₂-CH₂)N(CH₂-CH₂-CH₂)N, CH₂-NH₂); 3.21-3.50 (m, 6H, N(CH₂-CH₂)N(CH₂-CH₂-CH₂)N); 4.16-4.19 (m, 2H, O-CH₂-CH₂-CH₂-O); 4.22-4.24 (m, 2H, O-CH₂-CH₂-CH₂-O); 6.18-6.21 (m, 2H, H-Phenyl); 6.71-6.74 (m, 1H, H-Phenyl).

### Herstellung der Amine vom Typ C9:

### 8-(4-(4-Aminobutyl)piperazin- 1-yl)-6-chlor-3,4-dihydro-2H-benzo[1,4]oxazin-3-on

Amine vom Typ C9 werden nach folgendem Reaktionsschema ausgehend von 2-Amino-4-chlor-6-nitrophenol synthetisiert:

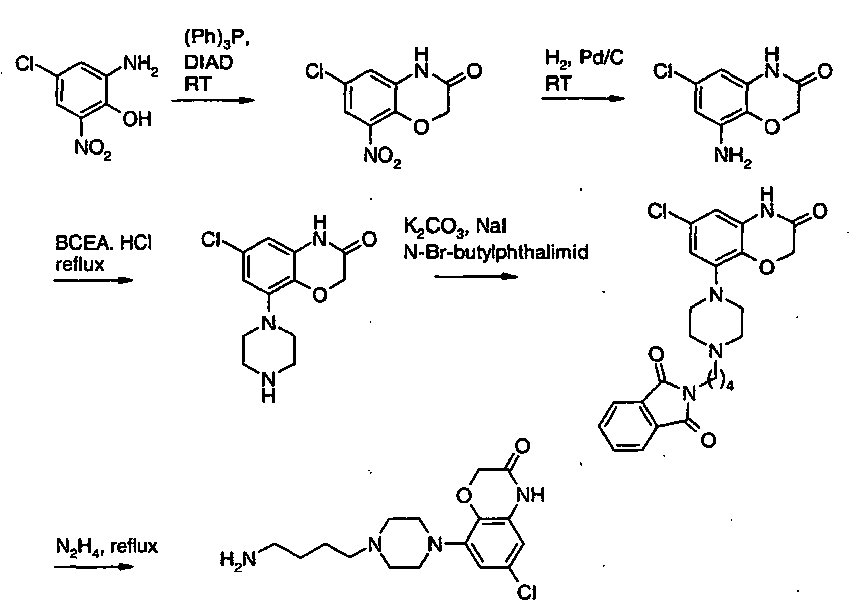

Zu einer gekühlten Lösung (0° C) von 5 g (26,5 mmol) 2-Amino-4-chlor-6-nitrophenol (*Aldrich, Taufkirchen;* Nr. 5303871), 2,4 g (26,5 mmol) Methylglycolat und 7,77 g (29,2 mmol) Triphenylphosphin in trockenem THF (200 ml) wird langsam eine Lösung von Düsopropylazodicarboxylat (DIAD; 5,7 ml, 29,5 mmol) in trockenem THF (10 ml) zugetropft. Die Reaktionslösung wird für 4 Tage bei RT gerührt, danach wird das Lösungsmittel im Vakuum abgedampft und das entstehende Öl in Ethanol suspendiert.

Dabei präzipitiert 6-Chlor-8-nitro-3,4-dihydro-2H benzo[1,4]oxazin-3-on als dunkelgrüner Feststoff.
Ausbeute: 3,6 g (59%)
IR (NaCl) v (cm⁻¹): 3390; 2923; 2854; 1707; 1631; 1473; 1342; 1028; 893. ¹H NMR (CDCl₃, 600 MHz) δ (ppm): 4.86 (s, 2H, O-CH₂-CONH); 7.65 (d, J=2.5 Hz, 1H, H-5); 8.02 (d, J=2.5 Hz, 1H, H-7).
Zu einer Lösung von 2,5 g (10,9 mmol) 6-Chlor-8-nitro-3,4-dihydro-2*H*-benzo[1,4]oxazin-3-on in 50 ml EtOH/EtOAc (1/1; v/v) werden 0,8 g Pd/C gegeben und dann für 24 h unter H₂-Atmosphäre gerührt. Das Reaktionsgemisch wird über Celite filtriert und das Filtrat am Rotationsverdampfer eingeengt. Der Rückstand wird durch Flashchromatographie (CH₂Cl₂-MeOH: 90-10) gereinigt und liefert 8-Amino-6-chlor-3,4-dihydro-2*H-*benzo[1,4]oxazin-3-on.
Ausbeute: 0,93 g (43%)
(APCl) MS: m/z 199 ((M+H)⁺). IR (NaCl) v (cm⁻¹): 3365; 2925; 2854; 1704; 1631; 1414; 771. ¹H NMR (CD₃OD, 600 MHz) ö (ppm): 4.51 (s, 2H, O-CH₂-CONH); 6.22 (d, J=2.5 Hz, 1H, H-7); 6.40 (d, J=2.5 Hz, 1H, H-5).
Zu der Lösung von 0,93 g (4,7 mmol) 8-Amino-6-chlor-3,4-dihydro-2*H*-benzo[1,4]oxazin-3-on in 50 ml Chlorbenzol wird Bischlorethylenaminhydrochlorid (BCEA; 0,87g, 4,9 mmol) gegeben und unter Rückfluss für 80 h erhitzt. Einengen des Reaktionsgemisches am Rotationsverdampfer und Reinigung des entstehenden Rückstands durch Flashchromatographie (CH₂Cl₂-MeOH-Et₃N: 80-18-2) liefert 6-Chlor-8-piperazin-1-yl-3,4-dihydro-2*H*-benzo[1,4]oxazin-3-on.
Ausbeute: 0,50 g (45%)
(APCI) MS: m/z 268 ((M+H)⁺). IR (NaCl) v (cm⁻¹): 3392; 2848; 1689; 1620; 1591; 1396; 1228; 1036. ¹H NMR (CD₃OD, 600 MHz) δ (ppm): 2.96-2.98 (m, 4H, pip); 3.04-3.06 (m, 4H, pip). 4.57 (s, 2H, O-CH₂-CONH); 6.44 (d, J=2.9 Hz, 1H, H-5); 6.64 (d, J=2.9 Hz, 1H, H-7).
6-Chlor-8-piperazin-1-yl-3,4-dihydro-2*H*-benzo[1,4]oxazin-3-on (0,5 g; 1,9 mmol), K₂CO₃ (0,8 g; 5,8 mmol) und Nal (0,6 g, 0,4 mmol) werden in 10 ml trockenem Acetonitril gelöst.
Dann wird N-(4-Brombutyl)phthalimid (0,63 g; 2,2 mmol) zugegeben und für 15 Stunden unter Rückfluss erhitzt. Nach Abkühlen auf Raumtemperatur wird das Lösungsmittel im Vakuum abgedampft und der Rückstand durch Flashchromatographie (CH₂Cl₂-MeOH: 95-5) gereinigt, was zu 2-(4-(4-(4-Chlor-3-oxo-3,4-dihydro-2*H*-benzo[1,4]oxazin-8-yl)piperazin-1-yl)butyl)isoindol-1,3-dion führt.
Ausbeute: 0,2 g (22%).
(APCI) MS: m/z 469 ((M+H)⁺). IR (NaCl) v (cm⁻¹): 1768; 1707; 1620; 1518; 1398; 1223; 1047; 908. ¹H NMR (CDCl₃, 360 MHz) δ (ppm): 1.52-1.61 (m, 2H, CH₂-CH₂); 1.70-1.78 (m, 2H, CH₂-CH₂); 2.42 (t, J=7.5 Hz, 2H, CH₂-N); 2.54-2.58 (m, 4H, pip); 3.06-3.10 (m. 4H, pip). 3.72 (t, J=7.0 Hz, 2H, CH₂N(CO)₂); 4.64 (s, 2H, O-CH₂-CONH); 6.26 (d, J=2.7 Hz, 1H, H-5); 6.58 (d, J=2.7 Hz, 1H, H-7); 7.70-7.73 (m, 2H, Isoindol); 7.83-7.86 (m, 2H, Isoindol); 8.47 (brs, 1H, NHCO).
Zu einer Suspension von 2-(4-(4-(6-Chlor-3-oxo-3,4-dihydro-2H benzo[1,4]oxazin-8-yl)piperazin-1-yl)butyl)isoindol-1,3-dion (0,2 g; 0,43 mmol) in 10 ml Ethanol wird vorsichtig eine Lösung von Hydrazinhydrat 80% (0,2 ml; 5,5 mmol) in Ethanol (5 ml) zugetropft. Dann wird für 30 Minuten unter Rückfluss erhitzt, auf Raumtemperatur abgekühlt und das Lösungsmittel am Rotationsverdampfer abgedampft. Der Rückstand wird durch Flashchromatographie (CH₂Cl₂-MeOH-Et₃N: 80-18-2) gereinigt und liefert 8-(4-(4-Aminobutyl)piperazin-1-yl)-6-chlor-3,4-dihydro-2*H*-benzo[1,4]oxazin-3-on.
Ausbeute: 0,12 g (80%)
(APCI) MS: m/z 339 ((M+H)⁺). IR (NaCl) v (cm⁻¹): 2821; 1699; 1653; 1475; 1296; 1230; 750. ¹H NMR (CD₃OD, 360 MHz) δ (ppm): 1.64-1.69 (m, 4H, CH₂-CH₂); 2.45-2.49 (m, 2H, CH₂N); 2.64-2.69 (m, 4H, pip); 2.87-2.91 (m, 2H, CH₂-NH₂); 3.12-3.15 (m, 4H, pip); 4.60 (s, 2H, O-CH₂-CONH); 6.48 (d, J=2.7 Hz, 1H, H-5); 6.66 (d, J=2.7 Hz, 1H, H-7).

### SYNTHESE DER BEISPIELVERBINDUNGEN

### Beispiel 1:

### N-(4-(4-(2,3-Dihydrobenzofuran-7-yl)piperazin-1-yl)butyl)benzo[b]thiophen-2-ylcarbamid-

Benzo[b]thiophen-2-carbonsäure (21 mg, 0,12 mmol) wird in trockenem Methylenchlorid (4 ml) gelöst und DIEA (0,07 ml, 0,42 mmol) zugegeben, danach wird auf 0°C gekühlt. Das in 0,3 ml DMF gelöste TBTU (42 mg, 0,13 mmol) wird hinzugefügt und anschließend das in 5 ml Methylenchlorid gelöste 4-(4-(2,3-Dihydrobenzofuran-7-yl)piperazin-1-yl)butylamin (36 mg, 0,13 mmol) zugetropft. Das Reaktionsgemisch wird für 0,5 Stunden bei Raumtemperatur gerührt. Dann wird der Ansatz mehrmals mit gesättigter Natriumhydrogencarbonatlösung ausgeschüttelt und die vereinigten wässrigen Phasen erneut mit Methylenchlorid extrahiert. Die gesammelten organischen Phasen werden mit gesättigter Natriumchloridlösung gewaschen, mit Magnesiumsulfat getrocknet und am Rotationsverdampfer eingeengt. Der Rückstand wird durch Flashchromatographie gereinigt (CH₂Cl₂-MeOH: 98-2).
Ausbeute: 51 mg (97 %) weißer Feststoff.
Smp.:139°C. MS m/z 435 (M⁺). IR (NaCl) v (cm⁻¹): 3317; 2935; 2816; 1630; 1543;, 1252; 1155; 756. ¹H NMR (CDCl₃, 360 MHz) ö (ppm): 1.65-1.73 (m, 4H, CH₂-CH₂); 2.48 (t, J=6.7 Hz, 2H, CH₂N(CH₂-CH₂)₂N); 2.61-2.66 (m, 4H, N(CH₂-CH₂)₂N); 3.17-3.22 (m, 6H, N(CH₂-CH₂)₂N, OCH₂CH₂); 3.48-3.53 (m, 2H, CH₂NHC0); 4.58 (t, J=8.3 Hz, 2H, OCH₂CH₂); 6.63 (d, J=7.5 Hz, 1H, H-Phenyl); 6.71 (br J=5.1 Hz, 1H, NHCO); 6.76-6.80 (m, 1H, H-Phenyl); 6.86 (d, J=7.1 Hz, 1H, H-Phenyl); 7.36-7.43 (m, 2H, H-5, H-6); 7.77 (s, 1H, H-3); 7.81 (d, J=7.5 Hz, 1H, H-4); 7.85 (d, J=7.3 Hz, 1H, H-7). ¹³C-NMR (CDCl₃, 90 MHz) δ (ppm): 24.2; 27.4; 30.0; 39.9; 49.4; 53.3; 57.9; 70.9; 115.6; 118.2; 121.0; 122.7; 124.8, 124.9, 125.0; 126.2; 127.5;136.2 138.7; 139.1; 140.7; 151.1; 162.4.
C H N (%): C₂₅H₂₉N₃O₂S x 0.25 H₂O
Ber.: C 68,23; H 6,76; N 9,55; S 7,28. Gef.: C 68,26; H 6,64; N 9,48; S 7,36.

### Beispiel 2:

### N-(4-(4-(2,3-Dihydrobenzofuran-7-yl)piperazin-1-yl)butyl)indol-2-ylcarbamid

Die Synthese erfolgt analog zu Beispiel 1.
Ausbeute: 40 mg (78 %) weißer Feststoff
Smp.: 154 °C. MS m/z 418 (M⁺). IR (NaCl) v (cm⁻¹): 3415; 2927; 2854; 2817; 1635; 1556; 1250; 1070; 752. ¹H NMR (CDCl₃. 360 MHz) δ (ppm): 1.65-1.71 (m, 4H, CH₂-CH₂); 2.52 (t, J=6.9 Hz, 2H, CH₂N(CH₂-CH₂)₂N); 2.70-2.73 (m, 4H, N(CH₂-CH₂)₂N); 3.17-3.22 (m, 6H, O CH₂CH₂ , N(CH₂-CH₂)₂N); 3.50-3.55 (m, 2H, CH₂NHCO); 4.59 (t, J=8.7 Hz, 2H, O-CH₂-CH₂); 6.67-6.70 (m, 2H, Phenyl, NHCO); 6.77-6.82 (m, 1H, Phenyl,); 6.86-6.87 (m, 2H, Phenyl, H-3); 7.11-7.15 (m, 1H, H-5); 7.25-7.30 (m, 1H, H-6); 7.44 (dd, J=8.3 Hz, J=0.7 Hz, 1H, H-7); 7.64 (d, J=8.0 Hz, 1H, H-4); 9.57 (brs, 1H, NH). ¹³C-NMR (CDCl₃, 90 MHz) δ (ppm): 24.2; 27.4; 30.0; 39.4; 49.2; 53.2; 57.9; 71.0; 102.0; 111.9; 115.7; 118.3; 120.6; 121.1, 121.9; 124.3; 127.5, 127.6; 130.9; 136.0, 136.2; 151.1; 161.7.
C H N (%):C₂₅H₃₀N₄O₂x 0.1 H₂O
Ber.: C 71,40; H 7,24; N 13,32. Gef.: C 71,38; H 7,22; N 13,33.

### Beispiel 3:

### N-(4-(4-(Chroman-8-yl)piperazin-1-yl)butyl)benzo[b]thiophen-2-ylcarbamid

Benzo[b]thiophen-2-carbonsäure (21 mg, 0,12mmol) wird in trockenem Methylenchlorid (4 ml) gelöst und 0,07 ml DIPEA (0,42 mmol) zugegeben, dann auf 0°C gekühlt. Das in 0,5 ml DMF gelöste TBTU (42 mg, 0,13 mmol) wird hinzugefügt und anschließend das in 5 ml Methylenchlorid gelöste 4-(4-(Chroman-8-yl)piperazin-1-yl)butylamin (39 mg, 0,13 mmol) zugetropft. Das Reaktionsgemisch wird für 1 Stunde bei Raumtemperatur gerührt, danach mehrmals mit gesättigter Natriumhydrogencarbonatlösung ausgeschüttelt und die vereinigten wässrigen Phasen erneut mit Methylenchlorid extrahiert. Die gesammelten organischen Phasen werden mit gesättigter Natriumchloridlösung gewaschen, mit Magnesiumsulfat getrocknet und am Rotationsverdampfer eingeengt. Der Rückstand wird mittels Flashchromatographie gereinigt (CH₂Cl₂-MeOH: 98-2).
Ausbeute: 53 mg (98 %) weißer Feststoff.
Smp.: 134 °C. MS: m/z 449 (M⁺). IR (NaCl) v (cm⁻¹): 3325; 2930; 2853; 2817; 1631; 1544; 1248; 1217; 754. ¹H NMR (CDCl₃, 360 MHz) ö (ppm): 1.69-1.74 (m, 4H, CH₂-CH₂); 1.96-2.03 (m, 2H, O-CH₂-CH₂-CH₂); 2.55 (t, J=6.9 Hz, 2H, CH₂N(CH₂-CH₂)₂N); 2.74-2.80 (m, 6H, N(CH₂-CH₂)₂N, O-CH₂-CH₂-CH₂); 3.09-3.12 (m, 4H, N(CH₂-CH₂)₂N); 3.48-3.53 (m, 2H, CH₂NHCO); 4.24 (t, J=5.1 Hz, 2H, O-CH₂-CH₂-CH₂; 6.69 (dd, 1H, J=7.1 Hz, J=2.5 Hz, H-Phenyl); 6.72-6.80 (m, 2H, H-Phenyl); 6.83 (brt, J=4.9 Hz, 1H, NHCO); 7.36-7.44 (m, 2H, H-5, H-6); 7.79 (brs, 1H, H-3); 7.81-7.86 (m, 2H, H-4, H-7). ¹³C NMR (COCl₃, 90 MHz) δ (ppm): 22.1; 23.9; 25.1; 27.2; 39.8; 50.2; 53.4; 57.9; 66.5; 115.9; 119.9; 122.6; 122.7; 124.1; 124.8; 125.0;125.1; 126.2; 139.7; 139.1; 140.4; 140.7; 147.6; 162.5.
C H N (%): C₂₆H₃₁N₃O₂S × 0.45 H₂O
Ber.: C 68,21; H 7,03; N 9,18; S 7,00. Gef.: C 68,09; H 6,87; N 8,95; S 7,05.

### Beispiel 4:

### N-(4-(4-(Chroman-8-yl)piperazin-1-yl)butyl)indol-2-ylcarbamid

Die Synthese erfolgt analog zu Beispiel 3.
Ausbeute: 31 mg (61 %) weißer Feststoff
Smp.:141 °C. MS: m/z 432 (M⁺). IR (NaCl) v (cm⁻¹): 3265; 2929; 2853; 2822; 1635; 1554; 1248; 1217. ¹H NMR (CDCl₃, 360 MHz) δ (ppm): 1.66-1.74 (m, 4H, CH₂-CH₂); 1.96-2.03 (m, 2H, O-CH₂-CH₂-CH₂); 2.50 (t, J=6.9 Hz, 2H, CH₂N(CH₂-CH₂)₂N); 2.63-2.72 (m, 4H, N(CH₂-CH₂)₂N); 2.78 (t, J=6.4 Hz, 2H, O-CH₂-CH₂-CH₂); 3.08-3.14 (m, 4H, N(CH₂-CH₂)₂N); 3.50-3.55 (m, 2H, CH₂NHCO); 4.25 (t, J=5.1 Hz, 2H, O-CH₂-CH₂-CH₂); 6.65 (brt, J=5.3 Hz, 1H, NHCO); 6.73 (dd, J=2.3 Hz, J= 6.9 Hz, 1H, H-Phenyl); 6.73-6.80 (m, 2H, H-Phenyl); 6.86 (dd, J=2.2 Hz, J=0.7 Hz, 1H, H-3); 7.14 (dd J=7.5 Hz, J=1.2 Hz, 1H, H-5); 7.29 (dd, J=8.3 Hz, J=1.2 Hz, 1H, H-6); 7.44 (dd, J=8.3 Hz, J=0.8 Hz, 1H, H-7); 7.64 (dd, J=7.5 Hz, J=0.8 Hz, 1H, H-4); 9.46 (s, 1H, NH). ¹³C NMR (CDCl₃, 90 MHz) δ (ppm): 22.1; 24.2; 25.1; 27.4 ; 39.5; 50.4; 53.4; 57.9; 66.5; 101.9; 111.9; 115.9; 119.9; 120.6; 121.9; 122.7; 124.0, 124.4; 127.6; 130.9; 136.2; 140.7; 147.6; 161.7.
C H N (%):C₂₆H₃₂N₄O₂ x 0,6 H₂O
Ber.: C 70,24; H 7,56; N 12,60. Gef.: C 70,58; H 7,42; N 12,22.

### Beispiel 5:

### N-(4-(4-(2,3-Dihydrobenzofuran-7-yl)piperazin-1-yl)butyl)benzo[b]thiophen-3-ylcarbamid

Die Synthese erfolgt analog zu Beispiel 1.
Ausbeute: 51 mg (97 %) weißer Feststoff.
Smp.: 159 °C. MS m/z 435 (M⁺). IR (NaCI) v (cm⁻¹): 3319; 2939; 2817; 1633; 1539; 1254; 1147; 764. ¹H NMR (CDCl₃, 360 MHz) δ (ppm): 1.66-1.75 (m, 4H, CH₂-CH₂); 2.46 (t, J=6.8 Hz, 2H, CH₂N(CH₂-CH₂)₂N); 2.59-2.61 (m, 4H, N(CH₂-CH₂)₂N); 3.03-3.03 (m, 6H, N(CH₂-CH₂)₂N); 3.19 (t, J=8.7 Hz, 2H, OCH₂CH₂); 3.49-3.55 (m, 2H, CH₂NHCO); 4.58 (t, J=8.9 Hz, 2H, OCH₂CH₂); 6.59 (d, J=7.5 Hz, 1H, H-Phenyl); 6.76-6.83 (m, 2H, H-Phenyl, NHCO); 6.85 (dd, J=7.5 Hz, J=1.1 Hz, 1H, H-Phenyl); 7.36-7.46 (m, 2H, H-5, H-6); 7.83 (s, 1H, H-2); 7.84-7.87 (m, 1H, H-4); 8.34 (d, J=7.5 Hz, 1H, H-7).

### Beispiel 6:

### N-(4-(4-(2,3-Dihydrobenzofuran-7-yl)piperazin-1- yl)butyl)benzofuran-3-ylcarbamid

Die Synthese erfolgt analog zu Beispiel 1.
Ausbeute: 41 mg (81 %) farbloses Öl
(APCI) MS m/z 420 ((M+H)⁺). IR (NaCl) v (cm⁻¹): 3315; 2941; 2819; 1637; 1566; 1452; 1254; 1012; 752. ¹H NMR (CDCl₃, 360 MHz) δ (ppm): 1.65-1.75 (m, 4H, CH₂-CH₂); 2.48 (t, J=6.9 Hz, 2H, CH₂N(CH₂-CH₂)₂N); 2.62-2.66 (m, 4H, N(CH₂-CH₂)₂N); 3.09-3.13 (m, 4H, N(CH₂-CH₂)₂N); 3.19 (t, J=8.7 Hz, 2H, O-CH₂-CH₂; 3.50-3.54 (m, 2H, CH₂NHCO); 4.58 (t, J=8.9 Hz, 2H, O-CH₂-CH₂); 6.47 (brt, J=4.9 Hz, 1H, NHCO); 6.64 (dd, J=7.4 Hz, J=0.7 Hz, 1H, H-Phenyl,); 6.76-6.80 (m, 1H, H-Phenyl); 7.85 (dd, J=7.4 Hz, J=0.7 Hz, 1H, H-Phenyl); 7.32-7.38 (m, 2H, H-5, H-6); 7.51-7.54 (m, 1H, H-7); 7.90-7.94 (m, 1H, H-4); 8.09 (s, 1H, H-2). ¹³C-NMR (CDCl₃, 90 MHz) δ (ppm): 24.3; 27.6: 30.1; 39.5; 49.4; 53.3; 58.1; 71.0; 111.9; 115.7; 118.1; 118.2; 121.0, 121.9; 123.9; 124.6; 125.2, 127.5; 136.2, 146.7; 151.1; 155.5; 163.0.

### Beispiel 7:

### N-(4-(4-(2,3-Dihydrobenzofuran-7-yl)piperazin- 1-yl)butyl)indol-3-ylcarbamid

Die Synthese erfolgt analog zu Beispiel 1.
Ausbeute: 39 mg (77 %) farbloses Öl
MS m/z 418 (M⁺). IR (NaCl) v (cm⁻¹): 2927; 2856; 2817; 1631; 1547; 1251; 1007; 752. ¹H NMR (CDCl₃, 360 MHz) δ (ppm): 1.66-1.74 (m, 4H, CH₂-CH₂); 2.47 (t, J=6.9 Hz, 2H, CH₂N(CH₂-CH₂)₂N); 2.62-2.65 (m, 4H, N(CH₂-CH₂)₂N); 3.10-3.14 (m, 4H, N(CH₂-CH₂)₂N); 3.19 (t, J=8.7 Hz, 2H, O-CH₂-CH₂); 3.51-3.56 (m, 2H, CH₂NHCO); 4.58 (t, J=8.9 Hz, 2H, O-CH₂-CH₂); 6.27 (brt, J=5.0 Hz, 1H, NHCO); 6.65 (d, J=7.4 Hz, 1H, H-Phenyl); 6.77-6.81 (m, 1H, H-Phenyl); 6.85 (dd, J=7.4 Hz, J=0.9 Hz, 1H, H-Phenyl); 7.22-7.26 (m, 2H, H-5, H-2); 7.41-7.46 (m, 1H, H-6); 7.74 (d, J=3.0 Hz, 1H, H-7 od. H-4); 7.94-7.98 (m, 1H, H-7 od. H-4); 8.94 (brs, 1H, NH).

### Beispiel 8:

### N-(4-(4-(2,3-Dihydrobenzofuran-7-yl)-1,4-diazepan-1-yl)butyl)benzo[b]thiophen-2-ylcarbamid

Benzo[*b*]thiophen-2-carbonsäure (21 mg, 0,12 mmol) wird mit 4-(4-(2,3-Dihydrobenzofuran-7-yl)-1,4-diazepan-1-yl)-butylamin (38 mg, 0,13 mmol) umgesetzt wie für Beispiel 1 beschrieben. Die Aufreinigung gelingt durch Flashchromatographie (CH₂Cl₂-MeOH:95-5).
Ausbeute: 42 mg (78 %) farbloses Öl.
MS m/z 449 (M⁺). IR (NaCI) v (cm⁻¹): 2925; 2852; 1631; 1547; 1240; 756. ¹H NMR (CDCl₃, 360 MHz) δ (ppm): 1.70-1.78 (m, 4H, CH₂-CH₂); 2.10-2.16 (m, 2H, N(CH₂-CH₂)N(CH₂-CH₂-CH₂)N); 2.73 (t, J=6.9 Hz, 2H, CH₂N(CH₂-CH₂)N(CH₂-CH₂-CH₂)N); 2.92-2.95 (m, 2H, N(CH₂-CH₂)N(CH₂-CH₂-CH₂)N); 3.00-3.03 (m, 2H, N(CH₂-CH₂)N(CH₂-CH₂-CH₂)N); 3.17 (t, J=8.7 Hz, 2H, O-CH₂-CH₂); 3.39 (t J=6.5 Hz, 2H, N(CH₂-CH₂)N(CH₂-CH₂-CH₂)N); 3.49-3.56 (m, 4H, CH₂NHCO, N(CH₂-CH₂)N(CH₂-CH₂-CH₂)N); 4.51 (t, J=8.7 Hz, 2H, OCH₂CH₂); 6.59 (dd, J=7.5 Hz, J=1.8 Hz, 1H, H-Phenyl); 6.71-6.78 (m, 2H, H-Phenyl); 7.08 (br t J=3.6 Hz, 1H, NHCO); 7.35-7.43 (m, 2H, H-5, H-6); 7.81-7.86 (m, 2H, H-4, H-7); 7.89 (brs, 1H, H-3). ¹³C-NMR (CDCl₃, 90 MHz) ö (ppm): 24.0; 26.8; 26.9; 30.2; 39.4; 49.5; 49.7; 53.8; 56.6; 56.8; 70.7; 114.5; 115.8; 121.1; 122.6; 124.7, 125.0; 125.1; 126.0; 127.5;136.3 138.9; 139.2; 140.8; 149.2; 162.5.

### Beispiel 9:

### N-(4-(4-(Chroman-8 yl)-1,4-diazepan-1-yl)butyl)benzo[b]thiophen-3-ylcarbamid

Benzo[*b*]thiophen-3-carbonsäure (21 mg, 0,12 mmol) wird mit 4-(4-(Chroman-8-yl)-1,4-diazepan-1-yl)butylamin (40 mg, 0,13 mmol) wie in Beispiel 1 beschrieben umgesetzt. Die Aufreinigung gelingt durch Flashchromatographie (CH₂Cl₂-MeOH: 95-5).
Ausbeute: 43 mg (77 %) farbloses Öl.
(APCI)MS: m/z 464 ((M+H)⁺). IR (NaCl) v (cm⁻¹): 3292; 2929; 2859; 2817; 1635; 1539; 1217; 752. ¹H NMR (CDCl₃, 360 MHz) δ (ppm): 1.69-1.75 (m, 4H, CH₂-CH₂); 1.92-2.01 (m, 4H, O-CH₂-CH₂-CH₂, N(CH₂-CH₂)N(CH₂-CH₂-CH₂)N); 2.63 (t, J=6.7 Hz, 2H, CH₂N(CH₂-CH₂)N(CH₂-CH₂-CH₂)N); 2.77 (t, J=6.7 Hz, 2H, N(CH₂-CH₂)N(CH₂-CH₂-CH₂)N); 2.79-2.82 (m, 2H, N(CH₂-CH₂)N(CH₂-CH₂-CH₂)N); 2.86-2.89 (m, 2H, N(CH₂-CH₂)N(CH₂-CH₂-CH₂)N); 3.22-3.25 (m, 2H, O-CH₂-CH₂-CH₂); 3.50-3.54 (m, 2H, CH₂NHCO); 3.29-3.32 (m, 2H, N(CH₂-CH₂)N(CH₂-CH₂-CH₂)N); 4.17-4.20 (m, 2H, O-CH₂-CH₂-CH₂); 6.63 (dd, 1H, J=7.0 Hz, J=2.0 Hz, H-Phenyl); 6.68-6.72 (m, 2H, H-Phenyl); 7.08 (brt, J=5.0 Hz, 1H, NHCO); 7.35-7.46 (m, 2H, H-5, H-6); 7.83-7.86 (m, 1H, H-7); 7.91 (brs, 1H, H-2); 8.39-8.41 (m, 1H, H-4).

### Beispiel 10:

### N-(4-(4-(Chroman-8-yl)-1,4-diazepan-1-yl)butyl)benzo[b]thiophen-2-ylcarbamid

Benzo[b]thiophen-2-carbonsäure (43 mg, 0,24mmol) wird mit 4-(4-(Chroman-8-yl)-1,4-diazepan-1-yl)butylamin (82 mg, 0,26 mmol) wie für Beispiel 1 beschrieben umgesetzt. Die Aufreinigung erfolgt mit Flashchromatographie (CH₂Cl₂-MeOH: 95-5.
Ausbeute: 101 mg (89 %) farbloses Öl.
(APCl)MS: m/z 464 ((M+H)⁺). IR (NaCl) v (cm⁻¹): 3319; 2925; 2852; 2817; 1631; 1545; 1242; 1215; 752. ¹H NMR (CDCl₃, 360 MHz) δ (ppm): 1.68-1.72 (m, 4H, CH₂-CH₂); 1.94-2.06 (m, 4H, N(CH₂-CH₂)N(CH₂-CH₂-CH₂)N, CH₂N(CH₂-CH₂)N(CH₂-CH₂-CH₂)N); 2.67-2.72 (m, 2H, N(CH₂-CH₂)N(CH₂-CH₂-CH₂)N); 2.78 (t, J=6.2 Hz, 2H, 0-CH₂-C.tk-CH₂); 2.89-2.92 (m, 2H, N(CH₂-CH₂)N(CH₂-CH₂-CH₂)N); 2.96-2.99 (m, 2H, N(CH₂-CH₂)N(CH₂-CH₂-CH₂)N); 3.27 (t, J=6.2 Hz, 2H, O-CH2-CH₂); 3.29-3.32 (m, 2H, N(CH₂-CH₂)N(CH₂-CH₂-CH₂)N); 3.46-3.52 (m, 2H, CH₂NHCO); 4:16-4.19 (m, 2H, O-CH₂-CH₂-CH₂); 6.63 (dd, 1H, J=6.6 Hz, J=2.7 Hz, H-Phenyl); 6.70-6.76 (m, 2H, H-Phenyl); 7.13 (brt, J=4.7, Hz, 1H, NHCO); 7.33-7.41 (m, 2H, H-5, H-6); 7.73-7.75 (m, 1H, H-4); 7.78 (brs, 1H, H-3); 7.81-7.84 (m, 1H, H-7).

### Beispiel 11: KS 478

### N-(4-(4-(Chroman-8-yl)-1,4-diazepan-1-yl)butyl)benzofuran-2-ylcarbamid

Benzofuran-2-carbonsäure (38 mg, 0,24 mmol) wird mit 4-(4-(Chroman-8-yl)-1,4-diazepan-1-y1)butylamin (79 mg, 0,26 mmol) wie für Beispiel 1 beschrieben umgesetzt. Die Aufreinigung erfolgt mit Flashchromatographie (CH₂Cl₂-MeOH: 95-5).
Ausbeute: 78mg (72 %) farbloses Öl.
(APCI)MS: m/z 448 ((M+H)⁺). IR (NaCl) v (cm⁻¹): 3305; 2939; 1657; 1595; 1520; 1219; 750. ¹H NMR (CDCl₃, 360 MHz) ö (ppm): 1.68-1.77 (m, 4H, CH₂-CH₂); 1.95-2.01 (m, 2H, O-CH₂-CH₂-CH₂,); 2.09-2.15 (m, 2H, N(CH₂-CH₂)N(CH₂-CH₂-CH₂)N); 2.78 (t, J=6.6 Hz, 2H, CH₂N(CH₂-CH₂)₂N); 2.89-2.92 (m, 2H, N(CH₂-CH₂)N(CH₂-CH₂-CH₂)N); 2.99-3.10 (m, 4H, N(CH₂-CH₂)N(CH₂-CH₂-CH₂)N); 3.29 (t, J=6.4 Hz, 2H, O-CH₂-CH₂-CH₂); 3.34-3.39 (m, 2H, N(CH₂-CH₂)N(CH₂-CH₂-CH₂)N); 3.48-3.53 (m, 2H, CH₂NHCO); 4.19 (t, J=5.2 Hz, 2H, O-CH₂-CH₂-CH₂); 6.64 (dd, 1H, J=6.1 Hz, J=3.1 Hz, H-Phenyl); 6.72-6.74 (m, 2H, H-Phenyl); 7.19-7.29 (m, 2H, NHCO, H-5); 7.38-7.40 (m, 1H, H-6); 7.42-7.44 (m, 2H, H-7, H-3); 7.63-7.66 (m, 1H, H-4).

### Beispiel 12:

### N-(4-(4-(2,3,4,5-Tetrahydrobenzo[b]oxepin-9-yl)piperazin-1-yl)butyl)benzo[b]thiophen-2-ylcarbamid

Benzo[b]thiophen-2-carbonsäure (43 mg, 0,24 mmol) wird mit 4-(4=(2,3,4,5-Tetrahydrobenzo[b]oxepin-9-yl)piperazin-1-yl)butylamin (90 mg, 0,30 mmol) wie für Beispiel 1 beschrieben umgesetzt. Die Aufreinigung erfolgt mit Flashchromatographie (CH₂Cl₂-MeOH: 97-3).
Ausbeute: 91 mg (82 %) weißer Feststoff.
Smp.: 124 °C. MS: m/z 463 (M⁺). IR (NaCl) v (cm⁻¹): 3319; 2935; 2817; 1631; 1543; 1248; 1221; 754. ¹H NMR (CDCl₃, 360 MHz) δ (ppm): 1.70-1.80 (m, 6H, CH₂-CH₂, O-CH₂-CH₂-CH₂-CH₂); 1.96-2.01 (m, 2H, O-CH₂-CH₂-CH₂-CH₂); 2.66 (t, J=6.8 Hz, 2H, CH₂N(CH₂-CH₂)₂N); 2.80-2.85 (m, 6H, N(CH₂-CH₂)₂N, O-CH₂-CH₂-CH₂-CH₂); 3.17-3.20 (m, 4H, N(CH₂-CH₂)₂N); 3.53-3.58 (m, 2H, CH₂NHCO); 3.99-4.01 (m, 2H, O-CH₂-CH₂-CH₂-CH₂); 6.76 (dd, 1H, J=7.8 Hz, J=1.7 Hz, H-Phenyl); 6.83 (dd, 1H, J=7.5 Hz, J=1.6 Hz, H-Phenyl); 6.87-6.94 (m 2H, H-Phenyl, NHCO); 7.39-7.47 (m, 2H, H-5, H-6); 7.85 (brs, 1H, H-3); 7.86-7.89 (m, 2H, H-4, H-7). ¹³C NMR (CDCl₃, 90 MHz) δ (ppm): 23.6; 26.0; 27.1; 32.4; 34.4; 39.7; 50.3; 53.7; 57.9; 73.4; 116.9; 122.7; 123.5; 124.4; 124.8; 125.0; 125.1; 126.2; 137.1; 138.7; 139.2; 140.8; 144.1; 153.6; 162.6.

### Beispiel 13:

### N-(4-(4-(2,3,4,5-Tetrahydrobenzo[b]oxepin-9-yl)piperazin-1-yl)butyl)-5-cyanbenzo[b]thiophen-2-ylcarbamid

5-Cyanbenzo[b]thiophen-2-carbonsäure (21 mg, 0,12 mmol) wird mit 4-(4-(2,3,4,5-Tetrahydrobenzo[b]oxepin-9-yl)piperazin-1-yl)butylamin (39 mg, 0,15 mmol) wie für Beispiel 1 beschrieben umgesetzt. Die Aufreinigung erfolgt mit Flashchromatographie (CH₂Cl₂-MeOH: 97-3).
Ausbeute: 26 mg (44 %) farbloses Öl.
(APCI) MS: m/z 489 ((M+H)⁺). IR (NaCl) v (cm⁻¹): 3325; 2939; 2816; 2227; 1635; 1558; 1541; 1248; 752. ¹H NMR (CDCl₃, 360-MHz) δ (ppm): 1.70-1.80 (m, 6H, CH₂-CH₂, O-CH₂-CH₂-CH₂-CH₂); 1.96-2.02 (m, 2H, O-CH₂-CH₂-CH₂-CH₂); 2.51 (t, J=6.7 Hz, 2H, CH₂N(CH₂-CH₂)₂N); 2.69-2.71 (m, 4H, N(CH₂-CH₂)₂N); 2.80-2.83 (m, 2H, O-CH₂-CH₂-CH₂-CH₂); 3.11-3.13 (m, 4H, N(CH₂-CH₂)₂N); 3.53-3.59 (m, 2H, CH₂NHCO); 3.98-4.01 (m, 2H, O-CH₂-CH₂-CH₂-CH₂); 6.68 (dd, 1H, J=7.8 Hz, J=1.7 Hz, H-Phenyl); 6.79 (dd, 1H, J=7.5 Hz, J=1.6 Hz, H-Phenyl); 6.87-6.94 (m 1H, H-Phenyl); 7.05(brt, J=5.6 Hz, NHCO); 7.60 (dd, J=8.3 Hz, J=1.5 Hz, 1H, H-5); 7.82 (brs, 1H, H-3); 7.89 (dd, J=8.3 Hz, J=0.7 Hz, 1H, H-7); 8.18-8.19 (m, 1H, H-4).

### Beispiel 14:

### N-(4-(4-(2,3,4,5-Tetrahydrobenzo[b]oxepin-9-yl)piperazin-1 yl)butyl)benzofuran-2-ylcarbamid

Benzofuran-2-carbonsäure (38 mg, 0,24 mmol) wird mit 4-(4-(2,3,4,5-Tetrahydrobenzo[b]oxepin-9-yl)piperazin-1-yl)butylamin (90 mg, 0,30 mmol) wie für Beispiel 1 beschrieben umgesetzt. Die Aufreinigung erfolgt mit Flashchromatographie (CH₂Cl₂-MeOH: 97-3).
Ausbeute: 98 mg (91 %) farbloses Öl.
(APCI) MS: m/z 448 ((M+H)⁺). IR (NaCl) v (cm⁻¹): 3313; 2935; 2816; 1655; 1595; 1520; 1250; 750. ¹H NMR (CDCl₃, 360 MHz) δ (ppm): 1.67-1.75 (m, 6H, CH₂-CH₂, O-CH₂-CH₂-CH₂-CH₂);1.94-1.98 (m, 2H, O-CH₂-CH₂-CH₂-CH₂); 2.50 (t, J=7.0 Hz, 2H, CH₂N(CH₂-, CH₂)₂N); 2.66-2.70 (m, 4H, N(CH₂-CH₂)₂N); 2.78-2.80 (m, 2H, O-CH₂-CH₂-CH₂-CH₂); 3.12-3.15 (m, 4H, N(CH₂-CH₂)₂N); 3.51-3.54 (m, 2H, CH₂NHCO); 3.97-3.99 (m, 2H, O-CH₂-CH₂-CH₂-CH₂); 6.77-6.79 (m, 2H, H-Phenyl); 6.88-6.91 (m, 1H, H-Phenyl); 7.05 (brt, J=5.3 Hz, 1H, NHCO); 7.27-7.29 (m, 1H, H-5); 7.38-7.41 (m, 1H, H-6); 7.46 (brs, 1H, H-3); 7.47-7.49 (m; 1H, H-4); 7.66-7.67 (m, 1H, H-7).

### Beispiel 15:

### N-(4-(4-(2,3,4,5-Tetrahydrobenzo[b]oxepin-9-yl)piperazin-1-yl)butyl)-5-brombenzofuran-2-ylcarbamid

5-Brombenzo[b]furan-2-carbonsäure (29 mg, 0,12 mmol) wird mit 4-(4-(2,3,4,5-Tetrahydrobenzo[b]oxepin-9-yl)piperazin-1-yl)butylamin (39 mg, 0,15 mmol) wie für Beispiel 1 beschrieben umgesetzt. Die Aufreinigung erfolgt mit Flashchromatographie (CH₂Cl₂-MeOH: 97-3).
Ausbeute: 47 mg (74 %) farbloses Öl.
(APCI) MS: m/z 527 (M+H⁺). IR (NaCl) v (cm⁻¹): 3319; 2931; 2816; 1653; 1595; 1518; 1248; 754.¹H NMR (CDCl₃, 360 MHz) δ (ppm): 1.69-1.78 (m, 6H, CH₂-CH₂, O-CH2-CH₂-CH₂-CH₂); 1.97-2.03 (m, 2H, O-CH₂-CH₋₂-CH₂-CH₂); 2.52 (t, J=6.3 Hz, 2H, CH₂N(CH₂-CH₂)₂N); 2.68-2.71 (m, 4H, N(CH₂-CH₂)₂N); 2.80-2.84 (m, 2H, O-CH₂-CH₂-CH₂-CH₂); 3.15-3.18 (m, 4H, N(CH₂-CH₂)₂N); 3.53-3.59 (m, 2H, CH₂NHCO); 4.00-4.03 (m, 2H, O-CH₂-CH₂-CH₂-CH₂); 6.75-6.78 (m, 2H, H-Phenyl); 6.87-6.91 (m, 1H, H-Phenyl); 7.10 (brt, J=6.2 Hz, 1H, NHCO); 7.35 (brd, J=8.9 Hz, 1H, H-6); 7.39 (d, J=0.9 Hz, 1H, H-3); 7.48 (d, J=1.8 Hz, J=8.9 Hz, 1H, H-7); 7.80 (d, J=1.8 Hz, 1H, H-4).

### Beispiel 16:

### N-(4-(4-(2,3,4,5-Tetrahydrobenzo(bJoxepin-9 yl)piperazin-1-yl)butyl)indol-2-ylcarbamid

indol-2-carbonsäure (19 mg, 0,12 mmol) wird mit 4-(4-(2,3,4,5-Tetrahydrobenzo[b]oxepiri-9-yl)piperazin-1-yl)butylamin (39 mg, 0,13 mmol) wie für Beispiel 1 beschrieben umgesetzt. Die Aufreinigung erfolgt mit Flashchromatographie (CH₂Cl₂-MeOH: 95-5).
Ausbeute: 43 mg (80 %) farbloses Öl.
(APCI) MS: m/z 447 ((M+H)⁺). IR (NaCl) v (cm⁻¹): 3257; 2935; 2817; 1633; 1556; 1248; 733. ¹H NMR (CDCl₃, 360 MHz) δ (ppm): 1.71-1.82 (m, 6H, CH₂-CH₂, O-CH₂-CH₂-CH₂-CH₂); 1.97-2.03 (m, 2H, O-CH₂-CH₂-CH₂-CH₂); 2.63 (t, J=7.0.Hz, 2H, CH₂N(CH₂-CH₂)₂N); 2.78-2.84 (m, 6H, N(CH₂-CH₂)₂N, O-CH₂-CH₂-CH₂-CH₂); 3.21-3.23 (m, 4H, N(CH₂-CH₂)₂N); 3.55-3.61 (m, 2H, CH₂NHCO); 3.99-4.02 (m, 2H, O-CH₂-CH₂-CH₂-CH₂); 6.80 (dd, 1H, J=7.9 Hz, J=1.8 Hz, H-Phenyl); 6.84 (dd, 1H, J=7.6 Hz, J=1.7 Hz, H-Phenyl); 6.92-6.96 (m, 1H, H-Phenyl); 6.97 (m, 1H, NHCO);6.99 (dd, J=0.9 Hz, J=2.0 Hz, H-3); 7.16 (ddd, J=8.0 Hz, J=7.0 Hz, J=1.1 Hz, 1H, H-5); 7.31 (ddd, J=8.0 Hz, J=7.0 Hz, J=1.1 Hz, 1H, H-6); 7.47 (dd, J=8.1 Hz, J=0.9 Hz, 1 H. H-/); 7.68 (dd, J=8.1 Hz, J=0.9 Hz, 1H, H-4); 9.56 (brs, 1H, NH).

### Beispiel 17:

### N-(4-(4-(2,3,4,5-Tetrahydrobenzo[b]oxepin-9-yl)piperazin-1-yl)butyl)-6-cyanindol-2-ylcarbamid

6-Cyanindol-2-carbonsäure (22 mg, 0,12 mmol) wird mit 4-(4-(2,3,4,5-Tetrahydrobenzo[b]oxepin-9-yl)piperazin-1-yl)butylamin (39 mg, 0,13 mmol) wie für Beispiel 1 beschrieben umgesetzt. Die Aufreinigung erfolgt mit Flashchromatographie (CH₂Cl₂-MeOH: 95-5).
Ausbeute: 36 mg (64 %) farbloses Öl
(APCI) MS: m/z 472 ((M+H)⁺). IR (NaCl) v (cm⁻¹): 3234; 2935; 2817; 2220; 1641; 1552; 1323; 1248; 752. ¹H NMR (CDCl₃, 360 MHz) δ (ppm): 1.69-1.80 (m, 6H, CH₂-CH₂, O-CH₂-CH₂-CH₂-CH₂); 1.93-1.98 (m, 2H, O-CH₂CH₂-CH₂-CH₂); 2.54 (t, J=6.7 Hz, 2H, CH₂N(CH₂-CH₂)₂N); 2.69-2.72 (m, 4H, N(CH₂-CH₂)₂N); 2.77-2.80 (m, 2H, O-CH₂-CH₂-CH₂-CH₂); 3.12-3.15 (m, 4H, N(CH₂-CH₂)₂N); 3.58-3.63 (m, 2H, CH₂NHCO); 3.95-3.99 (m, 2H, O-CH₂-CH₂-CH₂-CH₂); 6.74 (dd, 1H, J=7.6 Hz, J=1.6 Hz, H-Phenyl); 6.79 (dd, 1H, J=7.6 Hz, J=1.6 Hz, H-Phenyl); 6.87-6.91 (m, 1H, H-Phenyl); 6.96 (brs, 1H, H-3); 7.09 (brt, J=5.4 Hz, 1H, NHCO); 7.34 (dd, J=8.3 Hz, J=1.4 Hz, H-5); 7.70 (d, J=8.3 Hz, 1H, H-4); 7.83 (brs, 1H, H-7); 10.66 (brs, 1H, NH). ¹³C NMR (CDCl₃, 90 MHz) δ (ppm): 24.1; 26.1; 27.2; 32.5; 34.5; 39.6; 50.7; 53.7; 57.8; 73.4; 102.4; 106.7; 116.9; 117.3; 120.1; 122.8; 123.0; 123.5; 124.2; 130.6; 134.4; 135.1; 137.1; 144.4; 153.5; 161.1.

### Beispiel 18:

### N-(4-(4-(2,3,4,5-Tetrahydrobenzo[b]oxepin-9-yl)-1,4-diazepan-1-yl)butyl)benzo[b]thiophen-2-ylcarbamid

Benzo[*b*]thiophen-2-carbonsäure (43 mg, 0,24 mmol) wird mit 4-(4-(2,3,4,5-Tetrahydrobenzo[b]oxepin-9-yl)-1,4-diazepan-1-yl)butylamin (83 mg, 0,26 mmol) wie für Beispiel 1 beschrieben umgesetzt. Die Aufreinigung erfolgt mit Flashchromatographie (CH₂Cl₂-MeOH: 95-5).
Ausbeute: 101 mg (88 %) farbloses Öl.
(APCI) MS: m/z 478 ((M+H)⁺). IR (NaCl) v (cm⁻¹): 3250; 2939; 2839;1643; 1543; 750. ¹H NMR (CDCl₃, 360 MHz) δ (ppm): 1.69-1.73 (m, 2H, O-CH₂-CH₂-C₋H₂-CH₂); 1.78-1.82 (m 2H, CH₂-CH₂); 1.93-1.96 (m, 2H, O-CH₂-CH₂-CH₂-CH₂); 2.02-2.06 (m, 2H, CH₂-CH₂); 2.43-2.46 (m, 2H, N(CH₂-CH₂)N(CH₂-CH₂-CH₂)N); 2.77-2.79 (m, 2H, O-CH₂-CH₂-CH₂-CH₂); 3.09 (t, J=7.6 Hz, 2H, CH₂N(CH₂-CH₂)N(CH₂-CH₂-CH₂)N); 3.25-3.28 (m, 2H, N(CH₂-CH₂)N(CH₂-CH₂-CH₂)N); 3.36-3.41 (m, 4H, N(CH₂-CH₂)N(CH₂-CH₂-CH₂)N); 3.46-3.48 (m, 2H, N(CH₂-CH₂)N(CH₂-CH₂-CH₂)N); 3.55-3.59 (m, 2H, OCNH-C,H₂); 3.88-3.91 (m, 2H, O= CH₂-CH₂-CH₂-CH₂); 6.74 (d, 1H, J=8.0 Hz, H-Phenyl); 6.76 (d, 1H, J=8.0 Hz, H-Phenyl); 6.87-6.90 (m, 1H, H-Phenyl); 7.33-7.39 (m, 2H, H-5, H-6); 7.83 (d, J=7.9 Hz, 1H, H-7); 7.85 (d, J=7.6 Hz, 1H, H-4); 8.09 (brt, J=6.0 Hz, 1H, NHCO); 8.21 (s, 1H, H-3). ¹³C NMR (CDCl₃, 90 MHz) ö (ppm): 21.6; 24.4; 25.9; 26.0; 32.3; 34.5; 38.0; 49.3; 49.5; 52.6; 56.5; 73.5; 116.5; 122.5; 123.5; 123.7; 124.6; 125.3; 125.6; 126.0; 137.2; 139.3; 139.6; 141.1; 144.5; 152.3; 162.9.

### Beispiel 19:

### N-(4-(4-(2,3,4,5-Tetrahydrobenzo[b]oxepin-9-yl)- 1,4-diazepan-1-yl)butyl)benzofuran-2-ylcarbamid

Benzofuran-2-carbonsäure (39 mg, 0,24 mmol) wird mit 4-(4-(2,3,4,5-Tetrahydrobenzo[b]oxepin-9-yl)-1,4-diazepan-1-yl)butylamin (83 mg, 0,26 mmol) wie für Beispiel 1 beschrieben umgesetzt. Die Aufreinigung erfolgt mit Flashchromatographie (CH₂Cl₂-MeOH: 95-5).
Ausbeute: 100 mg (90 %) farbloses Öl.
(APCl) MS: m/z 462 ((M+H)⁺). IR (NaCl) v (cm⁻¹): 3271; 2931; 2864; 1653; 1593; 1470; 750. ¹H NMR (CDCl₃, 360 MHz) δ (ppm): 1.69-1.73 (m, 2H, CH₂-CH₂); 1.78-1.82 (m 2H, CH₂-CH₂); 1.93-1.96 (m, 2H, O-CH₂-CH₂-CH₂-CH₂); 2.02-2.06 (m, 2H, O-CH₂-CH₂-CH₂-CH₂); 2.43-2.46 (m, 2H, N(CH₂-CH₂)N(CH₂-CH₂-CH₂)N); 2.77-2.79 (m, 2H, O-CH₂-CH₂-CH₂-CH₂); 3.09 (t, J=7.6 Hz, 2H, CH₂-N(CH₂-CH₂)N(CH₂-CH₂-CH₂)N); 3.25-3.28 (m, 2H, N(CH₂-CH₂)N(CH₂-CH₂-CH₂)N); 3.36-3.41 (m, 4H, N(CH₂-CH₂)N(CH₂-CH₂-CH₂)N); 3.46-3.48 (m, 2H, N(CH₂-CH₂)N(CH₂-CH₂-CH₂)N); 3.55-3.59 (m, 2H, CH₂NHCO); 3.88-3.91 (m, 2H, O-CH₂-CH₂-CH₂-CH₂); 6.74 (d, 1H, J=8.3 Hz, H-Phenyl); 6.76 (d, 1H, J=7.6 Hz, H-Phenyl); 6.87-6.90 (m, 1H, H-Phenyl); 7.33-7.39 (m, 2H, H-5, H-6); 7.83 (d, J=7.9 Hz, 1H, H-4); 7.85 (d, J=7.6 Hz, 1H, H-7); 8.09 (brt, J=6.0 Hz, 1H, NHCO); 8.21 (s, 1H, H-3). ¹³C NMR (CDCl₃, 90 MHz) ö (ppm): 21.7; 24.4; 26.0; 26.8; 32.3; 34.5; 38.0; 49.4; 49.6; 52.6; 56.9; 57.2; 73.4; 110.2; 111.9; 116.5; 1.22.6; 123.5; 123.5; 123.6; 126.8; 127.6; 137.2; 144.6; 148.6; 152.3; 154.8; 159.3.

### Beispiel 20:

### N-(4-(4-(Chroman-7-yl)piperazin-1-yl)butyl)benzo[b]thiophen-2-ylcarbamid

Benzo[b]thiophen-2-carbonsäure (21 mg, 0,12 mmol) wird mit 4-(4-(Chroman-7-yl)piperazin-1-yl)butylamin (38 mg, 0,13 mmol) wie für Beispiel 1 beschrieben umgesetzt.
Die Aufreinigung erfolgt mit Flashchromatographie (CH₂Cl₂-MeOH: 97-3).
Ausbeute: 40 mg (74 %) farbloses Öl.
(APCI) MS: m/z 450 ((M+H)⁺). IR (NaCl) v (cm⁻¹): 3284; 2929; 2856; 2817; 1624; 1512; 1294; 750. ¹H NMR (CDCl₃, 360 MHz) δ (ppm): 1.64-1.74 (m, 4H, CH₂-CH₂); 1.95-1.99 (m, 2H, O-CH₂-CH₂-CH₂); 2.46 (t, J=7.0 Hz, 2H, CH₂(CH₂-CH₂)₂N); 2.59-2.62 (m, 4H, N(CH₂-CH₂)₂N); 2.70 (t, J=6.4, 2H, O-CH₂-CH₂-CH₂); 3.14-3.16 (m, 4H, N(CH₂-CH₂)₂N); 3.49-3.53 (m, 2H, CH₂NHCO); 4.14-4.16 (m, 2H, O-CH₂-CH₂-CH₂); 6.33 (d, J=2.4 Hz, 1H, H-Phenyl); 6.44 (dd, J=8.3 Hz, J=2.4 Hz, 1H, H-Phenyl); 6.56 (brt, J=4.0 Hz, 1H, NHCO); 6.90 (d, J=8.3 Hz, 1H, H-Phenyl); 7.37-7.43 (m, 2H, H-5, H-6); 7.76 (brs, 1H, H-3); 7.81 (d, J=7.2 Hz, 1H, H-4); 7.84 (d, J=8.3 Hz, 1H, H-7). ¹³C NMR (CDCl₃, 90 MHz) δ (ppm): 22.6; 24.1; 24.6; 27.4; 40.0; 49.1; 53.2; 57.9; 66.5; 103.9; 109.0; 113.6; 122.7; 124.9; 125.0; 125.1; 126.2; 130.1; 138.6; 139.1; 140.7; 150.8; 155.3; 162.3.

### Beispiel 21:

### N-(4-(4-(2,3-Dihydrobenzofuran-5-yl)piperazin-1-yl)butyl)benzo[b]thiophen-2-ylcarbamid

Benzo[b]thiophen-2-carbonsäure (43 mg, 0,24 mmol) werden mit 4-(4-(2,3-Dihydrobenzofuran-5-yl)piperazin-1-yl)-butylamin (80 mg, 0,27 mmol) wie für Beispiel 1 beschrieben umgesetzt. Die Aufreinigung erfolgt mit Flashchromatographie (CH₂Cl₂-MeOH: 97-3).
Ausbeute: 100 mg (96 %) farbloses Öl.
(APCI) MS m/z 436 ((M+H)⁺). IR (NaCl) v (cm⁻¹): 3070; 2937; 2814; 1626; 1543; 1491; 1217; 752. ¹H NMR (CDCl₃, 360 MHz) δ (ppm): 1.64-1.74 (m, 4H, CH₂-CH₂); 2.47 (t, J=7.2 Hz, 2H, CH₂N(CH₂-CH₂)₂N); 2.61-2.63 (m, 4H, N(CH₂-CH₂)₂N); 3.05-3.07 (m, 4H, N(CH₂-CH₂)₂N); 3.16 (t, J=8.3 Hz, OCH₂CH₂); 3.49-3.53 (m, 2H, CH₂NHCO); 4.52 (t, J=8.3 Hz, 2H, OCH₂CH₂); 6.62 (br t J=5.1 Hz, 1H, NHCO); 6.67-6.68 (m, 2H, H-Phenyl); 6.82-6.83 (m, 1H, H-Phenyl); 7.37-7.43 (m, 2H, H-5, H-6); 7.76 (brs, 1H, H-3); 7.81 (d, J=7.9 Hz, 1H, H-4); 7.84 (d, J=7.9Hz, 1H, H-7). ¹³C-NMR (CDCl₃, 90 MHz) δ (ppm): 24.4; 27.5; 30.2; 40.0; 49.4; 51.2; 53.5; 57.9; 71.1; 109.1; 114.9; 117.1; 122.7; 124.8, 124.9, 125.0; 126.2; 127.6; 138.7; 139.1; 140.7; 146.0; 154:5; 162.4.

### Beispiel 22:

### N-(4-(4-(Chroman-6-yl)piperazin-1 yl)bufyl)benzo[b]thiophen-2-ylcarbamid

Benzo[*b*]thiophen-2-carbonsäure (42 mg, 0,24 mmol) wird mit 4-(4-(Chroman-6-yl)piperazin-1-yl)butylamin (80 mg, 0,28 mmol) wie für Beispiel 1 beschrieben umgesetzt. Die Aufreinigung erfolgt mit Flashchromatographie (CH₂Cl₂-MeOH: 97-3).
Ausbeute: 100 mg (93 %) farbloses Öl.
(APCl) MS: m/z 450 ((M+H)⁺). IR (NaCl) v (cm⁻¹): 3315; 2937; 2816; 1630; 1543; 1502; 1227; 754. ¹H NMR (CDCl₃, 360 MHz) δ (ppm): 1.65-1.74 (m, 4H, CH₂-CH₂); 1.96-1.99 (m, 2H, O-CH₂-CH₂-CH₂); 2.48 (t, J=7.0 Hz, 2H, CH₂N(CH₂-CH₂)₂N); 2.62-2.64 (m, 4H, N(CH₂-CH₂)₂N); 2.74 (t, J=6.4, 2H, O-CH₂-CH₂-CH₂); 3.07-3.09 (m, 4H, N(CH₂-CH₂)₂N); 3.49-3.53 (m, 2H, CH₂NHCO); 4.13 (t, J=5.1 Hz, 2H, O-CH₂-CH₂-CH₂); 6.59-6.60 (m, 1H, H-Phenyl); 6.64 (brt, J=6.0 Hz, 1H, NHCO); 6.70-6.71 (m, 2H, H-Phenyl); 7.37-7.43 (m, 2H, H-5, H-6); 7.76 (brs, 1H, H-3); 7.81 (d, J=7.6 Hz, 1H, H-4); 7.84 (d, J=7.9 Hz, 1H, H-7). ¹³C NMR (CDCl₃, 90 MHz) δ (ppm): 22.6; 24.3; 25.2; 27.4; 39.9; 50.5; 53.4; 57.9; 66.3; 116.8; 117.0; 118.2; 122.4; 122.7; 124.8; 124.9; 125.1; 126.2; 138.7; 139.1; 140.7; 145.0; 149.2; 162.4.

### Beispiel 23:

### N-(4-(4-(2,3,4,5-Tetrahydrobenzo[b]oxepin-7-yl)piperazin-1-yl)butyl)benzo[b]thiophen-2-ylcarbamid

Benzo[*b*]thiophen-2-carbonsäure (42 mg, 0,24 mmol) wird mit 4-(4-(2,3,4,5-Tetrahydrobenzo[b]oxepin-7-yl)piperazin-1-yl)butylamin (80 mg, 0,25 mmol) wie für Beispiel 1 beschrieben umgesetzt. Die Aufreinigung erfolgt mit Flashchromatographie (CH₂Cl₂-MeOH: 97-3).
Ausbeute: 54 mg (49 %) farbloses Öl.
(APCI) MS: m/z 464 ((M+H)⁺). IR (NaCl) v (cm⁻¹): 3321; 2931; 2817; 1630; 1544; 1502; 1232; 771; 729. ¹H NMR (CDCl₃, 360 MHz) δ (ppm): 1.64-1.75 (m, 6H, CH₂-CH₂, O-CH₂-CH₂-CH₂-CH₂); 1.90-1.96 (m, 2H, O-CH₂-CH₂-CH₂-CH₂); 2.46 (t, J=6.9 Hz, 2H, CH₂N(CH₂-CH₂)₂N); 2.59-2.62 (m, 4H, N(CH₂-CH₂)₂N); 2.74-2.77 (m, 2H, O-CH₂-CH₂-CH₂-CH₂); 3.11-3.14 (m, 4H, N(CH₂-CH₂)₂N); 3.48-3.53 (m, 2H, CH₂NHCO); 3.92-3.95 (m, 2H, O-CH₂-CH₂-CH₂-CH₂); 6.61 (brt, J=5.1 Hz, 1H, NHCO); 6.64 (dd, 1H, J=8.5 Hz, J=3.0 Hz, H-Phenyl); 6.68 (d, 1H, J=3.0 Hz, H-Phenyl); 6.88 (d, J=8.5 Hz, 1H, H-Phenyl); 7.36-7.44 (m, 2H, H-5, H-6); 7.76 (d, J=0.7 Hz, 1H, H-3); 7.79-7.85 (m, 2H, H-4, H-7).

### Beispiel 24:

### N-(4-(4-(Benzo[1,3]dioxol-4-yl)piperazin-1-yl)butyl)benzo[b]thiophen-2-ylcarbamid

Benzo[b]thiophen-2-carbonsäure (42 mg, 0,24 mmol) wird mit 4-(4-(Benzo[1,3]dioxol-4-yl)piperazin-1-yl)butylamin (70 mg, 0,25 mmol) wie für Beispiel 1 beschrieben umgesetzt. Die Aufreinigung erfolgt mit Flashchromatographie (CH₂Cl₂-MeOH: 97-3).
Ausbeute: 105 mg (99 %) weißer Feststoff.
Smp.: 184 °C. (APCI) MS: m/z 438 ((M+H)⁺). IR (NaCl) v (cm⁻¹): 3315; 2935; 2883; 2814; 1630; 1539; 1452; 1255; 759. ¹H NMR (CDCl₃, 360 MHz) δ (ppm): 1.63-1.68 (m, 2H, CH₂-CH₂); 1.69-1.73 (m, 2H, CH₂-CH₂); 2.46 (t, J=7.2 Hz, 2H, CH₂N(CH₂-CH₂)₂N); 2.61-2.62 (m, 4H, N(CH₂-CH₂)₂N); 3.19-3.20 (m, 4H, N(CH₂-CH₂)₂N); 3.49-3.52 (m, 2H, CH₂NHCO); 5.92 (s, 2H, O-CH₂-O); 6.40 (dd, J=8.3 Hz, J=0.8 Hz, 1H, H-Phenyl); 6.52 (dd, J=8.3 Hz, J=0.8 Hz, 1H, H-Phenyl); 6.59 (brt, J=5.9 Hz, 1H, NHCO); 6.74-6.77 (m, 1H, H-Phenyl); 7.37-7.43 (m, 2H, H-5, H-6); 7.76 (brs, 1H, H-3); 7.81 (d, J=7.9 Hz, 1H, H-4); 7.84 (d, J=7.9 Hz, 1H, H-7).

### Beispiel 25:

### N-(4-(4-(Benzo[1,3]dioxol-5- yl)piperazin-1- yl)butyl)benzo[b]thiophen- 2-ylcarbamid

Benzo[*b*]thiophen-2-carbonsäure (42 mg, 0,24 mmol) wird mit 4-(4-(Benzo[1,3]dioxol-5, yl)piperazin-1-yl)butylamin (80 mg, 0,27 mmol) wie für Beispiel 1 beschrieben umgesetzt Die Aufreinigung erfolgt mit Flashchromatographie (CH₂Cl₂-MeOH: 97-3).
Ausbeute: 96 mg (92 %) weißer Feststoff.
Smp.: 182-184 °C. MS: m/z 437 (M⁺). IR (NaCl) v (cm⁻¹): 3313; 2939; 2877; 2819; 2773; 1620; 1549; 1502; 1221; 754. ¹H NMR (CDCl₃, 360 MHz) δ (ppm): 1.63-1.68 (m, 2H, CH₂-CH₂); 1.69-1.73 (m, 2H, CH₂-CH₂); 2.45 (t, J=7.0 Hz, 2H, CH₂N(CH₂-CH₂)₂N); 2.59-2.61 (m, 4H, N(CH₂-CH₂)₂N); 3.06-3.07 (m, 4H, N(CH₂-CH₂)₂N); 3.49-3.52 (m, 2H, CH₂NHCO); 5.89 (s, 2H, O-CH₂-O); 6.32 (dd, J=8.6Hz, J=2.4 Hz, 1H, H-Phenyl); 6.53 (d, J=2.4 Hz, 1H, H-Phenyl); 6.60 (brt, J=4.5 Hz,1H, NHCO); 6.70 (d, J=8.6 Hz, 1H, H-Phenyl); 7.37-7.42 (m, 2H, H-5, H-6); 7.75 (brs, 1H, H-3); 7.80 (d, J=7.6 Hz, 1H, H-4); 7.84 (d, J=8.3 Hz, 1H, H-7). ¹³C NMR (CDCl₃, 90 MHz) δ (ppm): 24.4; 27.5; 40.1; 50.7; 53.3; 57.9; 99.9; 100.8; . 108.1; 109.0; 122.7; 124.9; 124.93; 125.1; 126.2; 138.6; 139.1; 140.7; 141.6; 147.4; 148.2; 162.4.

### Beispiel 26:

### N-(4-(4-(2,3-Dihydrobenzo[1,4]dioxin-6-yl)piperazin-1-yl)butyl)benzo[b]thiophen-2-ylcarbamid

Benzo[b]thiophen-2-carbonsäure (21 mg, 0,12 mmol) wird mit 4-(4-(2,3-Dihydrobenzo[1,4]dioxin-6-yl)piperazin-1-yl)butylamin (50 mg, 0,17 mmol) wie für Beispiel 1 beschrieben umgesetzt. Die Aufreinigung erfolgt mit Flashchromatographie (CH₂Cl₂-MeOH: 97-3).
Ausbeute: 30 mg (56 %) weißer Feststoff.
Smp.: 141-143 °C. (APCI) MS: m/z 452 ((M+H)⁺). IR (NaCl) v (cm⁻¹): 3278; 2966; 2935; 2871; 2823; 1618; 1564; 1508; 1224; 750. ¹H NMR (CDCl₃; 360 MHz) δ (ppm): 1.63-1.71 (m, 4H, CH₂-CH₂); 2.45 (t, J=6.8 Hz, 2H, CH₂N(CH₂-CH₂)₂N); 2.56-2.61 (m, 4H, N(CH₂-CH₂)₂N); 3.06-3.09 (m, 4H, N(CH₂-CH₂)₂N); 3.48-3.53 (m, 2H, CH₂NHCO); 4.19-4.25 (m, 4H, O-CH₂-CH₂-O); 6.42-4.44 (m, 2H, H-Phenyl); 6.57 (brt, J=4.5 Hz, 1H, NHCO); 6.75-6.77 (m, 1H, H-Phenyl); 7.36-7.44 (m, 2H, H-5, H-6); 7.75 (brs, 1H, H-3); 7.79-7.85 (m, 2H, H-4, H-7). ¹³C NMR (CDCl₃, 90 MHz) δ (ppm): 24.3; 27.4; 40.0; 50.1; 53.3; 57.9; 64.2; 64.6; 105.7; 110.3; 117.3; 122.7; 124.8; 124.9; 125.1; 126.2; 137.4; 138.6; 139.1; 140.7; 143.6; 146.4; 162.3.

### Beispiel 27:

### N-(4-(4-(2,3-Dihydrobenzo[1,4]dioxin-6- yl)-1,4-diazepan-1- yl)butyl)benzo[b]thiophen-2-ylcarbamid

Benzo[b]thiophen-2-carbonsäure (18 mg, 0,10 mmol) wird mit 4-(4-(2,3-Dihydrobenzo[1,4]dioxin-6-yl)-1,4-diazepan-1-yl)butylamin (39 mg, 0,13 mmol) wie für Beispiel 1 beschrieben umgesetzt. Die Aufreinigung erfolgt mit Flashchromatographie (CH₂Cl₂-MeOH: 97-3).
Ausbeute: 35 mg (75 %) farbloses Öl.
MS: m/z 465 ((M+H)⁺). IR (NaCl) v (cm⁻¹): 2933; 2868; 2824; 1628; 1543; 1510; 1288; 754.
¹H NMR (CDCl₃, 360 MHz) δ (ppm): 1.59-1.71 (m, 4H, CH₂-CH₂); 1.94-2.00 (m, 2H, N(CH₂-CH₂)N(CH₂-CH₂-CH₂)N); 2.58 (t, J=6.2 Hz, 2H, CH₂N(CH₂-CH₂)N(CH₂-CH₂-CH₂)N); 2.67-2.70 (m, 2H, N(CH₂-CH₂)N(CH₂-CH₂-CH₂)N); 2.80-2.83 (m, 2H, N(CH₂-CH₂)N(CH₂-CH₂-CH₂)N); 3.39 (t, J=6.2 Hz, 2H, CH₂NHCO); 3.45-3.50 (m, 4H, N(CH₂-CH₂)N(CH₂-CH₂-CH₂)N); 4.16-4.24 (m, 4H, O-CH₂-CH₂-O); 6.18-6.21(m, 2H, H-Phenyl); 6.64-6.75 (m, 2H, NHCO, H-Phenyl); 7.36-7.44 (m, 2H, H-5, H-6); 7.78 (brs, 1H, H-3); 7.80-7.86 (m, 2H, H-4, H-7).

### Beispiel 28:

### N-(4-(4-(3,4-Dihydro-2H-benzo[b][1,4]dioxepin-7-yl)piperazin- 1-yl)butyl)benzo[b]thiophen-2-ylcarbamid

Benzo[b]thiophen-2-carbonsäure (43 mg, 0,24 mmol) wird mit 4-(4-(3,4-Dihydro-2H-benzo[b][1,4]dioxepin-7-yl)piperazin-1-yl)butylamin (80 mg, 0,26 mmol) wie für Beispiel 1 beschrieben umgesetzt. Die Aufreinigung erfolgt mit Flashchromatographie (CH₂Cl₂-MeOH: 97-3).
Ausbeute: 105 mg (94 %) weißer Feststoff.
Smp.: 138 °C. (APCI) MS: m/z 466 ((M+H)⁺). IR (NaCl) v (cm⁻¹): 3284; 2964; 2937; 2845; 1616; 1564; 1504; 1298; 1226; 750. ¹H NMR (CDCl₃, 360 MHz) ö (ppm): 1.66-1.73 (m, 4H, CH₂-CH₂); 2.13-2.17 (m, 2H, (O-CH₂-CH₂-CH₂-O); 2.52 (t, J=7.0 Hz, 2H, CH₂N(CH₂-CH₂)₂N); 2.65-2.67 (m, 4H, N(CH₂-CH₂)₂N); 3.13-3.14 (m, 4H, N(CH₂-CH₂)₂N); 3.49-3.52 (m, 2H, CH₂NHCO); 4.12 (t, J=5.8 Hz, 2H, O-CH₂-CH₂-CH₂-O); 4.17 (t, J=5.8 Hz, O-CH₂-CH₂-CH₂-O); 6.48 (dd, J=8.8 Hz, J=2.9 Hz, 1H, H-Phenyl); 6.55 (d, J=2.9 Hz, 1H, H-Phenyl); 6.68 (brt, J=5.3 Hz, 1H, NHCO); 6.88 (d, J=8.8 Hz, 1H, H-Phenyl); 7.37-7.42 (m, 2H, H-5, H-6); 7.76 (brs, 1H, H-3); 7.80 (brd, J=7.2 Hz, 1H, H-4); 7.84 (brd, J=7.9 Hz, 1H, H-7). ¹³C NMR (CDCl₃, 90 MHz) δ (ppm): 23.8; 27.2; 32.3; 39.8; 49.3; 49.6; 53.1; 57:8; 70.7; 70.8; 109.7; 111.4; 121.9; 122.7; 124.9; 125.0; 125.1; 126.2; 138.7; 139.1; 140.8; 145.0; 147.4; 151.7; 162.4.

### Beispiel 136:

### N-(4-(4-(2,3,4,5-TetrahydrobenzojbJoxepin-9-yl)piperazin-7 yl)butyl)-3-chlorbenzo[b]thiophen-2-ylcarbamid

Zu einer Lösung von 3-Chlorbenzo[b]thiophen-2-carbonsäurechlorid (28 mg, 0,12 mmol) in trockenem Methylenchlorid (5 ml) werden 0,07 ml DIPEA (0,42 mmol) zugegeben und auf 0°C abgekühlt. Anschließend werden in 5 ml Methylenchlorid gelöstes 4-(4-(2,3,4,5-Tetrahydrobenzo[b]oxepin-9-yl)piperazin-1-yl)butylamin (39 mg, 0,13 mmol) zugetropft, das Reaktionsgemisch für 1 Stunde bei Raumtemperatur gerührt, danach mehrmals mit gesättigter Natriumhydrogencarbonatlösung ausgeschüttelt und die vereinigten wässrigen Phasen erneut mit Methylenchlorid extrahiert. Die gesammelten organischen Phasen werden mit gesättigter Natriumchloridlösung gewaschen, mit Magnesiumsulfat getrocknet und am Rotationsverdampfer eingeengt. Der Rückstand wird mittels Flashchromatographie gereinigt (CH₂Cl₂-MeOH: 90-10).
Ausbeute: 40 mg (67 %) farbloses Öl.
(APCI) MS: m/z 499 ((M+H)⁺). IR (NaCl) v (cm⁻¹): 3428; 2935; 2816; 1647; 1533; 1246; 752. ¹H NMR (CDCl₃, 600 MHz) δ (ppm): 1.67-1.77 (m, 6H, CH₂-CH₂, O-CH₂-CH₂-CH₂-CH₂); 1.94-1.97 (m, 2H, O-CH₂-CH2-CH₂-CH₂); 2.48-2.50 (m, 2H, CH₂N(CH₂-CH₂)₂N); 2.63-2.66 (m, 4H, N(CH₂-CH₂)₂N); 2.78-2.79 (m, 2H, O-CH₂-CH₂-CH₂-CH₂); 3.06-3.09 (m, 4H, N(CH₂-CH₂)₂N); 3.55-3.58 (m, 2H, CH₂NHCO); 3.96-3.98 (m, 2H, O-CH₂-CH₂-CH₂-CH₂); 6.71 (dd, 1H, J=7.8 Hz, J=1.3 Hz, H-Phenyl); 6.77 (dd, H, J=7.8 Hz, J=1.3 Hz, H-Phenyl); 6.86-6.89 (m 1H, H-Phenyl); 7.34 (brt, J=4.5 Hz, NHCO); 7.48-7.50 (m, 2H, H-5, H-6); 7.86-7.88 (m, 1H, H-4).

### Beispiel 137:

### N-(4-(4-(2,3,4,5-Tetrahydrobenzo[b]oxepin-9-yl)piperazin-1-yl)butyl)-6-ethinylbenzo[b]thiophen-2-ylcarbarnid

6-Ethinylbenzo[b]thiophen-2-carbonsäure (20 mg, 0,09 mmol) wird mit 4-(4-(2,3,4,5-Tetrahydrobenzo[b]oxepin-9-yl)piperazin-1-yl)butylamin (33 mg, 0,10 mmol) wie für Beispiel 1 beschrieben umgesetzt. Die Aufreinigung erfolgt mit Flashchromatographie (CH₂Cl₂-MeOH: 90-10).
Ausbeute: 19 mg (43 %) farbloses Öl.
(APCI) MS: m/z 488 ((M+H)⁺). IR (NaCl) v (cm⁻¹): 3291; 2927; 2856; 1633; 1562; 1277; 752. ¹H NMR (CDCl₃, 600 MHz) δ (ppm): 1.69-1.75 (m, 6H, CH₂-CH₂, O-CH₂-CH₂-CH₂-CH₂ ); 1.94-1.98 (m, 2H, O-CH₂-CH₂-CH₂-CH₂); 2.57-2.61 (m, 2H, CH₂N(CH₂CH₂)₂N); 2.74-2.76 (m, 4H, N(CH₂-CH₂)₂N); 2.77-2.79 (m, 2H, O-CH₂-CH₂-CH₂-CH₂); 2.80 (s, 1H, CCH); 3.12-3.15 (m, 4H, N(CH₂-CH₂)₂N); 3.50-3.53 (m, 2H, CH₂NHCO); 3.96-3.98 (m, 2H, O-CH₂-CH₂-CH₂-CH₂); 6.71 (dd, 1H, J=7.7 Hz, J=1.5 Hz, H-Phenyl); 6.80 (dd, 1H, J=7.7 Hz, J=1.5 Hz, H-Phenyl); 6.88-6.92 (m 2H, H-Phenyl, NHCO); 7.47 (dd, J=8.3 Hz, J=1.1 Hz, 1H, H-5); 7.74-7.78 (m, 2H, H-4, H-3), 7.99 (brs, 1H, H-7).

### Beispiel 158:

### N-(4-(4-(6-Chlor-3-oxo-3,4-dihydro-2H-benzo[1,4]oxazin-8-yl)piperazin-1-yl)butyl)benzo[b]thiophen-2-ylcarbamid

Benzo[b]thiophen-2-carbonsäure (21 mg, 0,12 mmol) wird mit 8-(4-(4-Aminobutyl)piperazin-1-yl)-6-chlor-3,4-dihydro-2*H*-benzo[1,4]oxazin-3-on (44 mg, 0,13 mmol) wie für Beispiel 1 beschrieben umgesetzt. Die Aufreinigung erfolgt mit Flashchromatographie (CH₂Cl₂-MeOH: 95-5).
Ausbeute: 47 mg (79 %) farbloses Öl.
(APCI) MS: m/z 499 ((M+H)⁺). IR (NaCl) v (cm⁻¹): 2987; 2945; 1699; 1624; 1475; 1296; 1230; 750. ¹H NMR (CDCl₃, 360 MHz) δ (ppm): 1.66-1.76 (m, 4H, CH₂-CH₂); 2.49 (t, J=7:0 Hz, 2H, CH₂N); 2.60-2.63 (m, 4H, pip); 3.09-3.12 (m. 4H, pip); 3.51-3.56 (m, 2H, CH₂NHCO); 4.63 (s, 2H, O-CH₂-CONH); 6.53 (d, J=2.8 Hz, 1H, H-5'); 6.55 (d, J=2.8 Hz, 1H, H-7'); 6.82-6.85 (m, 1H, CH₂-NHCO); 7.39-7.45 (m, 2H, H-5, H-6); 7.81-7.87 (m, 3H, H-4, H-3, H-7); 8.99 (brs, 1H, NHCO-CH₂-O)

### SYNTHESE WEITERER MÖGLICHER AUSFÜHRUNGSBEISPIELE:

Weitere Ausführungsbeispiele können synthetisiert werden in dem eine Heteroarencarbonsäure vom Typ A1 wie Benzo[b]thiophen-2-carbonsäure; 5-Brombenzo[b]thiophen-2-carbonsäure; Benzo[b]thiophen-3-carbonsäure; Benzofuran-2-carbonsäure; Indol-2-carbonsäure; Indol-3-carbonsäure oder vom Typ A2 wie Benzofuran-3-carbonsäure; 6-Cyanindol-2-carbonsäure; 5-Cyanbenzo[b]thiophen-2-carbonsäure oder 6-Ethinylbenzo[b]thiophen-2-carbonsäure mit den verschiedenen Beispielen für Aminkomponenten gekuppelt wird, wie sie oben für die Typen C1 bis C8 ausführlich beschrieben sind. Die Synthese der jeweiligen Ausführungsbeispiele kann dabei analog der Vorschrift für die Herstellung von Beispiel 1 erfolgen. Sollen Ausführungsbeispiele wie z.B. die Verbindungen Beipiel 138, 140, 142, 144, 146, 148; 150, 152, 154 oder 156 ausgehend von 3-Chlorbenzo[b]thiophen-2-carbonsäurechlorid und den Aminen des Typs C1 bis C8 hergestellt werden, dann kann die Synthese der entsprechenden Ausführungsbeipiele analog der Vorschrift von Beispiel 136 erfolgen.

Konkrete mögliche Ausführungsbeispiele sind:

### Beispiel 29:

### N-(4-(4-(2,3-Dihydrobenzofuran-7-yl)piperazin-1-yl)butyl)-5-cyanbenzo[b]thiophen-2-ylcarbamid

### Beispiel 30:

### N-(4-(4-(2,3-Dihydrobenzofuran-7yl)piperazin-1-yl)butyl)benzofuran-2-ylcarbamid

### Beispiel 31:

### N-(4-(4-(2,3-Dihydrobenzofuran-7-yl)piperazin-1-yl)butyl)-5-brombenzofuran-2-ylcarbamid

### Beispiel 32:

### N-(4-(4-(2,3-Dihydrobenzofuran-7-yl)piperazin-1-yl)butyl)-6-cyanindol-2-ylcarbamid

### Beispiel 33:

### N-(4-(4-(Chroman-8-yl)piperazin-1-yl)butyl)-5-cyanbenzo[b]thiophen-2-ylcarbamid

### Beispiel 34:

### N-(4-(4-(Chroman-8-yl)piperazin- 1 -yl)butyl)benzofuran-2-ylcarbamid

### Beispiel 35:

### N-(4-(4-(Chroman-8-yl)piperazin-1-yl)butyl)-5-brombenzofuran-2-ylcarbamid

### Beispiel 36:

### N-(4-(4-(Chroman-8-yl)piperazin- 1 -yl)butyl)benzofuran-3-ylcarbamid

### Beispiel 37:

### N-(4-(4-(Chroman-8-yl)piperazin- 1-yl)butyl)-6-cyanindol-2-ylcarbamid

### Beispiel 38:

### N-(4-(4-(Chroman-8-yl)piperazin-1- yl)butyl)indol-3-ylcarbamid

### Beispiel 39:

### N-(4-(4-(2,3,4,5-Tetrahydrobenzo[b]oxepin-9-yl)piperazin-1-yl)butyl)benzo[b]thiophen-3-ylcarbamid

### Beispiel 40:

### N-(4-(4-(2,3,4,5-Tetrahydrobenzo[b]oxepin-9-yl)piperazin-1-yl)butyl)benzofuran-3-ylcarbamid

### Beispiel 41:

### N-(4-(4-(2,3,4,5-Tetrahydrobenzo[b]oxepin-9-yl)piperazin-1-yl)butyl)indol-3-ylcarbamid

### Beispiel 42:

### N-(4-(4-(2,3-Dihydrobenzofuran-7-yl)-1,4-diazepan-1-yl)butyl)-5-cyanbenzo[b]thiophen-2-ylcarbamid

### Beispiel 43:

### N-(4-(4-(2,3-Dihydrobenzofuran-7-yl)-1,4-diazepan-1 yl)butyl)benzo[b]thiophen-3-ylcarbamid

### Beispiel 44:

### N-(4-(4-(2,3-Dihydrobenzofuran-7-yl)-1,4-diazepan-1-yl)butyl)benzofuran-2-ylcarbamid

### Beispiel 45:

### N-(4-(4-(2,3-Dihydrobenzofuran-7-yl)- 1,4-diazepan-1-yl)butyl)-5-brombenzofuran-2-ylcarbamid

### Beispiel 46:

### N-(4-(4-(2,3-Dihydrobenzofuran-7-yl)-1,4-diazepan-1-yl)butyl)benzofuran-3-ylcarbamid

### Beispiel 47:

### N-(4-(4-(2,3-Dihydrobenzofuran-7-yl)- 1,4-diazepan-1-yl)buiyi)jndol-2-ylcarbamid

### Beispiel 48:

### N-(4-(4-(2,3-Dihydrobenzofuran-7-yl)-1,4-diazepan-1-yl)butyl)-6-cyanindol-2-ylcarbamid

### Beispiel 49:

### N-(4-(4-(2,3-Dihydrobenzofuran-7-yl)-1,4-diazepan-1 yl)butyl)indol-3-ylcarbamid

### Beispiel 50:

### N-(4-(4-(Chroman-8-yl)-1,4-diazepan-1-yl)butyl)-5-cyanbenzo[b]thiophen-2-ylcarbamid

### Beispiel 51:

### N-(4-(4-(Chroman-8-yl)piperazin-1-yl)butyl)benzo[b]thiophen-3-ylcarbamid

### Beispiel 52:

### N-(4-(4-(Chroman-8-yl)-1,4-diazepan-1-yl)butyl)-5-brombenzofuran-2-ylcarbamid

### Beispiel 53:

### N-(4-(4-(Chroman-8-yl)-1,4-diazepan-1-yl)butyl)benzofuran-3-ylcarbamid

### Beispiel 54:

### N-(4-(4-(Chroman-8-yl)-1,4-diazepan-1-yl)butyl)indol-2-ylcarbamid

### Beispiel 55:

### N-(4-(4-(Chroman-8-yl)-1,4-diazepan-1-yl)butyl)-6-cyanindol-2-ylcarbamid

### Beispiel 56:

### N-(4-(4-(Chroman-8-yl)-1,4-diazepan-1-yl)butyl)indol-3-ylcarbamid

### Beispiel 57:

### N-(4-(4-(2,3,4,5-Tetrahydrobenzo[b]oxepin-9-yl)-1,4-diazepan-1-yl)butyl)-5-cyanbenzo[b]thiophen-2-ylcarbamid

### Beispiel 58:

### N-(4-(4-(2,3,4,5-Tetrahydrobenzo[b]oxepin-9-yl)-7,4-diazepan-1-yl)butyl)benzo[b]thiophen-3-ylcarbamid

### Beispiel 59:

### N-(4-(4-(2,3,4,5-Tetrahydrobenzo[b]oxepin-9-yl)- 1,4-diazepan-1-yl)butyl)-5-brombenzofuran-2-ylcarbamid

### Beispiel 60:

### N-(4-(4-(2,3,4,5-Tetrahydrobenzo[b]oxepin-9-yl)- 1,4-diazepan- 1-yl)butyl)benzofuran-3-ylcarbamid

### Beispiel 61:

### N-(4-(4-(2,3,4,5-Tetrahydrobenzo[b]oxepin-9yl)-1,4-diazepan-1-yl)butyl)indol-2-ylcarbamid

### Beispiel 62:

### N-(4-(4-(2,3,4,5-Tetrahydrobenzo[b]oxepin-9-yl)-1,4-diazepan-1-yl)butyl)-6-cyanindol-2-ylcarbamid

### Beispiel 63:

### N-(4-(4-(2,3,4,5-Tetrahydrobenzo[b]oxepin-9- yl)-1,4-diazepan-1- yl)butyl)indol-3-ylcarbamid

### Beispiel 64:

### N-(4-(4-(Benzo[1,3]dioxol-4-yl)piperazin-1-yl)butyl)-5-cyanbenzo[b]thiophen-2-ylcarbamid

### Beispiel 65:

### N-(4-(4-(Benzo[1,3]dioxol-4-yl)piperazin-1-yl)butyl)benzo[b]thiophen-3-ylcarbamid

### Beispiel 66:

### N-(4-(4-(Benzo[1,3]dioxol-4-yl)piperazin-1-yl)butyl)benzofuran-2-ylcarbamid

### Beispiel 67:

### N-(4-(4-(Benzo[1,3]dioxol-4-yl)piperazin-1-yl)butyl)-5-brombenzofuran-2-ylcarbamid

### Beispiel 68:

### N-(4-(4-(Benzo[1,3]dioxol-4-yl)piperazin-1-yl)butyl)benzofuran-3-ylcarbamid

### Beispiel 69:

### N-(4-(4-(Benzo[1,3]dioxol-4-yl)piperazin-1-yl)butyl)indol-2-ylcarbamid

### Beispiel 70:

### N-(4-(4-(Benzo[1,3]dioxol-4-yl)piperazin-1-yl)butyl)-6-cyanindol-2-ylcarbamid

### Beispiel 71:

### N-(4-(4-(Benzo[1,3]dioxol-4-yl)piperazin-1-yl)butyl)indol-3-ylcarbamid

### Beispiel 72:

### N-(4-(4-(Chroman-7-yl)piperazin-1-yl)butyl)-5-cyanbenzo[b]thiophen-2-ylcarbamid

### Beispiel 73:

### N-(4-(4-(Chroman-7-yl)piperazin-1-yl)butyl)benzo[b]thiophen-3-ylcarbamid

### Beispiel 74:

### N-(4-(4-(Chroman-7-yl)piperazin-1-yl)butyl)benzofuran-2-ylcarbamid

### Beispiel 75:

### N-(4-(4-(Chroman-7yl)piperazin-1yl)butyl)-5-brombenzofuran-2-ylcarbamid

### Beispiel 76:

### N-(4-(4-(Chroman-7-yl)piperazin-1-yl)butyl)benzofuran-3-ylcarbamid

### Beispiel 77:

### N-(4-(4-(Chroman-7-yl)piperazin-1-yl)butyl)indol-2-ylcarbamid

### Beispiel 78:

### N-(4-(4-(Chroman-7-yl)piperazin-1-yl)butyl)-6-cyanindol-2-ylcarbamid

### Beispiel 79:

### N-(4-(4-(Chroman-7-yl)piperazin- 1-yl)butyl)indol-3-ylcarbamid

### Beispiel 80:

### N-(4-(4-(Benzojl,3Jdioxol-5-yl)piperazin-1-yl)butyl)-5-cyanbenzojbJthiophen-2-ylcarbamid

### Beispiel 81:

### N-(4-(4-(Benzo[1,3]dioxol-5-yl)piperazin-1-yl)butyl)benzo[b]thiophen-3-ylcarbamid

### Beispiel 82:

### N (4-(4-(Benzo[1,3]dioxol-5- yl)piperazin-1-yl)butyl)benzofuran-2-ylcarbamid

### Beispiel 83:

### N-(4-(4-(Benzo[1,3]dioxol-5-yl)piperazin-1-yl)butyl)-5-brombenzofuran-2-ylcarbamid

### Beispiel 84:

### N-(4-(4-(Benzo[1,3]dioxol-5-yl)piperazin-1-yl)butyl)benzofuran-3-ylcarbamid

### Beispiel 85:

### N-(4-(4-(Benzo[1,3]dioxol-5-yl)piperazin-1-yl)butyl)indol-2-ylcarbamid

### Beispiel 86:

### N-(4-(4-(Benzo[1,3]dioxot-5-yl)piperazin-1-yl)butyl)-6-cyanindol-2-ylcarbamid

### Beispiel 87:

### N-(4-(4-(Benzo[1,3]dioxol-5-yl)piperazin-7-yl)butyl)indol-3-ylcarbamid

### Beispiel 88:

### N-(4-(4-(2,3-Dihydrobenzo[1,4]dioxin-6- yl)piperazin-1- yl)butyl)-5-cyanbenzo[b]thiophen-2-ylcarbamid

### Beispiel 89:

### N-(4-(4-(2,3-Dihydrobenzo[1,4]dioxin-6-yl)piperazin-1-yl)butyl)benzo[b]thiophen-3-ylcarbamid

### Beispiel 90:

### N-(4-(4-(2,3-Dihydrobenzo[1,4]dioxin-6-yl)piperazin-1-yl)butyl)benzofuran-2-ylcarbamid

### Beispiel 91:

### N-(4-(4-(2,3-Dihydrobenzo[1,4]dioxin-6-yl)piperazin-1-yl)butyl)-5-brombenzofuran-2-ylcarbamid

### Beispiel 92:

### N-(4-(4-(2,3-Dihydrobenzo[1,4]dioxin-6-yl)piperazin-1-yl)butyl)benzofuran-3-ylcarbamid

### Beispiel 93:

### N-(4-(4-(2,3-Dihydrobenzo[1,4]dioxin-6-yl)piperazin-1-yl)butyl)indol-2-ylcarbamid

### Beispiel 94:

### N-(4-(4-(2,3-Dihydrobenzo[1,4]dioxin-6-yl)piperazin-1-yl)butyl)-6-cyanindol-2-ylcarbamid

### Beispiel 95:

### N-(4-(4-(2,3-Dihydrobenzo[1,4]dioxin-6-yl)piperazin-1-yl)butyl)indol-3-ylcarbamid

### Beispiel 96:

### N-(4-(4-(3,4-Dihydro-2H-benzo[b][1,4]dioxepin-7-yl)piperazin-1-yl)butyl)-5-cyanbenzo[b]fhiophen-2-ylcarbamid

### Beispiel 97:

### N-(4-(4-(3,4-Dihydro-2H-benzo[b][1,4]dioxepin-7 yl)piperazin-1-yl)butyl)benzo[b]thiophen-3-ylcarbamid

### Beispiel 98:

### N-(4-(4-(3,4-Dihydro-2H-benzo[b][1,4]dioxepin-7-yl)piperazin-1-yl)butyl)benzofuran-2-ylcarbamid

### Beispiel 99:

### N-(4-(4-(3,4-Dihydro-2H-benzo[b][1,4]dioxepin-7-yl)piperazin-1-yl)butyl)-5-brombenzofuran-2-ylcarbamid

### Beispiel 100:

### N-(4-(4-(3,4-Dihydro-2H-benzo[b][1,4]dioxepin-7-yl)piperazin-1-yl)butyl)benzofuran-3-ylcarbamid

### Beispiel 101:

### N-(4-(4-(3,4-Dihydro-2H-benzo[b][1,4]dioxepin-7-yl)piperazin-1-yl)butyl)indol-2-ylcarbamid

### Beispiel 102:

### N-(4-(4-(3,4-Dihydro-2H-benzo[b][1,4]dioxepin-7- yl)piperazin-1-yl)butyl)-6-cyanindol-2-ylcarbamid

### Beispiel 103:

### N-(4-(4-(3,4-Dihydro-2H-benzo[b][1,4]dioxepin-7-yl)piperazin-1-yl)butyl)indol-3-ylcarbamid

### Beispiel 104:

### N-(4-(4-(2,3-Dihydrobenzo[1,4]dioxin-6-yl)-1,4-diazepan-1-yl)butyl)-5-cyanbenzo[b]thiophen-2-ylcarbamid

### Beispiel 105:

### N-(4-(4-(2,3-Dihydrobenzo[1,4]dioxin-6 yl)-1,4-diazepan-1-yl)butyl)benzo[b]thiophen-3-ylcarbamid

### Beispiel 106:

### N-(4-(4-(2,3-Dihydrobenzo[1,4]dioxin-6-yl)-1,4-diazepan-1-yl)butyl)benzofuran-2-ylcarbamid

### Beispiel 107:

### N-(4-(4-(2,3-Dihydrobenzo[1,4]dioxin-6 yl)-1,4-diazepan-1-yl)butyl)-5-brombenzofuran 2-ylcarbamid

### Beispiel 108:

### N-(4-(4-(2,3-Dihydrobenzo[1,4]dioxin-6 yl)-1,4-diazepan-1-yl)butyl)benzofuran-3-ylcarbamid

### Beispiel 109:

### N-(4-(4-(2,3-Dihydrobenzo[1,4]dioxin-6-yl)-1,4-diazepan-1-yl)butyl)indol-2-ylcarbamid

### Beispiel 110:

### N-(4-(4-(2,3-Dihydrobenzo[1,4]dioxin-6-yl)-1,4-diazepan-1-yl)butyl)-6-cyanindol-2-ylcarbamid

### Beispiel 111:

### N-(4-(4-(2,3-Dihydrobenzo[1,4]dioxin-6-yl)-1,4-diazepan-1-yl)butyl)indol-3-ylcarbamid

### Beispiel 112:

### N-(4-(4-(2,3-Dihydrobenzofuran-5-yl)piperazin-1-yl)butyl)-5-cyanbenzo[b]thiophen-2-ylcarbamid

### Beispiel 113:

### N-(4-(4-(2,3-Dihydrobenzofuran-5-yl)piperazin-1-yl)butyl)benzo[b]thiophen-3-ylcarbamid

### Beispiel 114:

### N-(4-(4-(2,3-Dihydrobenzofuran-5-yl)piperazin- 1 -yl)butyl)benzofuran-2-ylcarbamid

### Beispiel 115:

### N-(4-(4-(2,3-Dihydrobenzofuran-5-yl)piperazin- 1 -yl)butyl)-5'brombenzofuran-2-ylcarbamid

### Beispiel 116:

### N-(4-(4-(2,3-Dihydrobenzofuran-5-yl)piperazin-1- yl)butyl)benzofuran-3-ylcarbamid

### Beispiel 117:

### N-(4-(4-(2,3-Dihydrobenzofuran-5 yl)piperazin-1-yl)butyl)indol-2-ylcarbamid

### Beispiel 118:

### N-(4-(4-(2,3-Dihydrobenzofuran-5-yl)piperazin-1-yl)butyl)-6-cyanindol-2-ylcarbamid

### Beispiel 119:

### N-(4-(4-(2,3-Dihydrobenzofuran-5-yl)piperazin-1yl)butyl)indol-3-ylcarbamid

### Beispiel 120:

### N-(4-(4-(Chroman-6-yl)piperazin-1-yl)butyl)-5-cyanbenzo[b]thiophen-2-ylcarbamid

### Beispiel 121:

### N-(4-(4-(Chroman-6-yl)piperazin-1-yl)butyl)benzo[b]thiophen-3-ylcarbamid

### Beispiel 122:

### N-(4-(4-(Chroman-6-yl)piperazin-1-yl)butyl)benzofuran-2-ylcarbamid

### Beispiel 123:

### N-(4-(4-(Chroman-6-yl)piperazin-1-yl)butyl)-5-brombenzofuran-2-ylcarbamid

### Beispiel 124:

### N-(4-(4-(Chroman-6-yl)piperazin-1-yl)butyl)benzofuran-3-ylcarbamid

### Beispiel 125:

### N-(4-(4-(Chroman-6-yl)piperazin-1-yl)butylindol-2-ylcarbamid

### Beispiel 126:

### N-(4-(4-(Chroman-6-yl)piperazin-1-yl)butyl)-6-cyanindol-2-ylcarbamid

### Beispiel 127:

### N-(4-(4-(Chroman-6-yl)piperazin- 1 -yl)butyl)indol-3-ylcarbamid

### Beispiel 128:

### N-(4-(4-(2,3,4,5-Tetrahydrobenzo[b]oxepin-7-yl)piperazin-1-yl)butyl)-5-cyanbenzo[b]thiophen-2-ylcarbamid

### Beispiel 129:

### N-(4-(4-(2,3,4,5-Tetrahydrobenzo[b]oxepin-7-yl)piperazin-1-yl)butyl)benzo[b]thiophen-3-ylcarbamid

### Beispiel 130:

### N-(4-(4-(2,3,4,5-Tetrahydrobenzo[b]oxepin-7-yl)piperazin-1-yl)butyl)benzofuran-2-ylcarbamid

### Beispiel 131:

### N-(4-(4-(2,3,4,5-Tetrahydrobenzo[b]oxepin-7-yl)piperazin-1-yl)butyl)-5-brombenzofuran-2-ylcarbamid

### Beispiel 132:

### N-(4-(4-(2,3,4,5-Tetrahydrobenzo[b]oxepin-7-yl)piperazin-1-yl)butyl)benzofuran-3-ylcarbamid

### Beispiel 133:

### N-(4-(4-(2,3,4,5-Tetrahydrobenzo[b]oxepin-7-yl)piperazin-1-yl)butyl)indol-2-ylcarbamid

### Beispiel 134:

### N-(4-(4-(2,3,4,5-Tetrahydrobenzo[b]oxepin-7- yl)piperazin-1-yl)butyl)-6-cyanindol-2-ylcarbamid

### Beispiel 135:

### N-(4-(4-(2,3,4,5-Tetrahydrobenzo[b]oxepin-7-yl)piperazin-1-yl)butyl)indol-3-ylcarbamid

### Beispiel 138:

### N-(4-(4-(2,3-Dihydrobenzofuran-7-yl)piperazin-1-yl)butyl)-3-chlorbenzo[b]thiophen-2-ylcarbamid

### Beispiel 139:

### N-(4-(4-(2,3-Dihydrobenzofuran-7-yl)piperazin-1-yl)butyl)-6-ethinylbenzo[b]thiophen-2-ylcarbamid

### Beispiel 140:

### N-(4-(4-(Chroman-8-yl)piperazin-1- yl)butyl)-3-chlorbenzo[b]thiophen-2-ylcarbamid

### Beispiel 141:

### N-(4-(4-(Chroman-8-yl)piperazin- 1-yl)butyl)-6-ethinylbenzo[b]thiophen-2-ylcarbamid

### Beispiel 142:

### N-(4-(4-(2,3-Dihydrobenzofuran-7-yl)-1,4-diazepan-1-yl)butyl)-3-chlorbenzo[b]thiophen-2-ylcarbamid

### Beispiel 143:

### N-(4-(4-(2,3-Dihydrobenzofuran-7-yl)-1,4-diazepan- 1-yl)butyl)-6-ethinylbenzo[b] thiophen-2-ylcarbamid

### Beispiel 144:

### N-(4-(4-(Chroman-8-yl)-1,4-diazepan-1-yl)butyl)-3-chlorbenzo[b]thiophen-2-ylcarbamid

### Beispiel 145:

### N-(4-(4-(Chroman-8-yl)-1,4-diazepan -1-yl)butyl)-6-ethinylbenzo[b]thiophen-2-ylcarbarnid

### Beispiel 146:

### N-(4-(4-(2,3,4,5-TetrahydrobenzojbJoxepin-9 yl)-1,4-diazepan -1 yl)butyl)-3-chlorbenzo[b]thiophen-2-ylcarbamid

### Beispiel 147:

### N-(4-(4-(2,3,4,5-Tetrahydrobenzo[b]oxepin-9-yl)-1,4-diazepan -1-yl)butyl)-6-ethinylbenzo[b]thiophen-2-ylearbarnid

### Beispiel 148:

### N-(4-(4-(Benzo[1,3]dioxol-4-yl)piperazin-1-yl)butyl)-3-chlorbenzo[b]thiophen-2-ylcarbamid

### Beispiel 149:

### N-(4-(4-(Benzo[1,3]dioxol-4-yl)piperazin-1-yl)butyl)-6-ethinylbenzo[b]thiophen-2-ylcarbamid

### Beispiel 150:

### N-(4-(4-(Chroman-7-yl)piperazin- 1-yl)butyl)-3-chlorbenzo[b]thiophen-2-ylcarbamid

### Beispiel 151:

### N-(4-(4-(Chroman-7-yl)piperazin- 1-yl)butyl)-6-ethinylbenzo[b]thiophen-2-ylcarbamid

### Beispiel 152: .

### N-(4-(4-(Benzo[1,3]dioxol-5-yl)piperazin-1-yl)butyl)-3-chlorbenzo[b]thiophen-2-ylcarbamid

### Beispiel 153:

### N-(4-(4-(Benzo[1,3]dioxol-5-yl)piperazin-1-yl)butyl)-6-ethinylbenzo[b]thiophen-2-ylcarbamid

### Beispiel 154:

### N-(4-(4-(2,3-Dihydrobenzo[1,4]dioxin-6 yl)piperazin-1- yl)butyl)-3-chlorbenzo[b]thiophen-2-ylcarbamid

### Beispiel 155:

### N-(4-(4-(2,3-Dihydrobenzo[1,4]dioxin-6-yl)piperazin-1-yl)butyl)-6-ethinylbenzo[b]thiophen-2-ylcarbamid

### Beispiel 156:

### N-(4-(4-(3,4-Dihydro-2H-benzo[b][1,4]dioxepin-7-yl)piperagin-1-yl)butyl)-3-chlorbenzo[b]thiophen-2-ylcarbamid

### Beispiel 157:

### N-(4-(4-(3,4-Dihydro-2H-benzo[b][1,4]dioxepin-7-yl)piperazin-1-yl)butyl)-6-ethinylbenzo[b]thiophen-2-ylcarbamid

### BIOLOGISCHE AKTIVITÄT

Die biologischen Aktivitäten der erfindungsgemäßen Verbindungen wurden in Radioligandbindungsuntersuchungen ermittelt. Alle Radioligandexperimente wurden nach von uns beschriebenen Methoden durchgeführt (Hübner, H. et al. J. Med. Chem. 2000; 43, 756-762). Für die Messung der Affinitäten zu den Rezeptoren der D2-Familie kamen Membranhomogenate von chinesischen Hamster-Ovarialzellen (CHO-Zellen) zum Einsatz, die jeweils den humanen D2long-, den humanen D2short- (Hayes, G. et al. Mol. Endocrinol. 1992, 6, 920-926), den humanen D3- (Sokotoff, P. et al. Eur. J. PharmacoL 1992, 225, 331-337) oder den humanen D4.4-Rezeptorsubtyp (Asghari, V. J. Neurochem. 1995, 65, 1157-1165) stabil exprimierten. Prinzipiell erfolgten die Bindungsassays durch Inkubation der Rezeptorhomogenate mit dem Radioligand [³H]Spiperon und der zu untersuchenden Verbindung in verschiedenen Konzentrationen. Die Ermittlung der Affinitäten zum D1-Rezeptor erfolgte mit nativen Membranhomogenaten, gewonnen aus dem Striatum des Schweines, und dem D1-selektiven Radioliganden [³H]SCH 23390.

Die Messung der Bindungsstärken der Verbindungen zu den Serotonin-Rezeptorsubtypen 5-HT1a und 5-HT2 wurden nach von uns beschriebenen Methoden (Heindl, C. et al. Tetrahedron: Asymmetry 2003, 14, 3141-3152) durchgeführt. Dazu inkubierten wir Cortex-Membranpräparationen des Schweines mit den Radioliganden [³H]8-OH-DPAT (für 5-HT1a) oder [³H]Ketanserin (5-HT2) und den Verbindungen in verschiedenen Konzentrationen. In gleicher Weise wurde die Affinität der Testverbindungen zum α1-Rezeptor des Schweins untersucht, wobei porcine Cortex-Membranpräparationen und der α1 -selektive Radioligand [³H]Prazosin eingesetzt wurden.

Alle im Dopaminrezeptor-Bindungsassay untersuchten Verbindungen zeigten gute bis sehr gute Affinitäten zu den Dopamin-Rezeptoren mit einer klaren Bindungspräferenz zu den D2- und D3-Subtypen. Dabei ist immer eine deutliche Selektivität zum D3-Rezeptor zu erkennen, der bei allen getesten Verbindungen 1-28 im pikomolaren oder unteren nanomolaren Bereich (< 20 nM) liegt. (Tabelle 1)

**Tabelle 1: Bindungsdaten zu den Ausführungsbeispielen nach Formel I und 11 für die Dopamin-Rezeptoren porcinD1, humanD2long, humanD2short, humanD3 und humanD4.4 sowie den porcinen Serotonin-Rezeptoren 5-HTIa und 5-HT2 und dem Adreno-Rezeptor alpha1^{a}**

| | Rezeptorbindung (Ki Werte in [nM]) | | | | | | | |
|---|---|---|---|---|---|---|---|---|
| Nr. | D1 | D2long | D2short | D3 | D4 | 5-HT1 | 5-HT2 | alpha1 |
| **1** | 260 | 73 | 50 | 0,31 | 65 | 18 | 100 | 4,2 |
| **2** | 300 | 45 | 27 | 0,16 | 22 | 30 | 110 | 2 |
| **3** | 300 | 110 | 83 | 0,35 | 50 | 17 | 77 | 4,6 |
| **4** | 290 | 42 | 22 | 0,17 | 34 | 20 | 110 | 2,3 |

| | | | | | | | | |
|---|---|---|---|---|---|---|---|---|
| ^{a} ermittelt als Durchschnittswerte aus 2-6 Einzelexperimenten jeweils durchgeführt als Triplikate | | | | | | | | |

Ein Vergleich zu den aus dem Stand der Technik (Wo 2004/004729) bekannten Verbindungen zeigt die überlegene D3-Bindung der erfindungsgemäßen Verbindungen (Tabelle 1a - 1c):

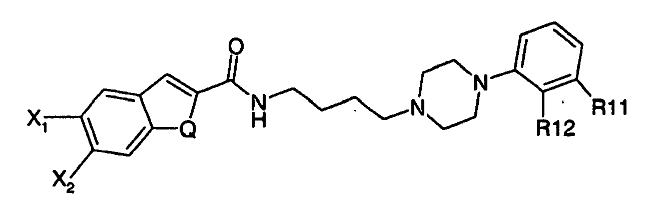

**Table 1a: Q=NH; X1=H**

| **X2** | Vergleichssubstanz | D3-Bindung (Ki in [nM]) | Erfindungsgemäße Substanz; R11+R12 bilden Ringsystem | D3-Bindung (Ki in [nM]) |
|---|---|---|---|---|
| **CN** | R11, R12=Chlor | 0.35 | Tetrahydrobenzoxepin | 0.075 |
| **CN** | R11=H; R12= OCH3 | 0.25 | | |
| **H** | R11, R12=Chlor | 0.56 | Dihydrobenzofuran | 0.35 |
| **H** | | | Chroman | 0.17 |
| **H** | | | Tetrahydrobenzoxepin | 0.052 |

**Table 1b: Q=O; X2=H**

| **X1** | Vergleichssubstanz | D3-Bindung (Ki in [nM]) | Erfindungsgemäße Substanz; R11+R12 bilden Ringsystem | D3-Bindung (Ki in [nM]) |
|---|---|---|---|---|
| **Br** | R11, R12=Chlor | 3.4 | Tetrahydrobenzoxepin | 0.11 |
| **Br** | R11=H; R12= OCH3 | 0.69 | | |
| **H** | R11, R12=Chlor | 1.2 -1.5 | Tetrahydrobenzoxepin | 0.089 . |
| **H** | R11=H; R12= OCH3 | 1,1 | | |

**Table 1 c: Q=S; X2=H**

| **X1** | Vergleichssubstanz | D3-Bindung (Ki in [nM]) | Erfindungsgemäße Substanz; R11+R12 bilden Ringsystem | D3-Bindung (Ki in [nM]) |
|---|---|---|---|---|
| **CN** | R11, R12=Chlor | 0.25 | Tetrahydrobenzoxepin | 0.097 |
| **CN** | R11=H; R12= OCH3 | 0.46 | | |
| **H** | R11, R12=Chlor | 0.50 | Dihydrobenzofuran | 0.31 |
| **H** | R11=H;R12=OCH3 | 0.23 | Chroman | 0.16 |
| **H** | | | Tetrahydrobenzoxepin | 0.098 |

Untersuchungen zur Bestimmung der intrinsischen Aktivität der Beispielverbindungen wurden in einem Mitogenese-Assay in Anlehnung an die Literatur (Hübner, H. et al. J. Med. Chem. 2000, 43, 4563-4569; Bettinetti, L. et al. J. Med. Chem. 2002, 45, 4594-4597) durchgeführt. Dabei wurden verschieden Konzentrationen der zu untersuchenden Verbindung mit D3-Rezeptor exprimierenden Zellen inkubiert und anschließend die rezeptorvermittelte Stimulation der Mitogeneserate durch Einbau des radioaktiven Markers [³H]Thymidin gemessen. Agonistische, partialagonistische oder antagonistische Effekte wurden im Vergleich zur Wirkung des vollen Agonisten Quinpirol ermittelt. (Tabelle 2)

**Tabelle 2: Ergebnisse der Mitogenese-Experimente mit den Ausführungsbeispielen am Dopamin-D3-Rezeptor zur Ermittlung der intrinsischen Aktivität^{a}**

| Verbindungen | EC₅₀-Wert [nM]^{b} | agonistische Aktivität [%]^{c} |
|---|---|---|
| **Beispiel 1** | 3,2 | 42 |
| **Beispiel 2** | -- | 0 |
| **Beispiel 3** | 1,8 | 38 |
| **Beispiel 4** | -- | 0 |
| **Quinpirol** | 3,2 | 100 |

| | | |
|---|---|---|
| ^{a} dosisabhängiger Einbau des Radiornarkers [³H]Thymidin als Maß für die Stimulation der Mitogeneserate gemessen bei sieben verschiedenen Konzentrationen in Quadruplikaten ^{b} EC₅₀-Wert der Dosis-Wirkungskurve abgeleitet von den Durchschnittswerten aller Einzelversuche ^{c} agonistische Aktivität in [%] bezogen, auf den maximalen Effekt des vollen Agonisten Quinpirol | | |

## Patentansprüche

1. Verbindungen der allgemeinen Formel 1, 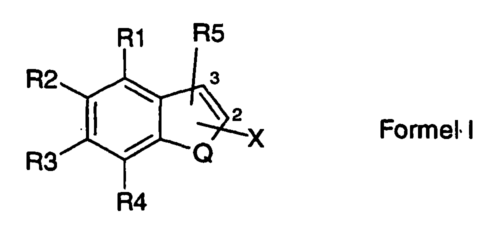 in der bedeuten:
Q ist ausgewählt aus S, O und NR;
R ist ausgewählt aus Wasserstoff, Alkyl, Phenyl, Alkylcarbonyl, Phenylalkylcarbonyl, Phenylcarbonyl, Phenylalkyl und Phenylsulfonyl;
R1, R2, R3 und R4 sind jeweils unabhängig voneinander ausgewählt aus der Gruppe Wasserstoff, Hydroxy, Alkyl, Alkyloxy, Alkylthio, Alkenyl, Alkinyl, Phenyl, Phenylalkyl, Phenoxy, Halogen, Trifluormethyl, Alkylcarbonyl, Phenylcarbonyl, Phenylalkylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Alkylsulfonylamino;
R5 ist eine an Position 2 oder 3 des bizyklischen Heteroaryls gebundene Gruppe, die ausgewählt ist aus Wasserstoff, Alkyl, Halogen, Alkoxy und Amino;
X ist eine an Position 2 oder 3 des bizyklischen Heteroaryls gebundene Gruppe der allgemeinen Formel X1 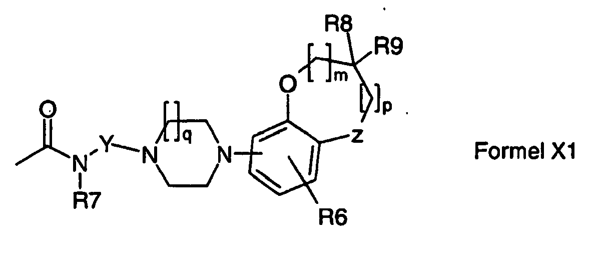
in der gilt:
R6 ist ausgewählt aus der Gruppe Wasserstoff, Hydroxy, Alkyl, Alkyloxy, Alkytthio, Alkenyl, Alkinyl, Phenyl, Phenylalkyl, Phenoxy, Halogen, Trifluormethyl, Alkylcarbonyl, Phenylcarbonyl, Phenylalkylcarbonyl, Alkyloxycarbonyl, Phenylalkyloxycarbonyl, Cyano, Nitro, Amino, Carboxy, Sulfo, Sulfamoyl, Sulfonylamino, Alkylaminosulfonyl und Alkylsulfonylamino;
R7 ist Wasserstoff, Alkyl oder Phenylalkyl;
Y ist eine unverzweigte, gesättigte oder ungesättigte Kohlenwasserstoffkette mit 2-5 Kohlenstoffatomen;
m und p sind jeweils unabhängig voneinander 0, 1, oder 2, wobei die Summe aus m und p höchstens 2 ist;
q ist 1 oder 2;
Z ist CH₂, NH oder O;
R8 und R9 sind jeweils unabhängig voneinander ausgewählt aus Wasserstoff, Alkyl und Phenyl oder bilden gemeinsam eine Oxo-Gruppe;
in Form der freien Base, deren physiologisch akzeptable Salze sowie möglicher Enantiomere und Diastereomere.

2. Verbindungen nach Anspruch 1, wobei X die allgemeine Formel X2 aufweist 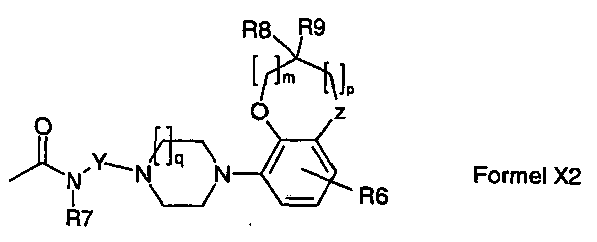 in der R6, R7, R8, R9, m, p, q, Y und Z die Bedeutung haben, wie in Anspruch 1 definiert.

3. Verbindung nach einem der vorhergehenden Ansprüche, wobei R8 und R9 beide jeweils ein Wasserstoffatom repräsentieren.

4. Verbindung nach einem der vorhergehenden Ansprüche, wobei R6 und R7 jeweils Wasserstoffe repräsentieren.

5. Verbindungen nach einem der vorhergehenden Ansprüche, wobei Z eine CH₂-Gruppe oder ein O darstellt.

6. Verbindungen nach einem der Ansprüche 1-4, wobei Z NH repräsentiert.

7. Verbindung nach einem der vorhergehenden Ansprüche, wobei die Summe aus m und p 1 oder 2 ist.

8. Verbindung nach einem der vorhergehenden Ansprüche, wobei R1, R4, R5, R6 und R7 jeweils ein Wasserstoffatom repräsentieren, Y eine gesättigte, unverzweigte Kohlenstoffkette mit 4 oder 5 Kohlenstoffen repräsentiert.

9. Verbindung nach einem der vorhergehenden Ansprüche, wobei q den Wert 1 hat.

10. Verbindung nach Anspruch 1 oder 2, wobei gilt:
- Q ist ausgewählt aus S, O oder NH;
- R1 und R4 sind H;
- R5 ist H oder Halogen;
- R2 und R3 sind unabhängig ausgewählt aus Wasserstoff, Hydroxy; Fluor; Chlor; Brom; Trifluormethyl; Cyano; Amino; Carboxy; Sulfo; Sulfamoyl; unsubstituiertes oder mit Hydroxy substituiertes C1-C6 Alkyl; unsubstituiertes oder mit Hydroxy substituiertes C1-C6 Alkyloxy; unsubstituiertes oder mit Hydroxy substituiertes C1-C6 Alkylthio; unsubstituiertes C2-C6 Alkinyl; unsubstituiertes oder mit Fluor, Chlor oder Brom und/oder mit einer oder mehreren Methoxygruppen substituiertes Phenyl; Phenyl(C1-C6)Alkyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; unsubstituiertes oder mit Fluor, Chlor oder Brom und/oder mit einem oder mehreren Methoxygruppen substituiertes Phenoxy; -C(O)-(C1-C6)Alkyl, wobei das Alkyl unsubstituiert oder mit Hydroxy substituiert ist; -C(O)-Phenyl, wobei das Phenyl unsubstituiert oder mit Fluor, Chlor oder Brom und/oder mit einer oder mehreren Methoxygruppen substituiert ist; -C(O)- (C1-C6)Alkyl-Phenyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; C1-C6 Alkyloxycarbonyl, wobei das Alkyl unsubstituiert oder mit Hydroxy substituiert ist; Phenyl(C1-C6)Alkyloxycarbonyl, wobei das Phenyl unsubstituiert oder mit Fluor, Chlor oder Brom und/oder mit einer oder mehreren Methoxygruppen substituiert,ist und wobei das C1-C6 Alkyl unsubstituiert oder mit Hydroxy substituiert ist; C1-C6 Alkylaminosulfonyl, insbesondere Methylaminosulfonyl und C1-C6 Alkylsulfonylamino; insbesondere Methansulfonylamino;
- X ist eine Gruppe der Formel X1 oder X2, für die gilt:
o R6 repräsentiert Wasserstoff, C1-C6 Alkyl, C1-C6 Alkoxy oder Halogen;
o R7 repräsentiert Wasserstoff oder C1 -C6 Alkyl;
o R8 und R9 sind Wasserstoff;
o Z ist CH2 oder O
o Summe aus m und p = 0, 1 oder 2 und besonders bevorzugt 1 oder 2;
o q ist 1 oder 2;
o Y ist eine unverzweigte gesättigte Kohlenwasserstoffkette mit 3,4 oder 5 C-Atomen.

11. Verbindung nach Anspruch 1 mit der allgemeinen Formel II 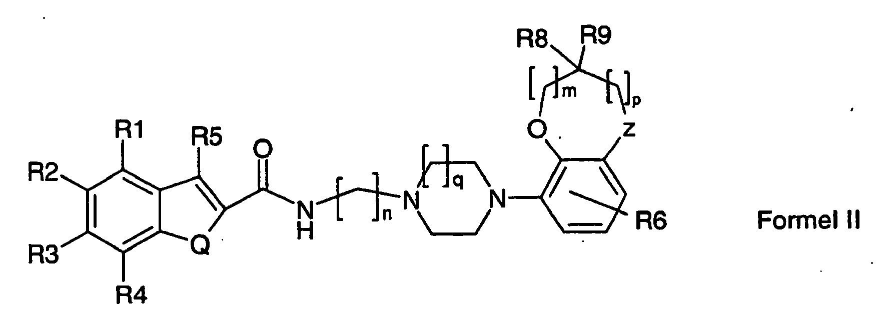 worin R, R1, R2, R3, R4, R5, R6, R8, R9, Q, Z, m, p und q die Bedeutung haben, wie in einem der vorhergehenden Ansprüche definiert und worin n den Wert 3, 4 oder 5 hat.

12. Verbindung nach Anspruch 1, mit der allgemeinen Formel III, IV, V, VI oder VII 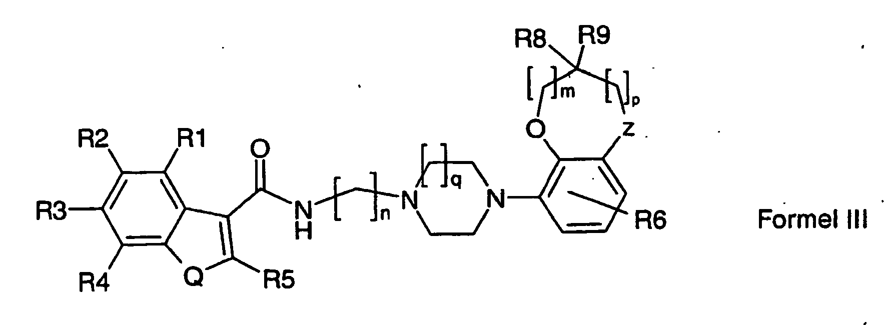 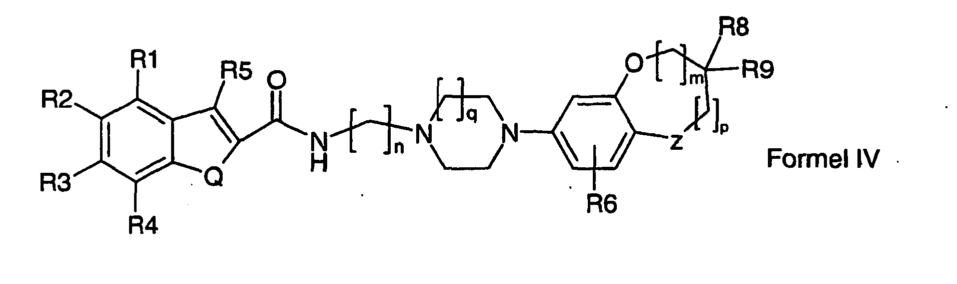 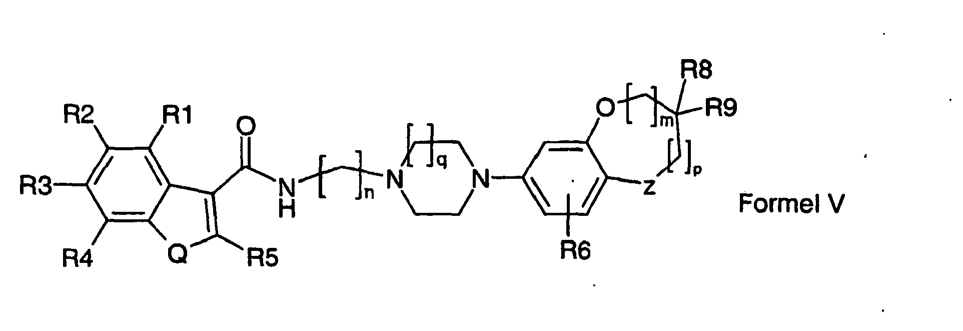 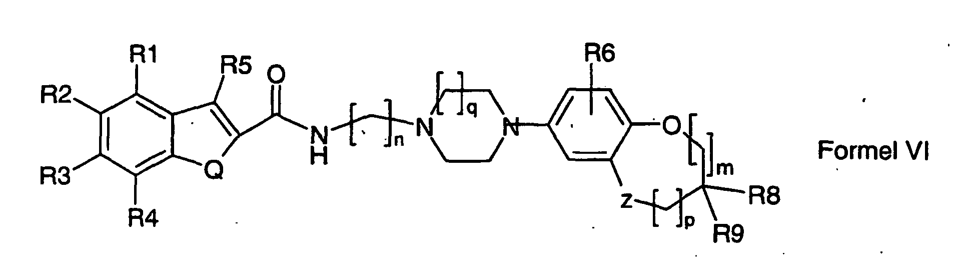 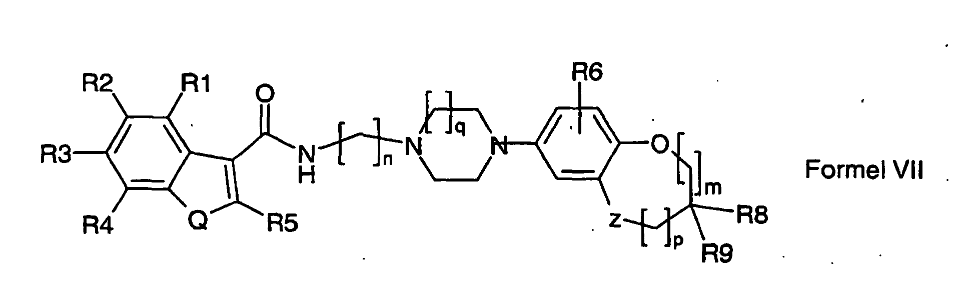 worin R, R1, R2, R3, R4, R5, R6, R8, R9, Q, Z, m, p und q jeweils die Bedeutung haben, wie in Anspruch 1 definiert und worin n den Wert 3, 4 oder 5 hat.

13. Verbindungen nach einem der Ansprüche 11 oder 12, wobei Z eine CH₂-Gruppe oder ein O darstellt.

14. Verbindung nach einem der Ansprüche 11-13, wobei die Summe aus m und p 1 oder 2 ist

15. Verbindung nach einem der Ansprüche 11-14, wobei R1, R4 und R6 jeweils ein Wasserstoffatom repräsentieren.

16. Verbindung nach einem der Ansprüche 11-15, wobei R8 und R9 beide je ein Wasserstoffatom repräsentieren.

17. Verbindung nach einem der Ansprüche 11-16, wobei q den Wert 1 hat.

18. Verbindung nach einem der Ansprüche 11 oder 12, wobei gilt:
- R1, R4, R6, R8 und R9 sind jeweils ein Wasserstoffatom;
- R2 und R3 sind jeweils unabhängig ausgewählt aus Wasserstoff, Hydroxy; Fluor; Chlor; Brom; Trifluormethyl; Cyano; Amino; Carboxy; Sulfo; Sulfamoyl; unsubstituiertes oder mit Hydroxy substituiertes C1-C6 Alkyl; unsubstituiertes oder mit Hydroxy substituiertes C1-C6 Alkyloxy; unsubstituiertes oder mit Hydroxy substituiertes C1-C6 Alkylthio; unsubstituiertes C2-C6 Alkinyl; unsubstituiertes oder mit Fluor, Chlor oder Brom und/oder mit einer oder mehreren Methoxygruppen substituiertes . Phenyl; Phenyl(C1-C6)Alkyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; unsubstituiertes oder mit Fluor, Chlor oder Brom und/oder, mit einem oder mehreren Methoxygruppen substituiertes Phenoxy; -C(O)-(C1-C6)Alkyl, wobei das Alkyl unsubstituiert oder mit Hydroxy substituiert ist; -C(O)-Phenyl, wobei das Phenyl unsubstituiert oder mit Fluor, Chlor oder Brom und/oder mit einer oder mehreren Methoxygruppen substituiert ist; - C(O)- (C 1 -C6)Alkyl-Phenyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; C1-C6 Alkyloxycarbonyl, wobei das Alkyl unsubstituiert oder mit Hydroxy substituiert ist; Phenyl(C1-C6)Alkyloxycarbonyl, wobei das Phenyl unsubstituiert oder mit Fluor, Chlor oder Brom und/oder mit einer oder mehreren Methoxygruppen substituiert ist und wobei das C1-C6 Alkyl unsubstituiert oder mit Hydroxy substituiert ist; C1-C6 Alkylaminosulfonyl, insbesondere Methylaminosulfonyl und C1-C6 Alkylsulfonylamino; insbesondere Methansulfonylamino;
- R5 ist Wasserstoff oder Halogen;
- n ist 4 oder 5
- q ist 1 oder 2;
- Z ist CH₂ oder Sauerstoff.

19. Verbindung nach Anspruch 11, ausgewählt aus
N-(4-(4-(2,3-Dihydrobenzofuran-7-yl)piperazin-1-yl)butyl)benzo[b]thiophen-2-ylcarbamid
N-(4-(4-(2,3-Dihydrobenzofuran-7-yl)piperazin-1-yl)butyl)-3-chlorbenzo[b]thiophen-2-ylcarbamid
N-(4-(4-(2,3-Dihydrobenzofuran-7-yl)piperazin-1-yl)butyl)-5-cyanbenzo[b]thiophen-2-ylcarbamid
N-(4-(4-(2,3-Dihydrobenzofuran-7-yl)piperazin-1-yl)butyl)-6-ethinylbenzo[b]thiophen-2-ylcarbamid
N-(4-(4-(2,3-Dihyd robenzofuran-7-yl)piperazin-1-yl)butyl)benzofuran-2-ylcarbamid
N-(4-(4-(2,3-Dihydrobenzofuran-7-yl)piperazin-1-yl)butyl)-5-brombenzofuran-2-ylcarbamid
N-(4-(4-(2,3-Dihydrobenzofuran-7-yl)piperazin-1-yl)butyl)indol-2-ylcarbamid
N-(4-(4-(2,3-Dihydrobenzofuran-7-yl)piperazin-1 -yl)butyl)-6-cyanindol-2-ylcarbamid
N-(4-(4-(2,3-Dihydrobenzofuran-7-yl)-1,4-diazepan-1-yl)butyl)benzo[b]thiophen-2-ylcarbamid
N-(4-(4-(2,3-Dihydrobenzofuran-7-yl)-1,4-diazepan-1-yl)butyl)-3-chlorbenzo[b]thiophen-2-ylcarbamid
N-(4-(4-(2,3-Dihydrobenzofuran-7-yl)-1,4-diazepan-1-yl)butyl)-5-cyanbenzo[b]thiophen-2-ylcarbamid
N-(4-(4-(2,3-Dihydrobenzofuran-7-yl)-1,4-diazepan-1-yl)butyl)-6-ethinylbenzo[b]thiophen-2-ylcarbamid
N-(4-(4-(2,3-Dihydrobenzofuran-7-yl)-1,4-diazepan-1-yl)butyl)benzofuran-2-ylcarbamid
N-(4-(4-(2,3-Dihydrobenzofuran-7-yl)-1,4-diazepan-1-yl)butyl)-5-brombenzofuran-2-ylcarbamid
N-(4-(4-(2,3-Dihydrobenzofuran-7-yl)-1,4-diazepan-1-yl)butyl)indol-2-ylcarbamid
N-(4-(4-(2,3-Dihydrobenzofuran-7-yl)-1,4-diazepan-1-yl)butyl)-6-cyanindol-2-ylcarbamid
N-(4-(4-(Chroman-8-yl)piperazin-1-yl)butyl)benzo[b]thiophen-2-ylcarbamid
N-(4-(4-(Chroman-8-yl)piperazin-1-yl)butyl)-3-chlorbenzo[b]thiophen-2-ylcarbamid
N-(4-(4-(Chroman-8-yl)piperazin-1-yl)butyl)-5-cyanbenzo[b]thiophen-2-ylcarbamid
N-(4-(4-(Chroman-8-yl)piperazin-1-yl)butyl)-6-ethinylbenzo[b]thiophen-2-ylcarbamid
N-(4-(4-(Chroman-8-yl)piperazin-1-yl)butyl)benzofuran-2-ylcarbamid
N-(4-(4-(Chroman-8-yl)piperazin-1-yl)butyl)-5-brombenzofuran-2-ylcarbamid
N-(4-(4-(Chroman-8-yl)piperazin-1-yl)butyl)indol-2-ylcarbamid
N-(4-(4-(Chroman-8-y1)piperazin-1-yl)butyl)-6-cyanindol-2-ylcarbamid
N-(4-(4-(Chroman-8-yl)-1,4-diazepan-1-yl)butyl)benzo[b]thiophen-2-ylcarbamid
N-(4-(4-(Chroman-8-yl)-1,4-diazepan -1-yl)butyl)-3-chlorbenzo[b]thiophen-2-ylcarbamid
N-(4-(4-(Chroman-8-yl)-1,4-diazepan-1-yl)butyl)-5-cyanbenzo[b]thiophen-2-ylcarbamid
N-(4-(4-(Chroman-8-yl)-1,4-diazepan -1-yl)butyl)-6-ethinylbenzo[b]thiophen-2-ylcarbamid
N-(4-(4-(Chroman-8-yl)-1,4-diazepan-1-yl)butyl)benzofuran-2-ylcarbamid
N-(4-(4-(Chroman-8-yl)-1,4-diazepan-1-yl)butyl)-5-brombenzofuran-2-ylcarbamid
N-(4-(4-(Chroman-8-yt)-1,4-diazepan-l -y))butyl)indot-2-ylcarbamid
N-(4-(4-(Chroman-8-yl)-1,4-diazepan-1-yl)butyl)-6-cyanindol-2-ylcarbamid
N-(4-(4-(2,3,4,5-Tetrahydrobenzo[b]oxepin-9-yl)piperazin-1-yl)butyl)benzo[b]thiophen-2-ylcarbamid
N-(4-(4-(2,3,4,5-Tetrahydrobenzo[b]oxepin-9-yl)piperazin-1-yl)butyl)-3-chlorbenzo[b]thiophen-2-ylcarbamid
N-(4-(4-(2,3,4,5-Tetrahydrobenzo[b]oxepin-9-yl)piperazin-1-yl)butyl)-5-cyanbenzo[b]thiophen-2-ylcarbamid
N-(4-(4-(2,3,4,5-Tetrahydrobenzo[b]oxepin-9-yl)piperazin-1-yl)butyl)-6-ethinylbenzo[b]thiophen-2-ylcarbamid
N-(4-(4-(2,3,4,5-Tetrahydrobenzo[b]oxepin-9-yl)piperaz7in-1 -yl)butyl)benzofuran-2-ylcarbamid
N-(4-(4-(2,3,4,5-Tetrahydrobenzo[b]oxepin-9-yl)piperazin-1-yl)butyl)-5-brombenzofuran-2-ylcarbamid
N-(4-(4-(2,3,4,5-Tetrahydrobenzo[b]oxepin-9-yl)piperazin-1-yl)butyl)indol-2-ylcarbamid
N-(4-(4-(2,3,4,5-Tetrahydrobenzo[b]oxepin-9-yl)piperazin-1-yl)butyl)-6-cyanindol-2-ylcarbamid
N-(4-(4-(2,3,4,5-Tetrahydrobenzo[b]oxepin-9-yl)-1,4-diazepan-1-yl)butyl)benzo[b]thiophen-2-ylcarbamid
N-(4-(4-(2,3,4,5-Tetrahydrobenzo[b]oxepin-9-yl)-1,4-diazepan-1-yl)butyl)-3-chlorbenzo[b]thiophen-2-ylcarbamid
N-(4-(4-(2,3,4,5-Tetrahydrobenzo[b]oxepin-9-y))-1,4-diazepan-1-yl)butyl)-5-cyanbenzo[b]thiophen-2-ylcarbamid
N-(4-(4-(2,3,4,5-Tetrahydrobenzo[b]oxepin-9-yl)-1,4-diazepan -1-yl)butyl)-6-ethinylbenzo[b]thiophen-2-ylcarbamid
N-(4-(4-(2,3,4,5-Tetrahyd robenzo[b]oxepin-9-yi)-1,4-diazepan-1-yl)butyl)benzofuran-2-ylcarbamid
N-(4-(4-(2,3,4,5-Tetrahydrobenzo[b]oxepin-9-yl)-1,4-diazepan-1-yl)butyl)-5-brombenzofuran-2-ylcarbamid
N-(4-(4-(2,3,4,5-Tetrahydrobenzo[b]oxepin-9-yl)-1,4-diazepan-1-yl)butyl)indol-2-ylcarbamid
N-(4-(4-(2,3,4,5-Tetrahydrobenzo[b]oxepin-9-yl)-1,4-diazepan-1-yl)butyl)-6-cyanindol-2-ylcarbamid
N-(4-(4-(Benzo[1,3]dioxo)-4-yl)piperazin-1-yl)butyl)benzo[b]thiophen-2-ylcarbamid
N-(4-(4-(Benzo[1,3]dioxol-4-yl)piperazin-1-yl)butyl)-3-chlorbenzo[b]thiophen-2-ylcarbamid
N-(4-(4-(Benzo[1,3]dioxol-4-yl)piperazin-1-yl)butyl)-5-cyanbenzo[b]thiophen-2-ylcarbamid
N-(4-(4-(Benzo[1,3]dioxol-4-yl)piperazin-1-yl)butyl)-6-ethinylbenzo[b]thiophen-2-ylcarbamid
N-(4-(4-(Benzo[1,3]dioxol-4-yl)piperazin-1 -yl)butyl)benzofuran-2-ylcarbamid
N-(4-(4-(Benzo[1,3]dioxot-4-yl)piperazin-1 -yl)butyl)-5-brombenzofuran-2-ylcarbamid
N-(4-(4-(Benzo[1,3]dioxol-4-yl)piperazin-1-yl)butyl)indol-2-ylcarbamid
N-(4-(4-(Benzo[1,3]dioxol-4-yl)piperazin-1-yl)butyl)-6-cyanindol-2-ylcarbamid
N-(4-(4-(6-Chlor-3-oxo-3,4-dihydro-2H-benzo[1,4]oxazin-8-yl)piperazin-1-yl)butyl)benzo[b]thiophen-2-ylcarbamid

20. Verbindung nach Anspruch 12, ausgewählt aus
(a) einer Verbindung der Formel III aus der Gruppe
N-(4-(4-(2,3-Dihydrobenzofuran-7-yl)piperazin-1-yl)butyl)benzo[b]thiophen-3-ylcarbamid
N-(4-(4-(2,3-Dihydrobenzofuran-7-yl)piperazin-1-yl)butyl)benzofuran-3-ylcarbamid
N-(4-(4-(2,3-Dihydrobenzofuran-7-yl)piperazin-1-yl)butyl)indol-3-ylcarbamid
N-(4-(4-(2,3-Dihydrobenzofuran-7-yl)-1,4-diazepan-1-yl)butyl)benzo[b]thiophen-3-ylcarbamid
N-(4-(4-(2,3-Dihydrobenzofuran-7-yl)-1,4-diazepan-1-yl)butyl)benzofuran-3-ylcarbamid
N-(4-(4-(2,3-Dihydrobenzofuran-7-yl)-1,4-diazepan-1-yl)butyl)indol-3-ylcarbamid
N-(4-(4-(Chroman-8-yl)piperazin-1-yl)butyl)benzo[b]thiophen-3-ylcarbamid
N-(4-(4-(Chroman-8-yl)piperazin-1-yl)butyl)benzofuran-3-ylcarbamid
N-(4-(4-(Chroman-8-yl)piperazin-1-yl)butyl)indol-3-ylcarbamid
N-(4-(4-(Chroman-8-yl)-1,4-diazepan-1-yl)butyl)benzo[b]thiophen-3-ylcarbamid
N-(4-(4-(Chroman-8-yl)-1,4-diazepan-1 -yl)butyl)benzofuran-3-ylcarbamid
N-(4-(4-(Chroman-8-yl)-1,4-diazepan-1-yl)butyl)indol-3-ylcarbamid
N-(4-(4-(2,3,4,5-Tetrahydrobenzo[b]oxepin-9-yl)piperazin-1-yl)butyl)benzo[b]thiophen-3-ylcarbamid
N-(4-(4-(2,3,4,5-Tetrahydrobenzo[b]oxepin-9-yl)piperazin-1-yl)butyl)benzofuran-3-ylcarbamid
N-(4-(4-(2,3,4,5-Tetrahydrobenzo[b]oxepin-9-yl)piperazin-1-yl)butyl)indol-3-ylcarbamid
N-(4-(4-(2,3,4,5-Tetrahydrobenzo[b]oxepin-9-yl)-1,4-diazepan-1-yl)butyl)benzo[b]thiophen-3-ylcarbamid
N-(4-(4-(2,3,4,5-Tetrahydrobenzo[b]oxepin-9-yl)-1,4-diazepan-1-yl)butyl)benzofuran-3-ylcarbamid
N-(4-(4-(2,3,4,5-Tetrahydrobenzo[b]oxepin-9-yl)-1,4-diazepan-1-yl)butyl)indol-3-ylcarbamid
N-(4-(4-(Benzo[1,3]dioxol-4-yl)piperazin-1-yl)butyl)benzo[b]thiophen-3-ylcarbamid
N-(4-(4-(Benzo[1,3]dioxol-4-yl)piperazin-1-yl)butyl)benzofuran-3-ylcarbamid
N-(4-(4-(Benzo[1,3]dioxol-4-yl)piperazin-1 -yl)butyl)indol-3-ylcarbamid
(b) einer Verbindung der Formel IV aus der Gruppe :
N-(4-(4-(Chroman-7-yl)piperazin-1-yl)butyl)benzo[b]thiophen-2-ylcarbamid
N-(4-(4-(Chroman-7-yl)piperazin-1-yl)butyl)-3-chlorbenzo[b]thiophen-2-ylcarbamid
N-(4-(4-(Chroman-7-yl)piperazin-1-yl)butyl)-5-cyanbenzo[b]thiophen-2-ylcarbamid
N-(4-(4-(Chroman-7-yl)piperazin-1-yl)butyl)-6-ethinylbenzo[b]thiophen-2-ylcarbamid
N-(4-(4-(Chroman-7-yl)piperazin-1-yl)butyl)benzofuran-2-ylcarbamid
N-(4-(4-(Chroman-7-yl)piperazin-1 -yl)butyl)-5-brombenzofuran-2-ylcarbamid
N-(4-(4-(Chroman-7-yl)piperazin-1-yl)butyl)indol-2-ylcarbamid
N-(4-(4-(Chroman-7-yl)piperazin-1-yl)butyl)-6-cyanindol-2-ylcarbamid
N-(4-(4-(Benzo[1,3]dioxol-5-yl)piperazin-1-yl)butyl)benzo[b]thiophen-2-ylcarbamid
N-(4-(4-(Benzo[1,3]dioxol-5-yl)piperazin-1-yl)butyl)-3-chlorbenzo[b]thiophen-2-ylcarbamid
N-(4-(4-(Benzo[1,3]dioxol-5-yl)piperazin-1 -yl)butyl)-5-cyanbenzolbphiophen-2-ylcarbamid
N-(4-(4-(Benzo[1,3]dioxol-5-yl)piperazin-1-yl)butyl)-6-ethinylbenzo[b]thiophen-2-ylcarbamid
N-(4-(4-(Benzo[1,3]dioxol-5-yl)piperazin-1 -yl)butyl)benzof uran-2-ylcarbamid
N-(4-(4-(Benzo[1,3]dioxol-5-yl)piperazin-1-yl)butyl)-5-brombenzofuran-2-ylcarbamid
N-(4-(4-(Benzo[1,3]dioxol-5-yl)piperazin-1 -yl)butyl)indol-2-ylcarbamid
N-(4-(4-(Benzo[1,3]dioxol-5-yl)piperazin-1-yl)butyl)-6-cyanindol-2-ylcarbamid '
N-(4-(4-(2,3-Dihydrobenzo[1,4]dioxin-6-yl)piperazin-1-yl)butyl)benzo[b]thiophen-2-ylcarbamid
N-(4-(4-(2,3-Dihydrobenzo[1,4]dioxin-6-yl)piperazin-1-yl)butyl)-3-chlorbenzo[b]thiophen-2-ylcarbamid
N-(4-(4-(2,3-Dihydrobenzo[1,4]dioxin-6-yl)piperazin-1-yl)butyl)-5-cyanbenzo[b]thiophen-2-ylcarbamid
N-(4-(4-(2,3-Dihydrobenzo[1,4]dioxin-6-yl)piperazin-1-yl)butyl)-6-ethinylbenzo[b]thiophen-2-ylcarbamid
N-(4-(4-(2,3-Dihydrobenzo[1,4]dioxin-6-yl)piperazin-1-yl)butyl)benzofuran-2-ylcarbamid
N-(4-(4-(2,3-Dihydrobenzo[1;4]dioxin-6-yl)piperazin-1-yl)butyl)-5-brombenzofuran-2-ylcarbamid
N-(4-(4-(2,3-Dihydrobenzo[1,4]dioxin-6-yl)piperazin-1-yl)butyl)indol-2-ylcarbamid
N-(4-(4-(2,3-Dihydrobenzo[1,4]dioxin-6-yl)piperazin-1 -yl)butyl)-6-cyanindol-2-ylcarbamid
N-(4-(4-(2,3-Dihydrobenzo[1,4]dioxin-6-yl)-1,4-diazepan-1-yl)butyl)benzo[b]thiophen-2-ylcarbamid
N-(4-(4-(2,3-Dihydrobenzo[1,4]dioxin-6-yl)-1,4-diazepan-1-yl)butyl)-5-cyanbenzo[b]thiophen-2-ylcarbamid
N-(4-(4-(2,3-Dihydrobenzo[1,4]dioxin-6-yl)-1,4-diazepan-1-yl)butyl)benzofuran-2-ylcarbamid
N-(4-(4-(2,3-Dihydrobenzo[1,4]dioxin-6-y))-1,4-diazepan-1-yl)butyl)-5-brombenzofuran-2-ylcarbamid
N-(4-(4-(2,3-Dihydrobenzo[1,4]dioxin-6-yl)-1,4-diazepan-1-yl)butyl)indol-2-ylcarbamid
N-(4-(4-(2,3-Dihydrobenzo[1,4]dioxin-6-yl)-1,4-diazepan-1-yl)butyl)-6-cyanindol-2-ylcarbamid
N-(4-(4.(3,4-Dihydro-2H-benzo[b][1,4]dioxepin-7-yl)piperazin-1-yl)butyl)benzo[b]thiophen-2-ylcarbamid
N-(4-(4-(3,4-Dihydro-2H-benzo[b][1,4]dioxepin-7-yl)piperazin-1-yl)butyl)-3-chlorbenzo[b]thiophen-2-ylcarbamid
N-(4-(4-(3,4=Dihydro-2H-benzo[b][1,4]dioxepin-7=yl)piperäzin-1-yl)butyl)-5-cyanbenzo[b]thiophen-2-ylcarbamid
N-(4-(4-(3,4-Dihydro-2H-benzo[b][1,4]dioxepin-7-yl)piperazin-1-yl)butyl)-6-ethinylbenzo[b]thiophen-2-ylcarbamid
N-(4-(4-(3,4-Dihydro-2H-benzo[b][1,4]dioxepin-7-yl)piperazin-1-yl)butyl)benzofuran-2-ylcarbamid
N-(4-(4-(3,4-Dihydro-2H-benzo[b][1 ,4]dioxepin-7 -yl)piperazin-1-yl)butyl)-5-brombenzofuran-2-ylcarbamid
N-(4-(4-(3,4-Dihydro-2H-benzo[b][1,4]dioxepin-7-yl)piperazin-1-yl)butyl)indol-2-ylcarbamid
N-(4-(4-(3,4-Dihydro-2H-benzo[b][1,4]dioxepin-7-yl)piperazin-1-yl)butyl)-6-cyanindol-2-ylcarbamid
(c) einer Verbindung der Formel V aus der Gruppe:
N-(4-(4-(Chroman-7-yl)piperazin-1-yl)butyl)benzo[b]thiophen-3-ylcarbamid
N-(4-(4-(Chroman-7-yl)piperazin-1-yl)butyl)benzofuran-3-ylcarbamid
N-(4-(4-(Chroman-7-yl)piperazin-1-yl)butyl)indol-3-ylcarbamid
N-(4-(4-(Benzo[1,3]dioxol-5-yl)piperazin-1-yl)butyl)benzo[b]thiophen-3-ylcarbamid
N-(4-(4-(Benzo[1,3]dioxol-5-yl)piperazin-1-yl)butyl)benzofuran-3-ylcarbamid
N-(4-(4-(Benzo[1,3]dioxol-5-yl)piperazin-1-yl)butyl)indol-3-ylcarbamid
N-(4-(4-(2,3-Dihyd robenzo[1,4]dioxin-6-yl)piperazin-1-yl)butyl)benzo[b]thiophen-3-ylcarbamid
N-(4-(4-(2,3-Dihydrobenzo[1,4]dioxin-6-yl)piperazin-1-yl)butyl)benzofuran-3-ylcarbamid
N-(4-(4-(2,3-Dihydrobenzo[1,4]dioxin-6-yl)piperazin-1-yl)butyl)indol-3-ylcarbamid
N-(4-(4-(2,3-Dihyd robenzo[1,4]dioxin-6-yl)-1,4-diazepan-1-yl)butyl)benzo[b]thiophen-3-ylcarbamid
N-(4-(4-(2,3-Dihydrobenzo[1,4]dioxin-6-y))-1,4-diazepan-l-yl)butyl)benzofuran-3-ylcarbamid
N-(4-(4-(2,3-Dihydrobenzo[1 ,4]dioxin-6-yl)-1 ,4-diazepan-1-yl)butyl)indol-3-ylcarbamid
N-(4-(4-(3,4-Dihydro-2H-benzo[b][1,4]dioxepin-7-yl)piperazin-1-yl)butyl)benzo[b]thiophen-3-ylcarbamid
N-(4-(4-(3,4-Dihydro-2H-benzo[b][1,4]dioxepin-7-yl)piperazin-1-yl)butyl)benzofuran-3-ylcarbamid
N-(4-(4-(3,4-Dihydro-2H-benzo[b][1,4]dioxepin-7-yl)piperazin-1-yl)butyl)indol-3-ylcarbamid
(d) einer Verbindung der Formel VI aus der Gruppe
N-(4-(4-(2,3-Dihydrobenzofuran-5-yl)piperazin-1-yl)butyl)benzo[b]thiophen-2-ylcarbamid
N-(4-(4-(2,3-Dihydrobenzofuran-5-yl)piperazin-1-yl)butyl)-5-cyanbenzo[b]thiophen-2-ylcarbamid
N-(4-(4-(2,3-Dihydrobenzofuran-5-yl)piperazin-1-yl)butyl)benzofuran-2-ylcarbamid
N-(4-(4-(2,3-Dihydrobenzofuran-5-yl)piperazin-1 -yl)butyl)-5-brombenzofuran-2-ylcarbamid
N-(4-(4-(2,3-Dihydrobenzofuran-5-yl)piperazin-1-yl)butyl)indol-2-ylcarbamid
N-(4-(4-(2,3-Dihydrobenzofuran-5-yl)piperazin-1-yl)butyl)-6-cyanindol-2-ylcarbamid
N-(4-(4-(Chroman-6-yl)piperazin-1-yl)butyl)benzo[b]thiophen-2-ylcarbamid
N-(4-(4-(Chroman-6-yl)piperazin-1 -yl)butyl)-5-cyanbenzo[b]thiophen-2-ylcarbamid
N-(4-(4-(Chroman-6-yl)piperazin-1-yl)butyl)benzofuran-2-ylcarbamid
N-(4-(4-(Chroman-6-yl)piperazin-1-yl)butyl)-5-brombenzofuran-2-ylcarbamid
N-(4-(4-(Chroman-6-yl)piperazin-1-yl)butylindol-2-ylcarbamid
N-(4-(4-(Chroman-6-yl)piperazin-1-yl)butyl)-6-cyanindol-2-ylcarbamid
N-(4-(4-(2,3,4,5-Tetrahydrobenzo[b]oxepin-7-yl)piperazin-1-yl)butyl)benzo[b]thiophen-2-ylcarbamid
N-(4-(4-(2,3,4,5-Tetrahydrobenzo[b]oxepin-7-yl)piperazin-1 -yl)butyl)-5-cyanbenzo[b]thiophen-2-ylcarbamid
N-(4-(4-(2,3,4,5-Tetrahydrobenzo[b]oxepin-7-yl)piperazin-1-yl)butyl)benzofuran-2-ylcarbamid
N-(4-(4-(2,3,4,5-Tetrahydrobenzo[b]oxepin-7-yl)piperazin-1-yl)butyl)-5-brombenzofuran-2-ylcarbamid
N-(4-(4-(2,3,4,5-Tetrahydrobenzo[b]oxepin-7-yl)piperazin-1-yl)butyl)indol-2-ylcarbamid
N-(4-(4-(2,3,4,5-Tetrahydrobenzo[b]oxepin-7-yl)piperazin-1-yl)butyl)-6-cyanindol-2-ylcarbamid
(e) einer Verbindung der Formel VII aus der Gruppe:
N-(4-(4-(2,3-Dihydrobenzofuran-5-yl)piperazin-1-yl)butyl)benzo[b]thiophen-3-ylcarbamid
N-(4-(4-(2,3-Dihydrobenzofuran-5-yl)piperazin-1-yl)butyl)benzofuran-3-ylcarbamid
N-(4-(4-(2,3-Dihydrobenzofuran-5-yl)piperazin-1-yl)butyl)indol-3-ylcarbamid
N-(4-(4-(Chroman-6-yl)piperazin-1-yl)butyl)benzo[b]thiophen-3-ylcarbamid
N-(4-(4-(Chroman-6-yl)piperazin-1-yl)butyl)benzofuran-3-ylcarbamid
N-(4-(4-(Chroman-6-yl)piperazin-1-yl)butyl)indol-3-ylcarbamid
N-(4-(4-(2,3,4,5-Tetrahydrobenzo[b]oxepin-7-yl)piperazin-1-yl)butyl)benzo[b]thiophen-3-ylcarbamid
N-(4-(4-(2,3,4,5-Tetrahydrobenzo[b]oxepin-7-yl)piperazin-1-yl)butyl)benzofuran-3-ylcarbamid
N-(4-(4-(2,3,4,5-Tetrahydrobenzo[b]oxepin-7-yl)piperazin-1-yl)butyl)indol-3-ylcarbamid

21. Verbindungen gemäß einem der vorhergehenden Ansprüche als Arzneimittel.

22. Pharmazeutische Zusammensetzung umfassend eine oder mehrere der Verbindungen nach einem der Ansprüche 1-20 und mindestens ein pharmazeutisch akzeptables Hilfsmittel.

23. Verwendung einer Verbindung nach einem der Ansprüche 1-20 zur Herstellung eines Arzneimittels zur Behandlung von ZNS-Erkrankungen.

24. Verwendung einer Verbindung nach einem der Ansprüche 1-20 zur Herstellung eines Arzneimittels zur Behandlung von Störungen des Urinaltrakts..

25. Verwendung einer Verbindung nach einem der Ansprüche 1-20 zur Herstellung eines Arzneimittels zur Behandlung von Erkrankungen aus der Gruppe Psychosen, Schizophrenie, Angststörungen, Zwangsstörungen, Drogenabhängigkeit, depressive Störungen, Arzneimittel-induzierte extrapyramidalmotorische Bewegungsstörungen, Morbus Parkinson, Segawa-Syndrom, Tourette-Syndrom, Restless-Leg Syndrom, Schlafstörungen, Übelkeit, kognitive Störungen, männliche erektile Dysfunktion, Hyperprolaktinämie, Hyperprolaktinom, Glaukoma, Hyperaktivitätssyndrom (ADHS), Autismus, Schlaganfall und urinale Inkontinenz,

26. Verwendung nach einem der Ansprüche 1-20, wobei die Verbindung zur Herstellung eines Arzneimittel zur Behandlung von Schizophrenie, depressiven Störungen, L-Dopa- oder Neuroleptika-induzierten Bewegungsstörungen, Morbus Parkinson, Segawa-Syndrom, Restless-Leg-Syndrom, Hyperprolaktinämie, Hyperprolaktinom, Hyperaktivitätssyndrom (ADHS) oder urinale Inkontinenz verwendet werden.

27. Verwendung einer Verbindung nach einem der Ansprüche 1-20 zur Herstellung eines Arzneimittels zur Behandlung von Schmerzen

28. Verfahren zur Herstellung einer Verbindung nach einem der Anssprüche 1-20 umfassend die Umsetzung eines Säurederivats A 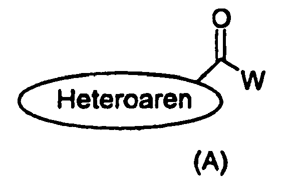 mit einer freien Base der generellen Formel C 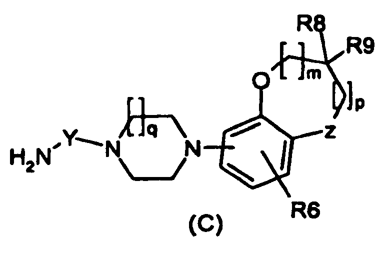 wobei gilt:
W ist ausgewählt aus OH, Cl, Br oder einer Gruppe 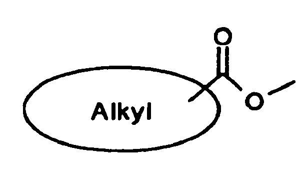 "Heteroarene" steht im Säurederivat A für eine Gruppe der generellen Formel la 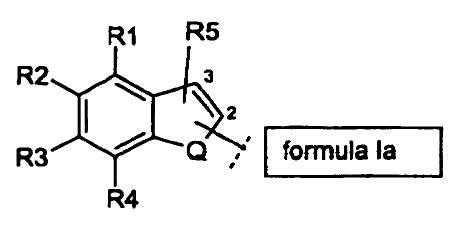
R1, R2, R3, R4, R5, R6, R8. R9, Q, Z, m, p und q haben die Bedeutung, wie in den Ansprüchen 1-20 definiert; wobei die durchgestrichene Bindung in Formula Ia für die Bindung der Gruppe -C(O)-W an die 2- oder 3-position des besagten Heteroarenes der Formel la steht.

29. Methode entsprechend Anspruch 28, wobei W Chlor, Brom oder OH repräsentiert.

30. Methode entsprechend Anspruch 28, wobei W eine Hydroxygruppe ist, und wobei die Säuregruppe vor der Umsetzung mit der freien Base der generellen Formel C durch ein Aktivierungsreagenz aktiviert wird.

31. Methode entsprechend Anspruch 30, wobei das Aktivierungsreagenz ausgewählt ist aus der Gruppe Hydroxybenzotriazol, Hydroxyazabenzotriazol, HATU und TBTU.

## Claims

1. Compounds of general formula I, 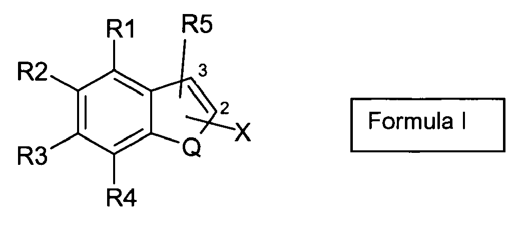 in which:
Q is selected from S, O and NR;
R is selected from among hydrogen, alkyl, phenyl, alkylcarbonyl, phenylalkylcarbonyl, phenylcarbonyl, phenylalkyl and phenylsulfonyl;
R1, R2, R3 and R4 are independently selected from the group comprising hydrogen, hydroxy, alkyl, alkyloxy, alkylthio, alkenyl, alkinyl, phenyl, phenylalkyl, phenoxy, halogen, trifluoromethyl, alkylcarbonyl, phenylcarbonyl, phenylalkylcarbonyl, alkyloxycarbonyl, phenylalkyloxycarbonyl, cyano, nitro, amino, carboxy, sulfo, sulfamoyl, sulfonylamino, alkylaminosulfonyl and alkylsulfonylamino;
R5 is a group bonded to position 2 or 3 of the bicyclic heteroaryl, selected from among hydrogen, alkyl, halogen, alkoxy and amino;
X is a group of general formula X1 bonded at position 2 or 3 of the bicyclic heteroaryl 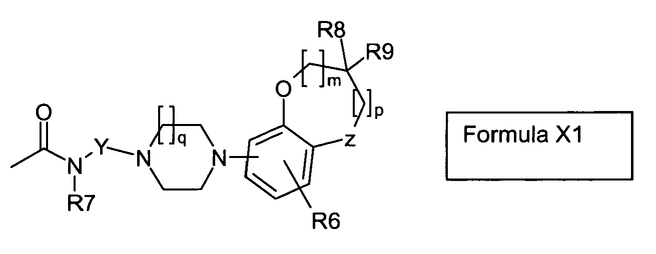
in which:
R6 is selected from the group comprising hydrogen, hydroxy, alkyl, alkyloxy, alkylthio, alkenyl, alkinyl, phenyl, phenylalkyl, phenoxy, halogen, trifluoromethyl, alkylcarbonyl, phenylcarbonyl, phenylalkylcarbonyl, alkyloxycarbonyl, phenylalkyloxycarbonyl, cyano, nitro, amino, carboxy, sulfo, sulfamoyl, sulfonylamino, alkylaminosulfonyl and alkylsulfonylamino;
R7 is hydrogen, alkyl or phenylalkyl;
Y is an unbranched, saturated or unsaturated hydrocarbon chain with 2-5 carbon atoms;
m and p are independently 0, 1, or 2, wherein the sum of m and p is a maximum of 2;
q is 1 or 2;
Z is CH₂, NH or O;
R8 and R9 are independently selected from among hydrogen, alkyl and phenyl or together form an oxo-group;
in the form of the free base, their physiologically acceptable salts and possible enantiomers and diastereomers.

2. Compounds according to claim 1, wherein X is of general formula X2 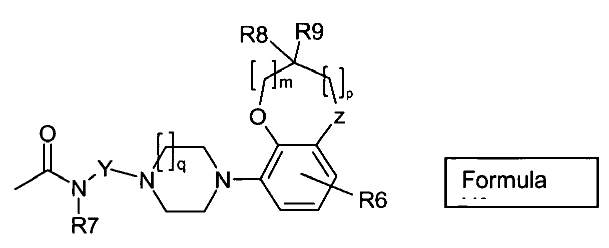 in which R6, R7, R8, R9, m, p, q, Y and Z are as defined in claim 1.

3. Compound according to either of the preceding claims, wherein R8 and R9 both represent a hydrogen atom.

4. Compound according to any one of the preceding claims, wherein R6 and R7 in each case represent a hydrogen atom.

5. Compounds according to any one of the preceding claims, wherein Z represents a CH₂- group or an O.

6. Compounds according to any one of claims 1-4, wherein Z represents NH.

7. Compound according to any one of the preceding claims, wherein the sum of m and p is 1 or 2.

8. Compound according to any one of the preceding claims, wherein R1, R4, R5, R6 and R7 in each case represents a hydrogen atom, and Y a saturated, unbranched carbon chain with 4 or 5 carbons.

9. Compound according to any one of the preceding claims, wherein q has the value 1.

10. Compound according to claim 1 or 2, wherein:
- Q is selected from S, O or NH;
- R1 and R4 are H;
- R5 is H or halogen;
- R2 and R3 are independently selected from among hydrogen; hydroxy; fluorine; chlorine; bromine; trifluoromethyl; cyano; amino; carboxy; sulfo; sulfamoyl; unsubstituted or hydroxy substituted C1-C6 alkyl; unsubstituted or hydroxy substituted C1-C6 alkyloxy; unsubstituted or hydroxy substituted C1-C6 alkylthio; unsubstituted C2-C6 alkinyl; phenyl which is unsubstituted or which is substituted with fluorine, chlorine or bromine and/or with one or more methoxy groups; phenyl(C1-C6)alkyl, wherein the phenyl is unsubstituted or substituted with fluorine, chlorine or bromine and/or with one or more methoxy groups and wherein the C1-C6 alkyl is unsubstituted or hydroxy substituted; unsubstituted phenoxy or phenoxy substituted with fluorine, chlorine or bromine and/or with one or more methoxy groups; -C(O)-(C1-C6)alkyl, wherein the alkyl is unsubstituted or hydroxy substituted; -C(O)-phenyl, wherein the phenyl is unsubstituted or substituted with fluorine, chlorine or bromine and/or with one or more methoxy groups; -C(O)-(C1-C6)alkyl-phenyl, wherein the phenyl is unsubstituted or substituted with fluorine, chlorine or bromine and/or with one or more methoxy groups and wherein the C1-C6 alkyl is unsubstituted or hydroxy substituted; C1-C6 alkyloxycarbonyl, wherein the alkyl is unsubstituted or hydroxy substituted; phenyl(C1-C6)alkyloxycarbonyl, wherein the phenyl is unsubstituted or substituted with fluorine, chlorine or bromine and/or with one or more methoxy groups and wherein the C1-C6 alkyl is unsubstituted or hydroxy substituted; C1-C6 alkylaminosulfonyl, in particular methylaminosulfonyl and C1-C6 alkylsulfonylamino; in particular methansulfonylamino;
- X is a group of formula X1 or X2, wherein
o R6 represents hydrogen, C1-C6 alkyl, C1-C6 alkoxy or halogen;
o R7 represents hydrogen or C1-C6 alkyl;
o R8 and R9 are hydrogen;
o Z is CH₂ or O;
o The sum of m and p = 0, 1 or 2 and particularly preferably 1 or 2;
o q is 1 or 2;
o Y is an unbranched, saturated hydrocarbon chain with 3, 4 or 5 C-atoms.

11. Compound according to claim 1 of general formula II 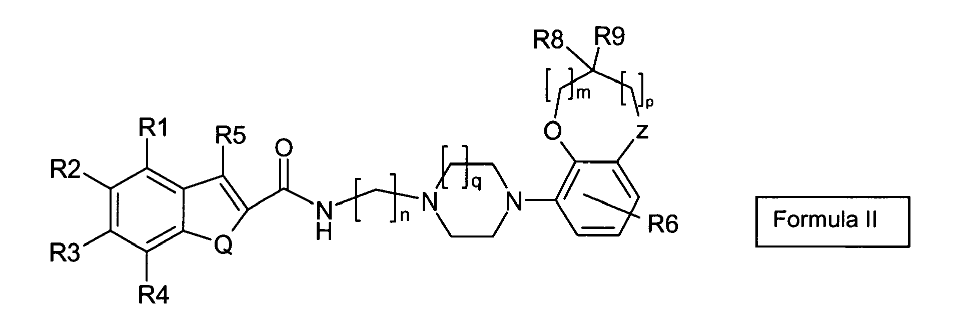 in which R, R1, R2, R3, R4, R5, R6, R8, R9, Q, Z, m, p and q are as defined in any one of the preceding claims and in which n has the value 3, 4 or 5.

12. Compound according to claim 1, of general formula III, IV, V, VI or VII 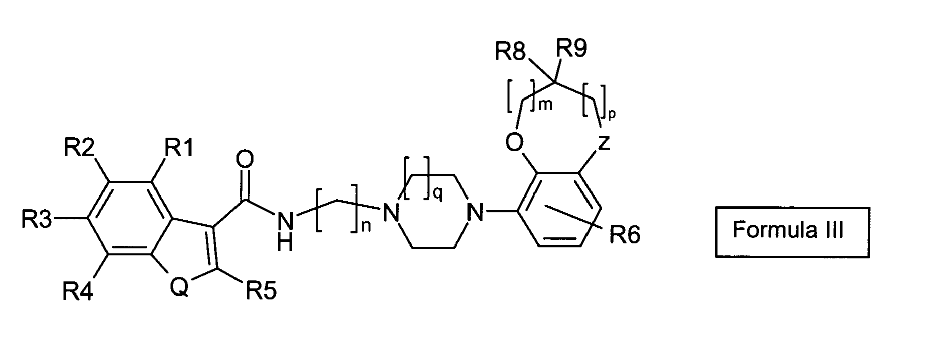 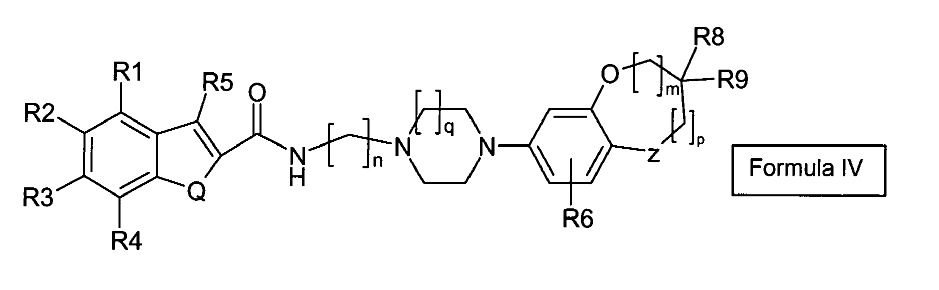 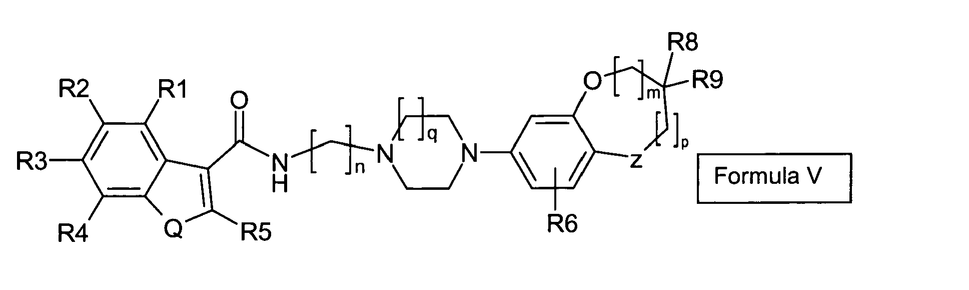 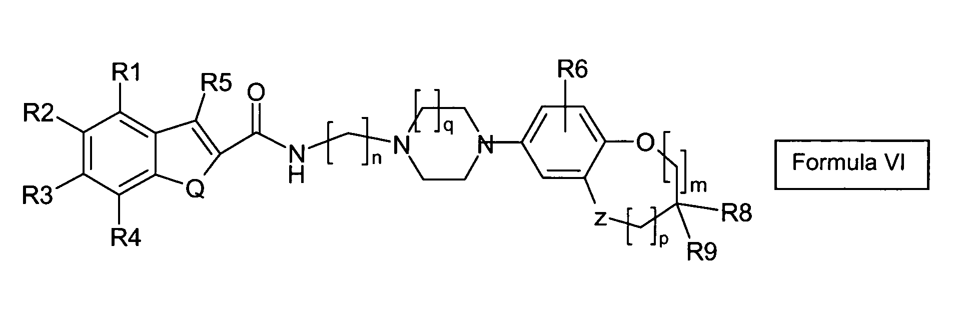 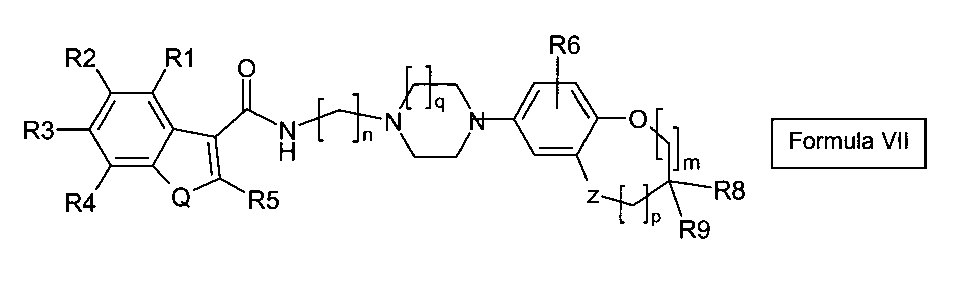 in which R, R1, R2, R3, R4, R5, R6, R8, R9, Q, Z, m, p and q in each case are as defined in claim 1 and in which n has the value 3, 4 or 5.

13. Compounds according to either of claims 11 or 12, wherein Z represents a CH₂-group or an O.

14. Compound according to any one of claims 11-13, wherein the sum of m and p is 1 or 2.

15. Compound according to any one of claims 11-14, wherein R1, R4 and R6 in each case represent a hydrogen atom.

16. Compound according to any one of claims 11-15, wherein R8 and R9 both each represent a hydrogen atom.

17. Compound according to any one of claims 11-16, wherein q has the value 1.

18. Compound according to either of claims 11 or 12, wherein:
- R1, R4, R6, R8 and R9 are in each case a hydrogen atom;
- R2 and R3 are in each case independently selected from the group comprising hydrogen; hydroxy; fluorine; chlorine; bromine; trifluoromethyl; cyano; amino; carboxy; sulfo; sulfamoyl; unsubstituted or hydroxy substituted C1-C6 alkyl; unsubstituted or hydroxy substituted C1-C6 alkyloxy; unsubstituted or hydroxy substituted C1-C6 alkylthio; unsubstituted C2-C6 alkinyl; unsubstituted phenyl or phenyl substituted with fluorine, chlorine or bromine and/or with one or more methoxy groups; phenyl(C1-C6)alkyl, wherein the phenyl is unsubstituted or substituted with fluorine, chlorine or bromine and/or with one or more methoxy groups, and wherein the C1-C6 alkyl is unsubstituted or hydroxy substituted; unsubstituted phenoxy or phenoxy substituted with fluorine, chlorine or bromine and/or with one or more methoxy groups; -C(O)-(C1-C6)alkyl, wherein the alkyl is unsubstituted or hydroxy substituted; -C(O)-phenyl, wherein the phenyl is unsubstituted or substituted with fluorine, chlorine or bromine and/or with one or more methoxy groups; -C(O)-(C1-C6)alkyl-phenyl, wherein the phenyl is unsubstituted or substituted with fluorine, chlorine or bromine and/or with one or more methoxy groups, and wherein the C1-C6 alkyl is unsubstituted or hydroxy substituted; C1-C6 alkyloxycarbonyl, wherein the alkyl is unsubstituted or hydroxy substituted; phenyl(C1-C6)alkyloxycarbonyl, wherein the phenyl is unsubstituted or substituted with fluorine, chlorine or bromine and/or with one or more methoxy groups, and wherein the C1-C6 alkyl is unsubstituted or hydroxy substituted; C1-C6 alkylaminosulfonyl, in particular methylaminosulfonyl and C1-C6 alkylsulfonylamino; in particular methansulfonylamino;
- R5 is hydrogen or halogen;
- n is 4 or 5;
- q is 1 or 2;
- Z is CH₂ or oxygen.

19. Compound according to claim 11, selected from among
N-(4-(4-(2,3-dihydrobenzofuran-7-yl)piperazine-1-yl)butyl)benzo[b]thiophene-2-ylcarbamide
N-(4-(4-(2,3-dihydrobenzofuran-7-yl)piperazine-1-yl)butyl)-3-chlorobenzo[b]thiophene-2-ylcarbamide
N-(4-(4-(2,3-dihydrobenzofuran-7-yl)piperazine-1-yl)butyl)-5-cyanobenzo[b]thiophene-2-ylcarbamide
N-(4-(4-(2,3-dihydrobenzofuran-7-yl)piperazine-1-yl)butyl)-6-ethinylbenzo[b]thiophene-2-ylcarbamide
N-(4-(4-(2,3-dihydrobenzofuran-7-yl)piperazine-1-yl)butyl)benzofuran-2-ylcarbamide
N-(4-(4-(2, 3-dihydrobenzofuran-7-yl)piperazine-1-yl)butyl)-5-bromobenzofuran-2-ylcarbamide
N-(4-(4-(2,3-dihydrobenzofuran-7-yl)piperazine-1-yl)butyl)indol-2-ylcarbamide
N-(4-(4-(2, 3-dihydrobenzofuran-7-yl)piperazine-1-yl)butyl)-6-cyanindol-2-ylcarbam ide
N-(4-(4-(2, 3-dihydrobenzofuran-7-yl)-1,4-diazepane-1-yl)butyl)benzo[b]thiophene-2-ylcarbamide
N-(4-(4-(2,3-dihydrobenzofuran-7-yl)-1,4-diazepane-1-yl)butyl)-3-chlorobenzo[b]thiophene-2-ylcarbamide
N-(4-(4-(2,3-dihydrobenzofuran-7-yl)-1,4-diazepane-1-yl)butyl)-5-cyanobenzo[b]thiophene-2-ylcarbamide
N-(4-(4-(2,3-dihydrobenzofuran-7-yl)-1,4-diazepane-1-yl)butyl)-6-ethinylbenzo[b]thiophene-2-ylcarbamide
N-(4-(4-(2,3-dihydrobenzofuran-7-yl)-1,4-diazepane-1-yl)butyl)benzofuran-2-ylcarbamide
N-(4-(4-(2,3-dihydrobenzofuran-7-yl)-1,4-diazepane-1-yl)butyl)-5-bromobenzofuran-2-ylcarbamide
N-(4-(4-(2, 3-dihydrobenzofuran-7-yl)-1,4-diazepane-1-yl)butyl)indol-2-ylcarbamide
N-(4-(4-(2,3-dihydrobenzofuran-7-yl)-1,4-diazepane-1-yl)butyl)-6-cyanindol-2-ylcarbamide
N-(4-(4-(chroman-8-yl)piperazine-1-yl)butyl)benzo[b]thiophene-2-ylcarbamide
N-(4-(4-(chroman-8-yl)piperazine-1-yl)butyl)-3-chlorobenzo[b]thiophene-2-ylcarbamide
N-(4-(4-(chroman-8-yl)piperazine-1-yl)butyl)-5-cyanobenzo[b]thiophene-2-ylcarbamide
N-(4-(4-(chroman-8-yl)piperazine-1-yl)butyl)-6-ethinylbenzo[b]thiophene-2-ylcarbamide
N-(4-(4-(chroman-8-yl)piperazine-1-yl)butyl)benzofuran-2-ylcarbamide
N-(4-(4-(chroman-8-yl)piperazine-1-yl)butyl)-5-bromobenzofuran-2-ylcarbamide
N-(4-(4-(chroman-8-yl)piperazine-1-yl)butyl)indol-2-ylcarbamide
N-(4-(4-(chroman-8-yl)piperazine-1-yl)butyl)-6-cyanindol-2-ylcarbamide
N-(4-(4-(chroman-8-yl)-1,4-diazepane-1-yl)butyl)benzo[b]thiophene-2-ylcarbamide
N-(4-(4-(chroman-8-yl)-1,4-diazepane-1-yl)butyl)-3-chlorobenzo[b]thiophene-2-ylcarbamide
N-(4-(4-(chroman-8-yl)-1,4-diazepane-1-yl)butyl)-5-cyanobenzo[b]thiophene-2-ylcarbamide
N-(4-(4-(chroman-8-yl)-1,4-diazepane -1-yl)butyl)-6-ethinylbenzo[b]thiophene-2-ylcarbamide
N-(4-(4-(chroman-8-yl)-1,4-diazepane-1-yl)butyl)benzofuran-2-ylcarbamide
N-(4-(4-(chroman-8-yl)-1,4-diazepane-1-yl)butyl)-5-bromobenzofuran-2-ylcarbamide
N-(4-(4-(chroman-8-yl)-1,4-diazepane-1-yl)butyl)indol-2-ylcarbamide
N-(4-(4-(chroman-8-yl)-1,4-diazepane-1-yl)butyl)-6-cyanindol-2-ylcarbamide
N-(4-(4-(2,3,4,5-tetrahydrobenzo[b]oxepin-9-yl)piperazine-1-yl)butyl)benzo[b]thiophene-2-ylcarbamide
N-(4-(4-(2,3,4,5-tetrahydrobenzo[b]oxepin-9-yl)piperazine-1-yl)butyl)-3-chlorobenzo[b]thiophene-2-ylcarbamide
N-(4-(4-(2,3,4,5-tetrahydrobenzo[b]oxepin-9-yl)piperazine-1-yl)butyl)-5-cyanobenzo[b]thiophene-2-ylcarbamide
N-(4-(4-(2,3,4,5-tetrahydrobenzo[b]oxepin-9-yl)piperazine-1-yl)butyl)-6-ethinylbenzo[b]thiophene-2-ylcarbamide
N-(4-(4-(2,3,4,5-tetrahydrobenzo[b]oxepin-9-yl)piperazine-1-yl)butyl)benzofuran-2-ylcarbamide
N-(4-(4-(2, 3,4,5-tetrahydrobenzo[b]oxepin-9-yl)piperazine-1-yl)butyl)-5-bromobenzofuran-2-ylcarbamide
N-(4-(4-(2,3,4,5-tetrahydrobenzo[b]oxepin-9-yl)piperazine-1-yl)butyl)indol-2-ylcarbamide
N-(4-(4-(2,3,4,5-tetrahydrobenzo[b]oxepin-9-yl)piperazine-1-yl)butyl)-6-cyanindol-2-ylcarbamide
N-(4-(4-(2,3,4,5-tetrahydrobenzo[b]oxepin-9-yl)-1,4-diazepane-1-yl)butyl)benzo[b]thiophene-2-ylcarbamide
N-(4-(4-(2,3,4,5-tetrahydrobenzo[b]oxepin-9-yl)-1,4-diazepane -1-yl)butyl)-3-chlorobenzo[b]thiophene-2-ylcarbamide
N-(4-(4-(2,3,4,5-tetrahydrobenzo[b]oxepin-9-yl)-1,4-diazepane-1-yl)butyl)-5-cyanobenzo[b]thiophene-2-ylcarbamide
N-(4-(4-(2,3,4,5-tetrahydrobenzo[b]oxepin-9-yl)-1,4-diazepane-1-yl)butyl)-6-ethinylbenzo[b]thiophene-2-ylcarbamide
N-(4-(4-(2,3,4,5-tetrahydrobenzo[b]oxepin-9-yl)-1,4-diazepane-1-yl)butyl)benzofuran-2-ylcarbamide
N-(4-(4-(2,3,4,5-tetrahydrobenzo[b]oxepin-9-yl)-1,4-diazepane-1-yl)butyl)-5-bromobenzofuran-2-ylcarbamide
N-(4-(4-(2,3,4,5-tetrahydrobenzo[b]oxepin-9-yl)-1,4-diazepane-1-yl)butyl)indol-2-ylcarbamide
N-(4-(4-(2,3,4,5-tetrahydrobenzo[b]oxepin-9-yl)-1,4-diazepane-1-yl)butyl)-6-cyanindol-2-ylcarbamide
N-(4-(4-(benzo[1,3]dioxol-4-yl)piperazine-1-yl)butyl)benzo[b]thiophene-2-ylcarbamide
N-(4-(4-(benzo[1,3]dioxol-4-yl)piperazine-1-yl)butyl)-3-chlorobenzo[b]thiophene-2-ylcarbamide
N-(4-(4-(benzo[1,3]dioxol-4-yl)piperazine-1-yl)butyl)-5-cyanobenzo[b]thiophene-2-ylcarbamide
N-(4-(4-(benzo[1,3]dioxol-4-yl)piperazine-1-yl)butyl)-6-ethinylbenzo[b]thiophene-2-ylcarbamide
N-(4-(4-(benzo[1,3]dioxol-4-yl)piperazine-1-yl)butyl)benzofuran-2-ylcarbamide
N-(4-(4-(benzo[1,3]dioxol-4-yl)piperazine-1-yl)butyl)-5-bromobenzofuran-2-ylcarbamide
N-(4-(4-(benzo[1,3]dioxol-4-yl)piperazine-1-yl)butyl)indol-2-ylcarbamide
N-(4-(4-(benzo[1,3]dioxol-4-yl)piperazine-1-yl)butyl)-6-cyanindol-2-ylcarbamide
N-(4-(4-(6-chloro-3-oxo-3,4-dihydro-2H-benzo[1,4]oxazin-8-yl)piperazine-1-yl)butyl)benzo[b]thiophene-2-ylcarbamide

20. Compound according to claim 12, selected from among
(a) a compound of formula III from the group comprising
N-(4-(4-(2, 3-dihydrobenzofuran-7-yl)piperazine-1-yl)butyl)benzo[b]thiophene-3-ylcarbamide
N-(4-(4-(2,3-dihydrobenzofuran-7-yl)piperazine-1-yl)butyl)benzofuran-3-ylcarbamide
N-(4-(4-(2,3-dihydrobenzofuran-7-yl)piperazine-1-yl)butyl)indol-3-ylcarbamide
N-(4-(4-(2,3-dihydrobenzofuran-7-yl)-1,4-diazepane-1-yl)butyl)benzo[b]thiophene-3-ylcarbamide
N-(4-(4-(2, 3-dihydrobenzofuran-7-yl)-1,4-diazepane-1-yl)butyl)benzofuran-3-ylcarbamide
N-(4-(4-(2,3-dihydrobenzofuran-7-yl)-1,4-diazepane-1-yl)butyl)indol-3-ylcarbamide
N-(4-(4-(chroman-8-yl)piperazine-1-yl)butyl)benzo[b]thiophene-3-ylcarbamide
N-(4-(4-(chroman-8-yl)piperazine-1-yl)butyl)benzofuran-3-ylcarbamide
N-(4-(4-(chroman-8-yl)piperazine-1-yl)butyl)indol-3-ylcarbamide
N-(4-(4-(chroman-8-yl)-1,4-diazepane-1-yl)butyl)benzo[b]thiophene-3-ylcarbamide
N-(4-(4-(chroman-8-yl)-1,4-diazepane-1-yl)butyl)benzofuran-3-ylcarbamide
N-(4-(4-(chroman-8-yl)-1,4-diazepane-1-yl)butyl)indol-3-ylcarbamide
N-(4-(4-(2,3,4,5-tetrahydrobenzo[b]oxepin-9-yl)piperazine-1-yl)butyl)benzo[b]thiophene-3-ylcarbamide
N-(4-(4-(2,3,4,5-tetrahydrobenzo[b]oxepin-9-yl)piperazine-1-yl)butyl)benzofuran-3-ylcarbamide
N-(4-(4-(2,3,4,5-tetrahydrobenzo[b]oxepin-9-yl)piperazine-1-yl)butyl)indol-3-ylcarbamide
N-(4-(4-(2,3,4,5-tetrahydrobenzo[b]oxepin-9-yl)-1,4-diazepane-1-yl)butyl)benzo[b]thiophene-3-ylcarbamide
N-(4-(4-(2,3,4,5-tetrahydrobenzo[b]oxepin-9-yl)-1,4-diazepane-1-yl)butyl)benzofuran-3-ylcarbamide
N-(4-(4-(2,3,4,5-tetrahydrobenzo[b]oxepin-9-yl)-1,4-diazepane-1-yl)butyl)indol-3-ylcarbamide
N-(4-(4-(benzo[1,3]dioxol-4-yl)piperazine-1-yl)butyl)benzo[b]thiophene-3-ylcarbamide
N-(4-(4-(benzo[1,3]dioxol-4-yl)piperazine-1-yl)butyl)benzofuran-3-ylcarbamide
N-(4-(4-(benzo[1,3]dioxol-4-yl)piperazine-1-yl)butyl)indol-3-ylcarbamide
(b) a compound of formula IV from the group comprising
N-(4-(4-(chroman-7-yl)piperazine-1-yl)butyl)benzo[b]thiophene-2-ylcarbamide
N-(4-(4-(chroman-7-yl)piperazine-1-yl)butyl)-3-chlorobenzo[b]thiophene-2-ylcarbamide
N-(4-(4-(chroman-7-yl)piperazine-1-yl)butyl)-5-cyanobenzo[b]thiophene-2-ylcarbamide
N-(4-(4-(chroman-7-yl)piperazine-1-yl)butyl)-6-ethinylbenzo[b]thiophene-2-ylcarbamide
N-(4-(4-(chroman-7-yl)piperazine-1-yl)butyl)benzofuran-2-ylcarbamide
N-(4-(4-(chroman-7-yl)piperazine-1-yl)butyl)-5-bromobenzofuran-2-ylcarbamide
N-(4-(4-(chroman-7-yl)piperazine-1-yl)butyl)indol-2-ylcarbamide
N-(4-(4-(chroman-7-yl)piperazine-1-yl)butyl)-6-cyanindol-2-ylcarbamide
N-(4-(4-(benzo[1,3]dioxol-5-yl)piperazine-1-yl)butyl)benzo[b]thiophene-2-ylcarbamide
N-(4-(4-(benzo[1,3]dioxol-5-yl)piperazine-1-yl)butyl)-3-chlorobenzo[b]thiophene-2-ylcarbamide
N-(4-(4-(benzo[1,3]dioxol-5-yl)piperazine-1-yl)butyl)-5-cyanobenzo[b]thiophene-2-ylcarbamide
N-(4-(4-(benzo[1,3]dioxol-5-yl)piperazine-1-yl)butyl)-6-ethinylbenzo[b]thiophene-2-ylcarbamide
N-(4-(4-(benzo[1,3]dioxol-5-yl)piperazine-1-yl)butyl)benzofuran-2-ylcarbamide
N-(4-(4-(benzo[1,3]dioxol-5-yl)piperazine-1-yl)butyl)-5-bromobenzofuran-2-ylcarbamide
N-(4-(4-(benzo[1,3]dioxol-5-yl)piperazine-1-yl)butyl)indol-2-ylcarbamide
N-(4-(4-(benzo[1,3]dioxol-5-yl)piperazine-1-yl)butyl)-6-cyanindol-2-ylcarbamide
N-(4-(4-(2,3-dihydrobenzo[1,4]dioxin-6-yl)piperazine-1-yl)butyl)benzo[b]thiophene-2-ylcarbamide
N-(4-(4-(2,3-dihydrobenzo[1,4]dioxin-6-yl)piperazine-1-yl)butyl)-3-chlorobenzo[b]thiophene-2-ylcarbamide
N-(4-(4-(2,3-dihydrobenzo[1,4]dioxin-6-yl)piperazine-1-yl)butyl)-5-cyanobenzo[b]thiophene-2-ylcarbamide
N-(4-(4-(2,3-dihydrobenzo[1,4]dioxin-6-yl)piperazine-1-yl)butyl)-6-ethinylbenzo[b]thiophene-2-ylcarbamide
N-(4-(4-(2,3-dihydrobenzo[1,4]dioxin-6-yl)piperazine-1-yl)butyl)benzofuran-2-ylcarbamide
N-(4-(4-(2,3-dihydrobenzo[1,4]dioxin-6-yl)piperazine-1-yl)butyl)-5-bromobenzofuran-2-ylcarbamide
N-(4-(4-(2,3-dihydrobenzo[1,4]dioxin-6-yl)piperazine-1-yl)butyl)indol-2-ylcarbamide
N-(4-(4-(2,3-dihydrobenzo[1,4]dioxin-6-yl)piperazine-1-yl)butyl)-6-cyanindol-2-ylcarbamide
N-(4-(4-(2,3-dihydrobenzo[1,4]dioxin-6-yl)-1,4-diazepane-1-yl)butyl)benzo[b]thiophene-2-ylcarbamide
N-(4-(4-(2,3-dihydrobenzo[1,4]dioxin-6-yl)-1,4-diazepane-1-yl)butyl)-5-cyanobenzo[b]thiophene-2-ylcarbamide
N-(4-(4-(2,3-dihydrobenzo[1,4]dioxin-6-yl)-1,4-diazepane-1-yl)butyl)benzofuran-2-ylcarbamide
N-(4-(4-(2,3-dihydrobenzo[1,4]dioxin-6-yl)-1,4-diazepane-1-yl)butyl)-5-bromobenzofuran-2-ylcarbamide
N-(4-(4-(2,3-dihydrobenzo[1,4]dioxin-6-yl)-1,4-diazepane-1-yl)butyl)indol-2-ylcarbamide
N-(4-(4-(2, 3-dihydrobenzo[1,4]dioxin-6-yl)-1,4-diazepane-1-yl)butyl)-6-cyanoindol-2-ylcarbamide
N-(4-(4-(3,4-dihydro-2H-benzo[b][1,4]dioxepin-7-yl)piperazine-1-yl)butyl)benzo[b]thiophene-2-ylcarbamide
N-(4-(4-(3,4-dihydro-2H-benzo[b][1,4]dioxepin-7-yl)piperazine-1-yl)butyl)-3-chlorobenzo[b]thiophene-2-ylcarbamide
N-(4-(4-(3,4-dihydro-2H-benzo[b][1,4]dioxepin-7-yl)piperazine-1-yl)butyl)-5-cyanobenzo[b]thiophene-2-ylcarbamide
N-(4-(4-(3,4-dihydro-2H-benzo[b][1,4]dioxepin-7-yl)piperazine-1-yl)butyl)-6-ethinylbenzo[b]thiophene-2-ylcarbamide
N-(4-(4-(3,4-dihydro-2H-benzo[b][1,4]dioxepin-7-yl)piperazine-1-yl)butyl)benzofuran-2-ylcarbamid
N-(4-(4-(3,4-dihydro-2H-benzo[b][1,4]dioxepin-7-yl)piperazine-1-yl)butyl)-5-bromobenzofuran-2-ylcarbamide
N-(4-(4-(3,4-dihydro-2H-benzo[b][1,4]dioxepin-7-yl)piperazine-1-yl)butyl)indol-2-ylcarbamide
N-(4-(4-(3,4-dihydro-2H-benzo[b][1,4]dioxepin-7-yl)piperazine-1-yl)butyl)-6-cyanindol-2-ylcarbamide
(c) a compound of formula V from the group comprising
N-(4-(4-(chroman-7-yl)piperazine-1-yl)butyl)benzo[b]thiophene-3-ylcarbamide
N-(4-(4-(chroman-7-yl)piperazine-1-yl)butyl)benzofuran-3-ylcarbamide
N-(4-(4-(chroman-7-yl)piperazine-1-yl)butyl)indol-3-ylcarbamide
N-(4-(4-(benzo[1, 3]dioxol-5-yl)piperazine-1-yl)butyl)benzo[b]thiophene-3-ylcarbamide
N-(4-(4-(benzo[1,3]dioxol-5-yl)piperazine-1-yl)butyl)benzofuran-3-ylcarbamide
N-(4-(4-(benzo[1,3]dioxol-5-yl)piperazine-1-yl)butyl)indol-3-ylcarbamide
N-(4-(4-(2,3-dihydrobenzo[1,4]dioxin-6-yl)piperazine-1-yl)butyl)benzo[b]thiophene-3-ylcarbamide
N-(4-(4-(2,3-dihydrobenzo[1,4]dioxin-6-yl)piperazine-1-yl)butyl)benzofuran-3-ylcarbamide
N-(4-(4-(2,3-dihydrobenzo[1,4]dioxin-6-yl)piperazine-1-yl)butyl)indol-3-ylcarbamide
N-(4-(4-(2, 3-dihydrobenzo[1,4]dioxin-6-yl)-1,4-diazepane-1-yl)butyl)benzo[b]thiophene-3-ylcarbamide
N-(4-(4-(2,3-dihydrobenzo[1,4]dioxin-6-yl)-1,4-diazepane-1-yl)butyl)benzofuran-3-ylcarbamide
N-(4-(4-(2,3-dihydrobenzo[1,4]dioxin-6-yl)-1,4-diazepane-1-yl)butyl)indol-3-ylcarbamide
N-(4-(4-(3,4-dihydro-2H-benzo[b][1,4]dioxepin-7-yl)piperazine-1-yl)butyl)benzo[b]thiophene-3-ylcarbamide
N-(4-(4-(3,4-dihydro-2H-benzo[b][1,4]dioxepin-7-yl)piperazine-1-yl)butyl)benzofuran-3-ylcarbamid
N-(4-(4-(3,4-dihydro-2H-benzo[b][1,4]dioxepin-7-yl)piperazine-1-yl)butyl)indol-3-ylcarbamide
(d) a compound of formula VI from the group comprising
N-(4-(4-(2,3-dihydrobenzofuran-5-yl)piperazine-1-yl)butyl)benzo[b]thiophene-2-ylcarbamide
N-(4-(4-(2,3-dihydrobenzofuran-5-yl)piperazine-1-yl)butyl)-5-cyanobenzo[b]thiophene-2-ylcarbamide
N-(4-(4-(2,3-dihydrobenzofuran-5-yl)piperazine-1-yl)butyl)benzofuran-2-ylcarbamide
N-(4-(4-(2,3-dihydrobenzofuran-5-yl)piperazine-1-yl)butyl)-5-bromobenzofuran-2-ylcarbamide
N-(4-(4-(2,3-dihydrobenzofuran-5-yl)piperazine-1-yl)butyl)indol-2-ylcarbamide
N-(4-(4-(2,3-dihydrobenzofuran-5-yl)piperazine-1-yl)butyl)-6-cyanindol-2-ylcarbamide
N-(4-(4-(chroman-6-yl)piperazine-1-yl)butyl)benzo[b]thiophene-2-ylcarbamide
N-(4-(4-(chroman-6-yl)piperazine-1-yl)butyl)-5-cyanobenzo[b]thiophene-2-ylcarbamide
N-(4-(4-(chroman-6-yl)piperazine-1-yl)butyl)benzofuran-2-ylcarbamide
N-(4-(4-(chroman-6-yl)piperazine-1-yl)butyl)-5-bromobenzofuran-2-ylcarbamide
N-(4-(4-(chroman-6-yl)piperazine-1-yl)butylindol-2-ylcarbamide
N-(4-(4-(chroman-6-yl)piperazine-1-yl)butyl)-6-cyanindol-2-ylcarbamide
N-(4-(4-(2,3,4,5-tetrahydrobenzo[b]oxepin-7-yl)piperazine-1-yl)butyl)benzo[b]thiophene-2-ylcarbamide
N-(4-(4-(2,3,4,5-tetrahydrobenzo[b]oxepin-7-yl)piperazine-1-yl)butyl)-5-cyanobenzo[b]thiophene-2-ylcarbamide
N-(4-(4-(2,3,4,5-tetrahydrobenzo[b]oxepin-7-yl)piperazine-1-yl)butyl)benzofuran-2-ylcarbamide
N-(4-(4-(2,3,4,5-tetrahydrobenzo[b]oxepin-7-yl)piperazine-1-yl)butyl)-5-bromobenzofuran-2-ylcarbamide
N-(4-(4-(2,3,4,5-tetrahydrobenzo[b]oxepin-7-yl)piperazine-1-yl)butyl)indol-2-ylcarbamide
N-(4-(4-(2,3,4,5-tetrahydrobenzo[b]oxepin-7-yl)piperazine-1-yl)butyl)-6-cyanindol-2-ylcarbamide
(e) a compound of formula VII from the group comprising
N-(4-(4-(2,3-dihydrobenzofuran-5-yl)piperazine-1-yl)butyl)benzo[b]thiophene-3-ylcarbamide
N-(4-(4-(2,3-dihydrobenzofuran-5-yl)piperazine-1-yl)butyl)benzofuran-3-ylcarbamide
N-(4-(4-(2,3-dihydrobenzofuran-5-yl)piperazine-1-yl)butyl)indol-3-ylcarbamide
N-(4-(4-(chroman-6-yl)piperazine-1-yl)butyl)benzo[b]thiophene-3-ylcarbamide
N-(4-(4-(chroman-6-yl)piperazine-1-yl)butyl)benzofuran-3-ylcarbamide
N-(4-(4-(chroman-6-yl)piperazine-1-yl)butyl)indol-3-ylcarbamide
N-(4-(4-(2,3,4,5-tetrahydrobenzo[b]oxepin-7-yl)piperazine-1-yl)butyl)benzo[b]thiophene-3-ylcarbamide
N-(4-(4-(2,3,4,5-tetrahydrobenzo[b]oxepin-7-yl)piperazine-1-yl)butyl)benzofuran-3-ylcarbamide
N-(4-(4-(2,3,4,5-tetrahydrobenzo[b]oxepin-7-yl)piperazine-1-yl)butyl)indol-3-ylcarbamide

21. Compounds according to any one of the preceding claims as a medicine.

22. Pharmaceutical composition comprising one or more of the compounds according to any one of claims 1-20 and at least one pharmaceutically acceptable adjuvant.

23. Use of a compound according to any one of claims 1-20 for the production of a pharmaceutical preparation for the treatment of central nervous system illnesses.

24. Use of a compound according to any one of claims 1-20 for the production of a pharmaceutical preparation for treatment of urinary tract disorders.

25. Use of a compound according to any one of claims 1-20 for production of a pharmaceutical preparation for the treatment of illnesses from the group comprising psychoses, schizophrenia, anxiety disorders, compulsive disorders, drug dependency, depressive disorders, drug-induced extrapyramidal motor disturbances, Parkinson's disease, Segawa syndrome, Tourette's syndrome, restless leg syndrome, sleeping disorders, nausea, cognitive disorders, male erectile dysfunction, hyperprolactinemia, hyperprolactinomia, glaucoma, attention deficit hyperactive syndrome (ADHS), autism, stroke and urinary incontinence.

26. Use according to any one of claims 1-20, wherein the compound is used for production of a pharmaceutical preparation for the treatment of schizophrenia, depressive disorders, L-dopa- or neuroleptic drug-induced motor disturbances, Parkinson's disease, Segawa syndrome, restless leg syndrome, hyperprolactinemia, hyperprolactinomia, attention deficit hyperactivity syndrome (ADHS) or urinary incontinence.

27. Use of a compound according to any one of claims 1-20 for the production of a pharmaceutical preparation for the treatment of pain.

28. Method of producing a compound according to anyone of claims 1-20 comprising combining an acid derivative A 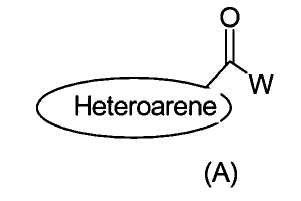 with a free base of general formula C 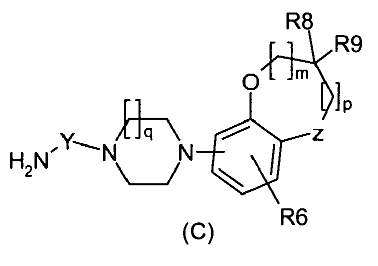 wherein:
W is selected from OH, CI, Br or a group 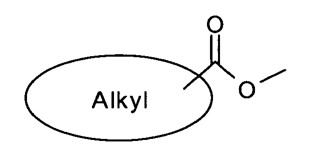 "Heteroarene" in the acid derivative A stands for a group of general formula la 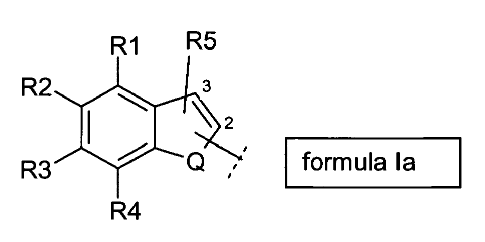
R1, R2, R3, R4, R5, R6, R8, R9, Q, Z, m, p and q are as defined in claims 1-20; and wherein the bond crossed by a dotted line in formula la stands for the bonding of the -C(O)-W group to the 2- or 3-position of said heteroarene with formula Ia.

29. Method according to claim 28, wherein W is chlorine, bromine or OH.

30. Method according to claim 28, wherein W is a hydroxy group, and wherein the acid group prior to the conversion with the free base of general formula C is activated by the addition of an activation reagent.

31. Method according to claim 30, wherein the activation reagent is selected from hydroxybenzotriazole, hydroxyazabenzotriazole, HATU and TBTU.

## Revendications

1. Composés selon la formule générale I, 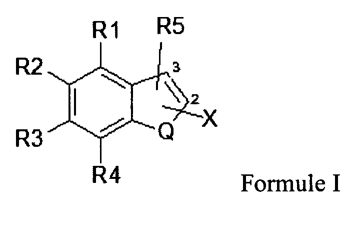 dans laquelle :
Q est choisi parmi S, O et NR ;
R est choisi parmi hydrogène, alkyle, phényle, alkylcarbonyle, phénylalkylcarbonyle, phénylcarbonyle, phénylalkyle et phénylsulfonyle ;
R1, R2, R3 et R4 sont choisis indépendamment les uns des autres parmi hydrogène, hydroxy, alkyle, alkyloxy, alkylthio, alkényle, alkynyle, phényle, phénylalkyle, phénoxy, halogène, trifluorométhyle, alkylcarbonyle, phénylcarbonyle, phénylalkylcarbonyle, alkyloxycarbonyle, phénylalkyloxycarbonyle, cyano, nitro, amino, carboxy, sulfo, sulfamoyle, sulfonylamino, alkylaminosulfonyle et alkylsulfonylamino ;
R5 est un groupe lié à la position 2 ou 3 de l'hétéroaryle bicyclique, qui est choisi parmi hydrogène, alkyle, halogène, alkoxy et amino ;
X est un groupe lié à la position 2 ou 3 de l'hétéroaryle bicyclique et ayant la formule générale X1 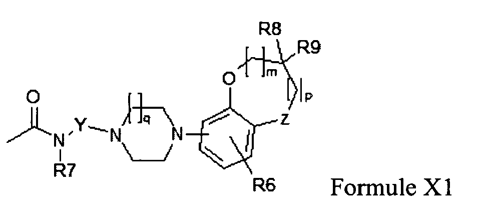
dans laquelle :
R6 est choisi parmi hydrogène, hydroxy, alkyle, alkyloxy, alkylthio, alkényle, alkynyle, phényle, phénylalkyle, phénoxy, halogène, trifluorométhyle, alkylcarbonyle, phénylcarbonyle, phénylalkylcarbonyle, alkyloxycarbonyle, phénylalkyloxycarbonyle, cyano, nitro, amino, carboxy, sulfo, sulfamoyle, sulfonylamino, alkylaminosulfonyle et alkylsulfonylamino ;
R7 est l'hydrogène, un alkyle ou un phénylalkyle ;
Y est une chaîne d'atomes de carbone saturée ou insaturée comprenant entre 2 et 5 atomes de carbone ;
m et p sont égaux, indépendamment l'un de l'autre, à 0, 1 ou 2, la somme de m et p étant au maximum égale à 2;
q est égal à 1 ou 2;
Z est CH₂, NH ou O ;
R8 et R9 sont choisis indépendamment l'un de l'autre parmi hydrogène, alkyle et phényle ou forment ensemble un groupe oxo ;
sous la forme de la base libre, de ses sels physiologiquement acceptables ainsi que d'éventuels énantiomères et diastéréomères.

2. Composés selon la revendication 1, dans lesquels X a la formule générale X2 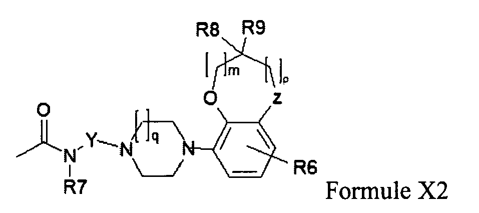 dans laquelle R6, R7, R8, R9, m, p, q, Y et Z ont la signification définie dans la revendication 1.

3. Composé selon l'une quelconque des revendications précédentes, dans lequel R8 et R9 représentent chacun un atome d'hydrogène.

4. Composé selon l'une quelconque des revendications précédentes, dans lequel R6 et R7 représentent chacun l'hydrogène.

5. Composés selon l'une quelconque des revendications précédentes, dans lesquels Z représente un groupe CH₂ ou un atome d'oxygène.

6. Composés selon l'une quelconque des revendications 1 à 4, dans lesquels Z représente NH.

7. Composé selon l'une quelconque des revendications précédentes, dans lequel la somme de m et p est égale à 1 ou 2.

8. Composé selon l'une quelconque des revendications précédentes, dans lequel R1, R4, R5, R6 et R7 représentent chacun un atome d'hydrogène et Y représente une chaîne carbonée linéaire saturée avec 4 ou 5 atomes de carbone.

9. Composé selon l'une quelconque des revendications précédentes, dans lequel q est égal à 1.

10. Composé selon la revendication 1 ou 2, dans lequel :
- Q est choisi parmi S, O et NH;
- R1 et R4 représentent H ;
- R5 est H ou un halogène ;
- R2 et R3 sont choisis indépendamment parmi hydrogène, hydroxy, fluor, chlore, brome, trifluorométhyle, cyano, amino, carboxy, sulfo, sulfamoyle, alkyle en C₁-C₆ non substitué ou substitué avec hydroxy ; alkyloxy en C₁-C₆ non substitué ou substitué avec hydroxy ; alkylthio en C₁-C₆ non substitué ou substitué avec hydroxy ; alkynyle en C-C6 non substitué ; phényle non substitué ou substitué avec fluor, chlore ou brome et/ou un ou plusieurs groupes méthoxy ; phénylalkyle en C₁-C₆, dans lequel le phényle est non substitué ou substitué avec fluor, chlore ou brome et/ou un ou plusieurs groupes méthoxy et dans lequel l'alkyle en C₁-C₆ est non substitué ou substitué avec hydroxy ; phénoxy non substitué ou substitué avec fluor, chlore ou brome et/ou un ou plusieurs groupes méthoxy ; -C(O)-alkyle en C₁-C₆, dans lequel l'alkyle est non substitué ou substitué avec hydroxy ; -C(O)-phényle, dans lequel le phényle est non substitué ou substitué avec fluor, chlore ou brome et/ou un ou plusieurs groupes méthoxy ; -C(O)- (C₁-C₆)alkyl-phényle, dans lequel le phényle est non substitué ou substitué avec fluor, chlore ou brome et/ou un ou plusieurs groupes méthoxy et dans lequel l'alkyle en C₁-C₆ est non substitué ou substitué avec hydroxy ; alkyloxycarbonyle en C₁-C₆, dans lequel l'alkyle est non substitué ou substitué avec hydroxy ; phénylalkyloxycarbonyle en C₁-C₆, dans lequel le phényle est non substitué ou substitué avec fluor, chlore ou brome et/ou un ou plusieurs groupes méthoxy et dans lequel l'alkyle en C₁-C₆ est non substitué ou substitué avec hydroxy ; alkylaminosulfonyle en C₁-C₆, en particulier méthylaminosulfonyle et alkylsulfonylamino en C₁-C₆ ; en particulier méthanesulfonylamino ;
- X est un groupe selon la formule X1 ou X2, dans laquelle :
o R6 représente l'hydrogène, un alkyle en C₁-C₆, un alkoxy en C₁-C₆ ou un halogène ;
o R7 représente l'hydrogène ou un alkyle en C₁-C₆;
o R8 et R9 représentent hydrogène;
o Z est CH₂ ou O;
o la somme de m et p est égale à 0, 1 ou 2 et en particulier à 1 ou 2 ;
o q est égal à 1 ou 2 ;
o Y est une chaîne carbonée linéaire saturée avec 3, 4 ou 5 atomes de carbone.

11. Composé selon la revendication 1 selon la formule générale II 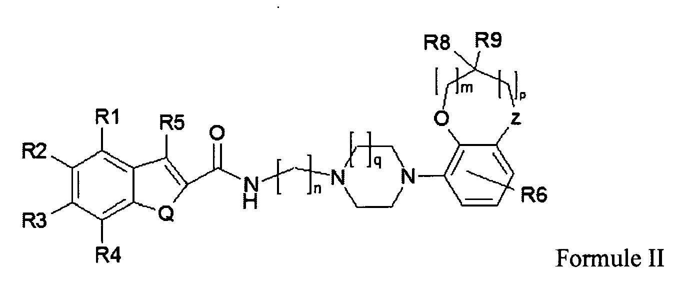 dans laquelle R, R1, R2, R3, R4, R5, R6, R8, R9, Q, Z, m, p et q ont la signification définie dans l'une des revendications précédentes et dans laquelle n est égal à 3, 4 ou 5.

12. Composé selon la revendication 1 selon la formule générale III, IV, V, VI ou VII 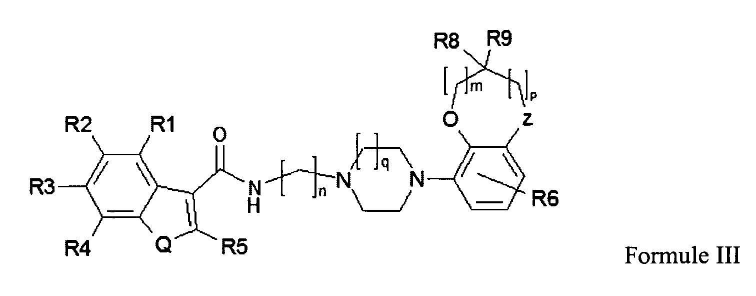 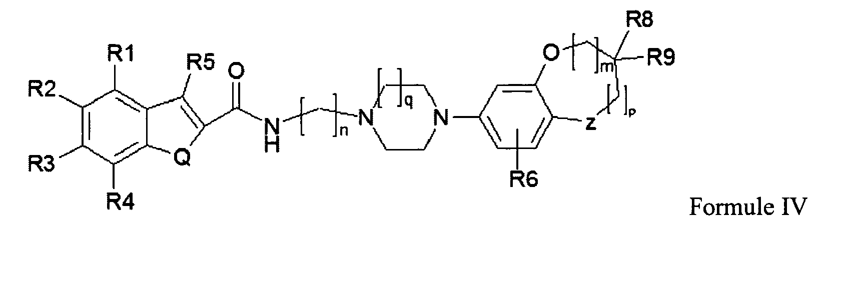 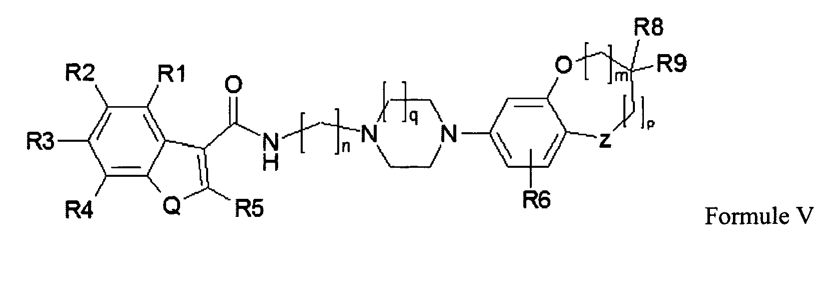 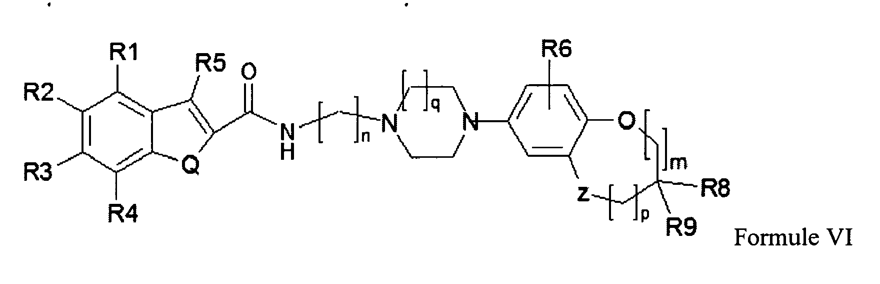 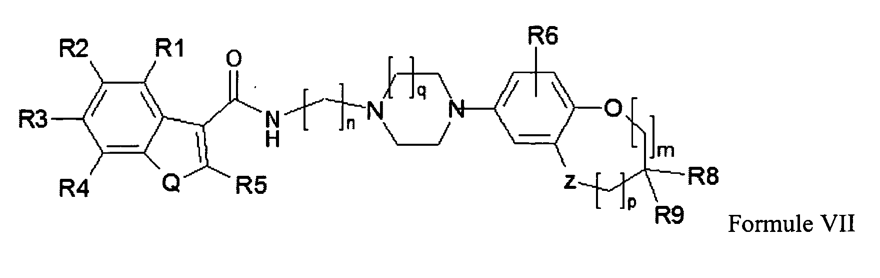 dans laquelle R, R1, R2, R3, R4, R5, R6, R8, R9, Q, Z, m, p et q ont chacun la signification définie dans la revendication 1 et dans laquelle n est égal à 3, 4 ou 5.

13. Composés selon l'une quelconque des revendications 11 ou 12, dans lesquels Z représente un groupe CH₂ ou un atome d'oxygène.

14. Composé selon l'une quelconque des revendications 11 à 13, dans lequel la somme de m et p est égale à 1 ou 2.

15. Composé selon l'une quelconque des revendications 11 à 14, dans lequel R1, R4 et R6 représentent chacun un atome d'hydrogène.

16. Composé selon l'une quelconque des revendications 11 à 15, dans lequel R8 et R9 représentent chacun un atome d'hydrogène.

17. Composé selon l'une quelconque des revendications 11 à 16, dans lequel q est égal à 1.

18. Composé selon l'une quelconque des revendications 11 ou 12, dans lequel :
- R1, R4, R6, R8 et R9 représentent chacun un atome d'hydrogène ;
- R2 et R3 sont choisis chacun indépendamment parmi hydrogène, hydroxy, fluor ; chlore ; brome ; trifluorométhyle ; cyano ; amino ; carboxy ; sulfo; sulfamoyle; alkyle en C₁-C₆ non substitué ou substitué avec hydroxy ; alkyloxy en C₁-C₆ non substitué ou substitué avec hydroxy ; alkylthio en C₁-C₆ non substitué ou substitué avec hydroxy ; alkynyle en C-C6 non substitué ; phényle non substitué ou substitué avec fluor, chlore ou brome et/ou un ou plusieurs groupes méthoxy ; phénylalkyle en C₁-C₆, dans lequel le phényle est non substitué ou substitué avec fluor, chlore ou brome et/ou un ou plusieurs groupes méthoxy et dans lequel l'alkyle en C₁-C₆ est non substitué ou substitué avec hydroxy ; phénoxy non substitué ou substitué avec fluor, chlore ou brome et/ou un ou plusieurs groupes méthoxy ; -C(O)-alkyle en C₁-C₆, dans lequel l'alkyle est non substitué ou substitué avec hydroxy; -C(O)-phényle, dans lequel le phényle est non substitué ou substitué avec fluor, chlore ou brome et/ou un ou plusieurs groupes méthoxy ; -C(O)- (C₁-C₆)alkyl-phényle, dans lequel le phényle est non substitué ou substitué avec fluor, chlore ou brome et/ou un ou plusieurs groupes méthoxy et dans lequel l'alkyle en C₁-C₆ est non substitué ou substitué avec hydroxy ; alkyloxycarbonyle en C₁-C₆, dans lequel l'alkyle est non substitué ou substitué avec hydroxy ; phénylalkyloxycarbonyle en C₁-C₆, dans lequel le phényle est non substitué ou substitué avec fluor, chlore ou brome et/ou un ou plusieurs groupes méthoxy et dans lequel l'alkyle en C₁-C₆ est non substitué ou substitué avec hydroxy ; alkylaminosulfonyle en C₁-C₆, en particulier méthylaminosulfonyle et alkylsulfonylamino en C₁-C₆; en particulier méthanesulfonylamino ;
- R5 est l'hydrogène ou un halogène;
- n est égal à 4 ou 5 ;
- q est égal à 1 ou 2 ;
- Z est CH₂ ou l'oxygène.

19. Composé selon la revendication 11, choisi parmi:
N-(4-(4-(2,3-dihydrobenzofuran-7-yl)pipérazin-1-yl)butyl)benzo[b]thiophèn-2-ylcarbamide
N-(4-(4-(2,3-dihydrobenzofuran-7-yl)pipérazin-1-yl)butyl)-3-chlorobenzo[b]thiophèn-2-ylcarbamide
N-(4-(4-(2,3-dihydrobenzofuran-7-yl)pipérazin-1-yl)butyl)-5-cyanobenzo[b]thiophèn-2-ylcarbamide
N-(4-(4-(2,3-dihydrobenzofuran-7-yl)pipérazin-1-yl)butyl)-6-éthinylbenzo[b]thiophèn-2-ylcarbamide
N-(4-(4-(2,3-dihydrobenzofuran-7-yl)pipérazin-1-yl)butyl)benzofuran-2-ylcarbamide
N-(4-(4-(2,3-dihydrobenzofuran-7-yl)pipérazin-1-yl)butyl)-5-bromobenzofuran-2-ylcarbamide
N-(4-(4-(2,3-dihydrobenzofuran-7-yl)pipérazin-1-yl)butyl)indol-2-ylcarbamide
N-(4-(4-(2,3-dihydrobenzofuran-7-yl)pipérazin-1-yl)butyl)-6-cyanoindol-2-ylcarbamide
N-(4-(4-(2,3-dihydrobenzofuran-7-yl)-1,4-diazépan-1-yl)butyl)benzo[b]thiophèn-2-ylcarbamide
N-(4-(4-(2,3-dihydrobenzofuran-7-yl)-1,4-diazépan-1-yl)butyl)-3-chlorobenzo[b]thiophèn-2-ylcarbamide
N-(4-(4-(2,3-dihydrobenzofuran-7-yl)-1,4-diazépan-1-yl)butyl)-5-cyanobenzo[b]thiophèn-2-ylcarbamide
N-(4-(4-(2,3-dihydrobenzofuran-7-yl)-1,4-diazépan-1-yl)butyl)-6-éthinylbenzo[b]thiophèn-2-ylcarbamide
N-(4-(4-(2,3-dihydrobenzofuran-7-yl)-1,4-diazépan-1-yl)butyl)benzofuran-2-ylcarbamide
N-(4-(4-(2,3-dihydrobenzofuran-7-yl)-1,4-diazépan-1-yl)butyl)-5-bromobenzofuran-2-yl-carbamide
N-(4-(4-(2,3-dihydrobenzofuran-7-yl)-1,4-diazépan-1-yl)butyl)indol-2-ylcarbamide
N-(4-(4-(2,3-dihydrobenzofuran-7-yl)-1,4-diazépan-1-yl)butyl)-6-cyanoindol-2-ylcarbamide
N-(4-(4-(chroman-8-yl)pipérazin-1-yl)butyl)benzo[b]thiophèn-2-ylcarbamide
N-(4-(4-(chroman-8-yl)pipérazin-1-yl)butyl)-3-chlorobenzo[b]thiophèn-2-ylcarbamide
N-(4-(4-(chroman-8-yl)pipérazin-1-yl)butyl)-5-cyanobenzo[b]thiophèn-2-ylcarbamide
N-(4-(4-(chroman-8-yl)pipérazin-1-yl)butyl)-6-éthinylbenzo[b]thiophèn-2-ylcarbamide
N-(4-(4-(chroman-8-yl)pipérazin-1-yl)butyl)benzofuran-2-ylcarbamide
N-(4-(4-(chroman-8-yl)pipérazin-1-yl)butyl)-5-bromobenzofuran-2-ylcarbamide
N-(4-(4-(chroman-8-yl)pipérazin-1-yl)butyl)indol-2-ylcarbamide
N-(4-(4-(chroman-8-yl)pipérazin-1-yl)butyl)-6-cyanoindol-2-ylcarbamide
N-(4-(4-(chroman-8-yl)-1,4-diazépan-1-yl)butyl)benzo[b]thiophèn-2-ylcarbamide
N-(4-(4-(chroman-8-yl)-1,4-diazépan-1-yl)butyl)-3-chlorobenzo[b]thiophèn-2-ylcarbamide
N-(4-(4-(chroman-8-yl)-1,4-diazépan-1-yl)butyl)-5-cyanobenzo[b]thiophèn-2-ylcarbamide
N-(4-(4-(chroman-8-yl)-1,4-diazépan-1-yl)butyl)-6-éthinylbenzo[b]thiophèn-2-ylcarbamide
N-(4-(4-(chroman-8-yl)-1,4-diazépan-1-yl)butyl)benzofuran-2-ylcarbamide
N-(4-(4-(chroman-8-yl)-1,4-diazépan-1-yl)butyl)-5-bromobenzo%ran-2-ylcarbamide
N-(4-(4-(chroman-8-yl)-1,4-diazépan-1-yl)butyl)indol-2-ylcarbamide
N-(4-(4-(chroman-8-yl)-1,4-diazépan-1-yl)butyl)-6-cyanoindol-2-ylcarbamide
N-(4-(4-(2,3,4,5-tétrahydrobenzo[b]oxépin-9-yl)pipérazin-1-yl)butyl)benzo[b]thiophèn-2-ylcarbamide
N-(4-(4-(2,3,4,5-tétrahydrobenzo[b]oxépin-9-yl)pipérazin-1-yl)butyl)-3-chlorobenzo[b] thiophèn-2-ylcarbamide
N-(4-(4-(2,3,4,5-tétrahydrobenzo[b]oxépin-9-yl)pipérazin-1-yl)butyl)-5-cyanobenzo [b]thiophèn-2-ylcarbamide
N-(4-(4-(2,3,4,5-tétrahydrobenzo[b]oxépin-9-yl)pipérazin-1-yl)butyl)-6-éthinylbenzo [b]thiophèn-2-ylcarbamide
N-(4-(4-(2,3,4,5-tétrahydrobenzo[b]oxépin-9-yl)pipérazin-1-yl)butyl)benzofuran-2-yl-carbamide
N-(4-(4-(2,3,4,5-tétrahydrobenzo[b]oxépin-9-yl)pipérazin-1-yl)butyl)-5-bromobenzofuran-2-ylcarbamide
N-(4-(4-(2,3,4,5-tétrahydrobenzo[b]oxépin-9-yl)pipérazin-1-yl)butyl)indol-2-ylcarbamide
N-(4-(4-(2,3,4,5-tétrahydrobenzo[b]oxépin-9-yl)pipérazin-1-yl)butyl)-6-cyanoindol-2-ylcarbamide
N-(4-(4-(2,3,4,5-tétrahydrobenzo[b]oxépin-9-yl)-1,4-diazépan-1-yl)butyl)benzo[b]thiophèn-2-ylcarbamide
N-(4-(4-(2,3,4,5-tétrahydrobenzo[b]oxépin-9-yl)-1,4-diazépan-1-yl)butyl)-3-chlorobenzo [b]thiophèn-2-ylcarbamide
N-(4-(4-(2,3,4,5-tétrahydrobenzo[b]oxépin-9-yl)-1,4-diazépan-1-yl)butyl)-5-cyanobenzo [b]thiophèn-2-ylcarbamide
N-(4-(4-(2,3,4,5-tétrahydrobenzo[b]oxépin-9-yl)-1,4-diazépan-1 -yl)butyl)-6-éthinylbenzo [b]thiophèn-2-ylcarbamide
N-(4-(4-(2,3,4,5-tétrahydrobenzo[b]oxépin-9-yl)-1,4-diazépan-1-yl)butyl)benzofuran-2-ylcarbamide
N-(4-(4-(2,3,4,5-tétrahydrobenzo[b]oxépin-9-yl)-1,4-diazépan-1-yl)butyl)-5-bromobenzofuran-2-ylcarbamide
N-(4-(4-(2,3,4,5-tétrahydrobenzo[b] oxépin-9-yl)-1,4-diazépan-1-yl)butyl)indol-2-ylcarbamide
N-(4-(4-(2,3,4,5-tétrahydrobenzo[b]oxépin-9-yl)- 1,4-diazépan- 1 -yl)butyl)-6-cyanoindol-2-ylcarbamide
N-(4-(4-(benzo[1,3]dioxol-4-yl)pipérazin-1-yl)butyl)benzo[b]thiophèn-2-ylcarbamide
N-(4-(4-(benzo[1,3]dioxol-4-yl)pipérazin-1-yl)butyl)-3-chlorobenzo[b]thiophèn-2-yl-carbamide
N-(4-(4-(benzo[1,3]dioxol-4-yl)pipérazin-1-yl)butyl)-5-cyanobenzo[b]thiophèn-2-yl-carbamide
N-(4-(4-(benzo[1,3]dioxol-4-yl)pipérazin-1-yl)butyl)-6-éthinylbenzo[b]thiophèn-2-yl-carbamide
N-(4-(4-(benzo[1,3]dioxol-4-yl)pipérazin-1-yl)butyl)benzofuran-2-ylcarbamide
N-(4-(4-(benzo[1,3]dioxol-4-yl)pipérazin-1-yl)butyl)-5-bromobenzofuran-2-ylcarbamide
N-(4-(4-(benzo[1,3]dioxol-4-yl)pipérazin-1-yl)butyl)indol-2-ylcarbamide
N-(4-(4-(benzo[1,3]dioxol-4-yl)pipérazin-1-yl)butyl)-6-cyanoindol-2-ylcarbamide
N-(4-(4-(6-chloro-3-oxo-3,4-dihydro-2H-benzo[1,4]oxazin-8-yl)pipérazin-1-yl)butyl) benzo[b]thiophèn-2-ylcarbamide.

20. Composé selon la revendication 12, choisi parmi :
(a) un composé selon la formule III faisant partie du groupe
N-(4-(4-(2,3-dihydrobenzofuran-7-yl)pipérazin-1-yl)butyl)benzo[b]thiophèn-3-ylcarbamide
N-(4-(4-(2,3-dihydrobenzofuran-7-yl)pipérazin-1-yl)butyl)benzofuran-3-ylcarbamide
N-(4-(4-(2,3-dihydrobenzofuran-7-yl)pipérazin-1-yl)butyl)indol-3-ylcarbamide
N-(4-(4-(2,3-dihydrobenzofuran-7-yl)-1,4-diazépan-1 -yl)butyl)benzo[b]thiophèn-3-ylcarbamide
N-(4-(4-(2,3-dihydrobenzofuran-7-yl)-1,4-diazépan-1-yl)butyl)benzofuran-3-ylcarbamide
N-(4-(4-(2,3-dihydrobenzofuran-7-yl)-1,4-diazépan-1-yl)butyl)indol-3-ylcarbamide
N-(4-(4-(chroman-8-yl)pipérazin-1-yl)butyl)benzo[b]thiophèn-3-ylcarbamide
N-(4-(4-(chroman-8-yl)pipérazin-1-yl)butyl)benzofuran-3-ylcarbamide
N-(4-(4-(chroman-8-yl)pipérazin-1-yl)butyl)indol-3-ylcarbamide
N-(4-(4-(chroman-8-yl)-1,4-diazépan-1-yl)butyl)benzo[b]thiophèn-3-ylcarbamide
N-(4-(4-(chroman-8-yl)-1,4-diazépan-1-yl)butyl)benzofuran-3-ylcarbamide
N-(4-(4-(chroman-8-yl)-1,4-diazépan-1-yl)butyl)indol-3-ylcarbamide
N-(4-(4-(2,3,4,5-tétrahydrobenzo[b]oxépin-9-yl)pipérazin-1-yl)butyl)benzo[b]thiophèn-3-ylcarbamide
N-(4-(4-(2,3,4,5-tétrahydrobenzo[b]oxépin-9-yl)pipérazin-1-yl)butyl)benzofuran-3-ylcarbamide
N-(4-(4-(2,3,4,5-tétrahydrobenzo[b]oxépin-9-yl)pipérazin-1-yl)butyl)indol-3-ylcarbamide
N-(4-(4-(2,3,4,5-tétrahydrobenzo[b]oxépin-9-yl)-1,4-diazépan-1-yl)butyl)benzo[b]thiophèn-3-ylcarbamide
N-(4-(4-(2,3,4,5-tétrahydrobenzo[b]oxépin-9-yl)-1,4-diazépan-1-yl)butyl)benzofuran-3-ylcarbamide
N-(4-(4-(2,3,4,5-tétrahydrobenzo[b]oxépin-9-yl)-1,4-diazépan-1-yl)butyl)indol-3-ylcarbamide
N-(4-(4-(benzo[1,3]dioxol-4-yl)pipérazin-1-yl)butyl)benzo[b]thiophèn-3-ylcarbamide
N-(4-(4-(benzo[1,3]dioxol-4-yl)pipérazin-1-yl)butyl)benzofuran-3-ylcarbamide
N-(4-(4-(benzo[1,3]dioxol-4-yl)pipérazin-1-yl)butyl)indol-3-ylcarbamide
(b) un composé selon la formule IV faisant partie du groupe
N-(4-(4-(chroman-7-yl)pipérazin-1-yl)butyl)benzo[b]thiophèn-2-ylcarbamide
N-(4-(4-(chroman-7-yl)pipérazin-1-yl)butyl)-3-chlorobenzo[b]thiophèn-2-ylcarbamide
N-(4-(4-(chroman-7-yl)pipérazin-1-yl)butyl)-5-cyanobenzo[b]thiophèn-2-ylcarbamide
N-(4-(4-(chroman-7-yl)pipérazin-1-yl)butyl)-6-éthinylbenzo[b]thiophèn-2-ylcarbamide
N-(4-(4-(chroman-7-yl)pipérazin-1-yl)butyl)benzofuran-2-ylcarbamide
N-(4-(4-(chroman-7-yl)pipérazin-1-yl)butyl)-5-bromobenzofuran-2-ylcarbamide
N-(4-(4-(chroman-7-yl)pipérazin-1-yl)butyl)indol-2-ylcarbamide
N-(4-(4-(chroman-7-yl)pipérazin-1-yl)butyl)-6-cyanoindol-2-ylcarbamide
N-(4-(4-(benzo[1,3]dioxol-5-yl)pipérazin-1-yl)butyl)benzo[b]thiophèn-2-ylcarbamide
N-(4-(4-(benzo[1,3]dioxol-5-yl)pipérazin-1-yl)butyl)-3-chlorobenzo[b]thiophèn-2-ylcarbamide
N-(4-(4-(benzo[1,3]dioxol-5-yl)pipérazin-1-yl)butyl)-5-cyanobenzo[b]thiophèn-2-ylcarbamide
N-(4-(4-(benzo[1,3]dioxol-5-yl)pipérazin-1-yl)butyl)-6-éthinylbenzo[b]thiophèn-2-ylcarbamide
N-(4-(4-(benzo[1,3]dioxol-5-yl)pipérazin-1-yl)butyl)benzofuran-2-ylcarbamide
N-(4-(4-(benzo[1,3]dioxol-5-yl)pipérazin-1-yl)butyl)-5-bromobenzofuran-2-ylcarbamide
N-(4-(4-(benzo[1,3]dioxol-5-yl)pipérazin-1-yl)butyl)indol-2-ylcarbamide
N-(4-(4-(benzo[1,3]dioxol-5-yl)pipérazin-1-yl)butyl)-6-cyanoindol-2-ylcarbamide
N-(4-(4-(2,3-dihydrobenzo[1,4]dioxin-6-yl)pipérazin-1-yl)butyl)benzo[b]thiophèn-2-ylcarbamide
N-(4-(4-(2,3-dihydrobenzo[1,4]dioxin-6-yl)pipérazin-1-yl)butyl)-3-chlorobenzo[b]thiophèn-2-ylcarbamide
N-(4-(4-(2,3-dihydrobenzo[1,4]dioxin-6-yl)pipérazin-l-yl)butyl)-5-cyanobenzo[b]thiophèn-2-ylcarbamide
N-(4-(4-(2,3-dihydrobenzo[1,4]dioxin-6-yl)pipérazin-1-yl)butyl)-6-éthinylbenzo[b]thiophèn-2-ylcarbamide
N-(4-(4-(2,3-dihydrobenzo[1,4]dioxin-6-yl)pipérazin-1-yl)butyl)benzofuran-2-yl-carbamide
N-(4-(4-(2,3-dihydrobenzo[1,4]dioxin-6-yl)pipérazin-1-yl)butyl)-5-bromobenzofuran-2-ylcarbamide
N-(4-(4-(2,3-dihydrobenzo[1,4]dioxin-6-yl)pipérazin-1-yl)butyl)indol-2-ylcarbamide
N-(4-(4-(2,3-dihydrobenzo[1,4]dioxin-6-yl)pipérazin-1-yl)butyl)-6-cyanoindol-2-ylcarbamide
N-(4-(4-(2,3-dihydrobenzo[1,4]dioxin-6-yl)-1,4-diazépan-1-yl)butyl)benzo[b]thiophèn-2-ylcarbamide
N-(4-(4-(2,3-dihydrobenzo[1,4]dioxin-6-yl)-1,4-diazépan-1-yl)butyl)-5-cyanobenzo[b] thiophèn-2-ylcarbamide
N-(4-(4-(2,3-dihydrobenzo[1,4]dioxin-6-yl)-1,4-diazépan-l-yl)butyl)benzofuran-2-ylcarbamide
N-(4-(4-(2,3-dihydrobenzo[1,4]dioxin-6-yl)-1,4-diazépan-1-yl)butyl)-5-bromobenzofuran-2-ylcarbamide
N-(4-(4-(2,3-dihydrobenzo[1,4]dioxin-6-yl)-1,4-diazépan-1-yl)butyl)indol-2-ylcarbamide
N-(4-(4-(2,3-dihydrobenzo[1,4]dioxin-6-yl)-1,4-diazépan-1-yl)butyl)-6-cyanoindol-2-ylcarbamide
N-(4-(4-(3,4-dihydro-2H-benzo[b][1,4]dioxépin-7-yl)pipérazin-1-yl)butyl)benzo[b]thiophèn-2-ylcarbamide
N-(4-(4-(3,4-dihydro-2H-benzo[b][1,4]dioxépin-7-yl)pipérazin-1-yl)butyl)-3-chlorobenzo [b]thiophèn-2-ylcarbamide
N-(4-(4-(3,4-dihydro-2H-benzo[b][1,4]dioxépin-7-yl)pipérazin-1-yl)butyl)-5-cyanobenzo [b]thiophèn-2-ylcarbamide
N-(4-(4-(3,4-dihydro-2H-benzo[b][1,4]dioxépin-7-yl)pipérazin-1-yl)butyl)-6-éthinylbenzo [b]thiophèn-2-ylcarbamide
N-(4-(4-(3,4-dihydro-2H-benzo[b][1,4]dioxépin-7-yl)pipérazin-1-yl)butyl)benzofuran-2-ylcarbamide
N-(4-(4-(3,4-dihydro-2H-benzo[b][1,4]dioxépin-7-yl)pipérazin-1-yl)butyl)-5-bromobenzofuran-2-ylcarbamide
N-(4-(4-(3,4-dihydro-2H-benzo[b][1,4]dioxépin-7-yl)pipérazin-1-yl)butyl)indol-2-ylcarbamide
N-(4-(4-(3,4-dihydro-2H-benzo[b][1,4]dioxépin-7-yl)pipérazin-1-yl)butyl)-6-cyanoindol-2-ylcarbamide
(c)uncomposéselonlaformuleVfaisantpartiedugroupe
N-(4-(4-(chroman-7-yl)pipérazin-1-yl)butyl)benzo[b]thiophèn-3-ylcarbamide
N-(4-(4-(chroman-7-yl)pipérazin-1-yl)butyl)benzofuran-3-ylcarbamide
N-(4-(4-(chroman-7-yl)pipérazin-1-yl)butyl)indol-3-ylcarbamide
N-(4-(4-(benzo[1,3]dioxol-5-yl)pipérazin-1-yl)butyl)benzo[b]thiophèn-3-ylcarbamide
N-(4-(4-(benzo[I,3]dioxol-5-yl)pipérazin-1-yl)butyl)benzofuran-3-ylcarbamide
N-(4-(4-(benzo[1,3]dioxol-5-yl)pipérazin-1-yl)butyl)indol-3-ylcarbamide
N-(4-(4-(2,3-dihydrobenzo[1,4]dioxin-6-yl)pipérazin-1-yl)butyl)benzo[b]thiophèn-3-ylcarbamide
N-(4-(4-(2,3-dihydrobenzo[1,4]dioxin-6-yl)pipérazin-1-yl)butyl)benzofuran-3-yl-carbamide
N-(4-(4-(2,3-dihydrobenzo[1,4]dioxin-6-yl)pipérazin-1-yl)butyl)indol-3-ylcarbamide
N-(4-(4-(2,3-dihydrobenzo[1,4]dioxin-6-yl)-1,4-diazépan-1-yl)butyl)benzo[b]thiophèn-3-ylcarbamide
N-(4-(4-(2,3-dihydrobenzo[1,4]dioxin-6-yl)-1,4-diazépan-1-yl)butyl)benzofuran-3-ylcarbamide
N-(4-(4-(2,3-dihydrobenzo[1,4]dioxin-6-yl)-1,4-diazépan-1-yl)butyl)indol-3-ylcarbamide
N-(4-(4-(3,4-dihydro-2H-benzo[b][1,4]dioxépin-7-yl)pipérazin-1-yl)butyl)benzo[b]thiophèn-3-ylcarbamide
N-(4-(4-(3,4-dihydro-2H-benzo[b][1,4]dioxépin-7-yl)pipérazin-1-yl)butyl)benzofuran-3-ylcarbamide
N-(4-(4-(3,4-dihydro-2H-benzo[b][1,4]dioxépin-7-yl)pipérazin-1-yl)butyl)indol-3-ylcarbamide
(d) un composé selon la formule VI faisant partie du groupe
N-(4-(4-(2,3-dihydrobenzofuran-5-yl)pipérazin-1-yl)butyl)benzo[b]thiophèn-2-ylcarbamide
N-(4-(4-(2,3-dihydrobenzofuran-5-yl)pipérazin-1-yl)butyl)-5-cyanobenzo[b]thiophèn-2-ylcarbamide
N-(4-(4-(2,3-dihydrobenzofuran-5-yl)pipérazin-1-yl)butyl)benzofuran-2-ylcarbamide
N-(4-(4-(2,3-dihydrobenzofuran-5-yl)pipérazin-1-yl)butyl)-5-bromobenzofuran-2-yl-carbamide
N-(4-(4-(2,3-dihydrobenzofuran-5-yl)pipérazin-1-yl)butyl)indol-2-ylcarbamide
N-(4-(4-(2,3-dihydrobenzofuran-5-yl)pipérazin-1-yl)butyl)-6-cyanoindol-2-ylcarbamide
N-(4-(4-(chroman-6-yl)pipérazin-1-yl)butyl)benzo[b]thiophèn-2-ylcarbamide
N-(4-(4-(chroman-6-yl)pipérazin-1-yl)butyl)-5-cyanobenzo[b]thiophèn-2-ylcarbamide
N-(4-(4-(chroman-6-yl)pipérazin-1-yl)butyl)benzofuran-2-ylcarbamide
N-(4-(4-(chroman-6-yl)pipérazin-1-yl)butyl)-5-bromobenzofuran-2-ylcarbamide
N-(4-(4-(chroman-6-yl)pipérazin-1-yl)butylindol-2-ylcarbamide
N-(4-(4-(chroman-6-yl)pipérazin-1-yl)butyl)-6-cyanoindol-2-ylcarbamide
N-(4-(4-(2,3,4,5-tétrahydrobenzo[b]oxépin-7-yl)pipérazin-1-yl)butyl)benzo[b]thiophèn-2-ylcarbamide
N-(4-(4-(2,3,4,5-tétrahydrobenzo[b]oxépin-7-yl)pipérazin-1-yl)butyl)-5-cyanobenzo[b] thiophèn-2-ylcarbamide
N-(4-(4-(2,3,4,5-tétrahydrobenzo[b]oxépin-7-yl)pipérazin-1-yl)butyl)benzofuran-2-yl-carbamide
N-(4-(4-(2,3,4,5-tétrahydrobenzo[b]oxépin-7-yl)pipérazin-1-yl)butyl)-5-bromobenzofuran-2-ylcarbamide
N-(4-(4-(2,3,4,5-tétrahydrobenzo[b]oxépin-7-yl)pipérazin-1-yl)butyl)indol-2-ylcarbamide
N-(4-(4-(2,3,4,5-tétrahydrobenzo[b]oxépin-7-yl)pipérazin-1-yl)butyl)-6-cyanoindol-2-ylcarbamide
(e) un composé selon la formule VII faisant partie du groupe :
N-(4-(4-(2,3-dihydrobenzofuran-5-yl)pipérazin-1-yl)butyl)benzo[b]thiophèn-3-ylcarbamide
N-(4-(4-(2,3-dihydrobenzofuran-5-yl)pipérazin-1-yl)butyl)benzofuran-3-ylcarbamide
N-(4-(4-(2,3-dihydrobenzofuran-5-yl)pipérazin-1-yl)butyl)indol-3-ylcarbamide
N-(4-(4-(chroman-6-yl)pipérazin-1-yl)butyl)benzo[b]thiophèn-3-ylcarbamide
N-(4-(4-(chroman-6-yl)pipérazin-1-yl)butyl)benzofuran-3-ylcarbamide
N-(4-(4-(chroman-6-yl)pipérazin-1-yl)butyl)indol-3-ylcarbamide
N-(4-(4-(2,3,4,5-tétrahydrobenzo[b]oxépin-7-yl)pipérazin-1-yl)butyl)benzo[b]thiophèn-3-ylcarbamide
N-(4-(4-(2,3,4,5-tétrahydrobenzo[b]oxépin-7-yl)pipérazin-1-yl)butyl)benzofuran-3-yl-carbamide
N-(4-(4-(2,3,4,5-tétrahydrobenzo[b]oxépin-7-yl)pipérazin-1-yl)butyl)indol-3-ylcarbamide

21. Composés selon l'une des revendications précédentes utilisés comme médicaments.

22. Composition pharmaceutique comprenant un ou plusieurs des composés selon l'une des revendications 1 à 20 et au moins un adjuvant pharmaceutiquement acceptable.

23. Utilisation d'un composé selon l'une quelconque des revendications 1 à 20 pour la fabrication d'un médicament destiné au traitement de maladies du système nerveux central.

24. Utilisation d'un composé selon l'une quelconque des revendications 1 à 20 pour la fabrication d'un médicament destiné au traitement de troubles de l'appareil urinaire.

25. Utilisation d'un composé selon l'une quelconque des revendications 1 à 20 pour la fabrication d'un médicament destiné au traitement de maladies entrant dans le groupe comprenant les psychoses, la schizophrénie, les troubles anxieux, les troubles compulsifs, les toxicomanies, les troubles dépressifs, les troubles moteurs extrapyramidaux d'origine médicamenteuse, la maladie de Parkinson, le syndrome de Segawa, le syndrome de Gilles de la Tourette, le syndrome des jambes sans repos, les troubles du sommeil, les nausées, les troubles cognitifs, les troubles de l'érection, l'hyperprolactinémie, les prolactinomes, le glaucome, le syndrome d'hyperactivité (HTA), l'autisme, les accidents vasculaires cérébraux et l'incontinence urinaire.

26. Utilisation selon l'une quelconque des revendications 1 à 20, dans laquelle le composé est utilisé pour fabriquer un médicament destiné au traitement de la schizophrénie, des troubles dépressifs, des troubles moteurs induits par la L-dopa ou les neuroleptiques, de la maladie de Parkinson, du syndrome de Segawa, du syndrome des jambes sans repos, de l'hyperprolactinémie, des prolactinomes, du syndrome d'hyperactivité (HTA) ou de l'incontinence urinaire.

27. Utilisation d'un composé selon l'une quelconque des revendications 1 à 20 pour la fabrication de médicaments destinés au traitement de la douleur.

28. Procédé pour la fabrication d'un composé selon l'une quelconque des revendications 1 à 20, comprenant la conversion d'un dérivé d'acide A. 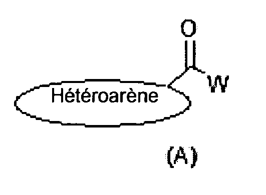 avec une base libre selon la formule générale C 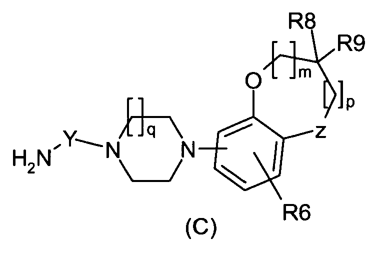 dans laquelle :
W est choisi parmi OH, CI, Br ou un groupe 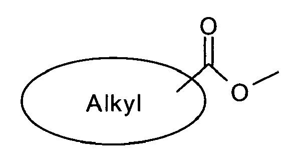 « hétéroarène » désigne, dans le dérivé d'acide A, un groupe selon la formule générale la 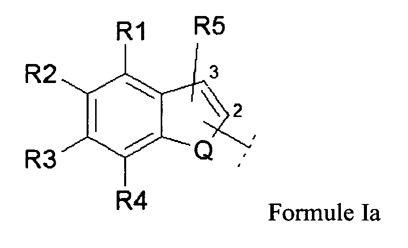
R1, R2, R3, R4, R5, R6, R8, R9, Q, Z, m, p et q ont la signification définie dans les revendications 1 à 20, la liaison en pointillés dans la formule la désignant la liaison du groupe -C(O)-W à la position 2 ou 3 dudit hétéroarène selon la formule Formel la.

29. Procédé selon la revendication 28, dans lequel W représente le chlore, le brome ou OH.

30. Procédé selon la revendication 28, dans lequel W est un groupe hydroxy et dans lequel le groupe acide est activé avec un réactif d'activation avant la conversion avec la base libre selon la formule générale C.

31. Procédé selon la revendication 30, dans lequel le réactif activation est choisi parmi les groupes hydroxybenzotriazole, hydroxybenzotriazole, HATU et TBTU.
